(19) Europäisches Patentamt
European Patent Office
Office européen des brevets

(11) EP 4 714 469 A1

(12) **EUROPEAN PATENT APPLICATION**
published in accordance with Art. 153(4) EPC

(43) Date of publication:
**25.03.2026 Bulletin 2026/13**

(21) Application number: **24806624.3**

(22) Date of filing: **16.05.2024**

(51) International Patent Classification (IPC):
***A61K 47/68*** (2017.01) ***C07K 16/28*** (2006.01)
***C07K 16/32*** (2006.01) ***A61K 31/4745*** (2006.01)
***A61K 9/19*** (2006.01) ***A61K 9/08*** (2006.01)
***A61P 35/00*** (2006.01) ***A61P 35/02*** (2006.01)

(52) Cooperative Patent Classification (CPC):
**A61K 9/08; A61K 9/19; A61K 31/4745; A61K 47/68; A61P 35/00; A61P 35/02; C07K 16/28; C07K 16/32**

(86) International application number:
**PCT/CN2024/093528**

(87) International publication number:
**WO 2024/235274 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.05.2023 CN 202310548571**

(71) Applicant: **CSPC Megalith Biopharmaceutical Co., Ltd.**
**Shijiazhuang, Hebei 050025 (CN)**

(72) Inventors:
- **HAN, Weiqiang**
**Shijiazhuang, Hebei 050025 (CN)**
- **XIE, Ru**
**Shijiazhuang, Hebei 050025 (CN)**
- **WANG, Xuming**
**Shijiazhuang, Hebei 050025 (CN)**
- **SU, Li**
**Shijiazhuang, Hebei 050025 (CN)**
- **ZHANG, Na**
**Shijiazhuang, Hebei 050025 (CN)**
- **WANG, Shanshan**
**Shijiazhuang, Hebei 050025 (CN)**
- **TIAN, Fangyuan**
**Shijiazhuang, Hebei 050025 (CN)**
- **SUN, Xiongfei**
**Shijiazhuang, Hebei 050025 (CN)**
- **HUI, Xiwu**
**Shijiazhuang, Hebei 050025 (CN)**
- **YAO, Bing**
**Shijiazhuang, Hebei 050025 (CN)**

(74) Representative: **Barrow, Nicholas Martin et al**
**Venner Shipley LLP**
**406 Cambridge Science Park**
**Milton Road**
**Cambridge CB4 0WW (GB)**

# (54) PHARMACEUTICAL COMPOSITION COMPRISING ANTIBODY-DRUG CONJUGATE

(57) A pharmaceutical composition comprising an antibody-drug conjugate, a preparation method therefor and a use thereof. Specifically, provided are a pharmaceutical composition comprising an antibody-drug conjugate represented by formula I, which is an injection, a preparation method therefor, and a use thereof in the preparation for a drug for treating proliferative diseases.

EP 4 714 469 A1

## Description

### Technical Field

The present invention relates to a pharmaceutical composition of an antibody-drug conjugate and a process for preparing the composition.

### Background of Art

The antibody-drug conjugate (ADC) consists of three different components (antibody, linker and drug). The ADC technology is to couple an antibody and a drug molecule together through a linker, utilize the specificity of the antibody to transport the drug molecule in a targeted manner to a target tissue to play a role, e.g. reducing the systemic toxic and side effects of the drug, expanding the drug treatment window, and improving the therapeutic potential of the antibody.

EGFR is an expression product of the proto-oncogene c-erbB1, and it is a member of the epidermal growth factor receptor (HER) family. This family includes HER1 (erbBl, EGFR), HER2 (erbB2, NEU), HER3 (erbB3), and HER4 (erbB4). EGFR is a transmembrane protein that plays an important role in cell growth, development, and differentiation. Among others, the overexpression of EGFR and its binding with specific ligands such as EGF and TGFα can lead to abnormal activation of EGFR; the activated EGFR can further activate and regulate multiple signal transduction pathways, thereby inhibiting tumor cell apoptosis, promoting tumor cell proliferation and migration, and promoting tumor angiogenesis by mediating VEGF expression, thus playing an important role in the process such as tumor occurrence, development, malignancy, and prognosis. Therefore, blocking the EGFR-mediated signal transduction pathway can achieve therapeutic effects against tumors.

HER3 (also known as ERBB3, human epidermal growth factor receptor 3) is a transmembrane receptor and a member of the epidermal growth factor receptor family (EGFR). The HER3 gene is located on the long arm of human chromosome 12 (12q13), and it encodes a protein with a molecular weight of either 160 or 180 kDa. HER3 is known to be expressed in various cancers, such as breast cancer, lung cancer, and colorectal cancer, and it forms heterodimers with tyrosine kinase receptors such as HER2 and EGFR, after which HER3 is phosphorylated, thereby inducing cancer cell growth or apoptosis inhibition signals.

CN202211461614.0 discloses an EGFR-targeted antibody-drug conjugate:

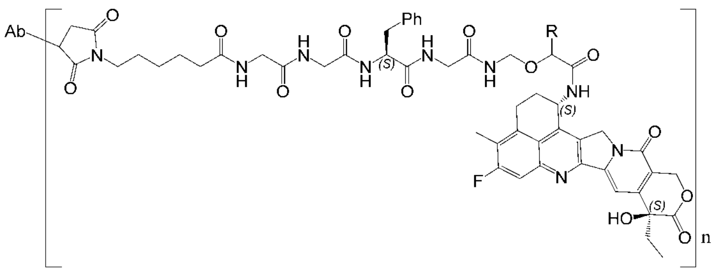

wherein Ab is an antibody targeting HER2, HER3, or EGFR or an antigen-binding fragment thereof, R is selected from $C_{1-6}$ alkyl, and n is an integer of 1-8 or a decimal of 1-8. In vitro studies have shown that this ADC has good inhibitory effects on various tumor cells.

In the study of antibody and antibody-drug conjugate (ADC) formulations, the formation of aggregates and the generation of degradation products cause pharmaceutically undesirable side effects, which pose risks of increased immunogenicity in patients receiving drug treatment or risks related to venous disorders. For the above reasons, it is necessary to inhibit the formation of aggregates and the generation of degradation products when preparing related formulations. Particularly when studying pharmaceutical formulations of antibody-drug conjugates, more technical challenges may be faced. It is necessary to consider not only the specific properties of the antibody part but also the specific properties of the drug-linker part. For example, during storage, small molecule payloads may detach from the antibody-drug conjugate, which can affect both the efficacy and toxicity of the relevant drug. It is our research direction to develop stable pharmaceutical compositions to solve the problems of aggregation and degradation of ADC molecules in the pharmaceutical compositions of antibody-drug conjugates.

### Summary of the Invention

The present invention provides a pharmaceutical composition, which contains an antibody-drug conjugate represented by formula I:

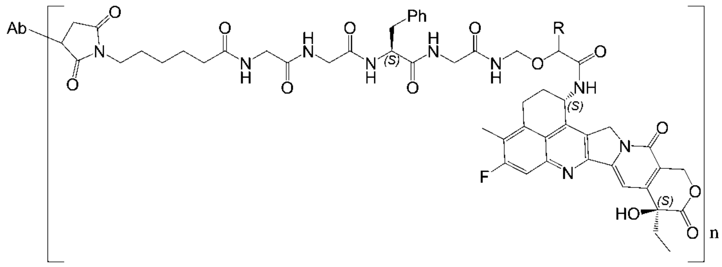

Formula I

wherein Ab is an antibody targeting HER2, HER3 or EGFR or an antigen-binding fragment thereof, R is $C_{1-6}$alkyl, and n is an integer of 1-8 or a decimal of 1-8.

Further, n is an integer of 4-8 or a decimal of 4-8.

Further,when n is an integral 1-8, it can be 1, 2, 3, 4, 5, 6, 7, or 8; when n is an integral of 4-8, it can be 4, 5, 6, 7, or 8.

Further, when n is a decimal, it refers to the average number of the linker-drug molecules conjugated per antibody unit.

In a preferable example of the present invention, R is $C_{1-3}$alkyl.

In a preferable example of the present invention, R is methyl, ethyl, propyl, or isopropyl.

In a preferable example of the present invention, R is methyl.

Further, the antibody-drug conjugate represented by formula I has a structure represented by formula Ia or formula Ib:

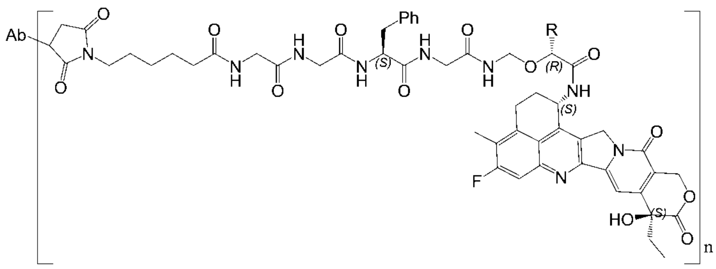

Formula Ia

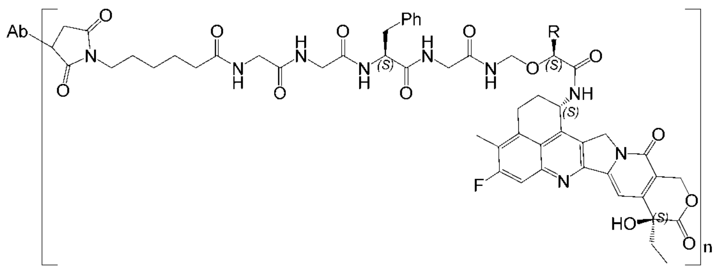

Formula Ib

wherein in formula Ia and formula Ib, Ab, R and n are defined as in formula I.

Further, the antibody-drug conjugate represented by formula I has a structure represented by formula I-1, Ia-1 or Ib-1:

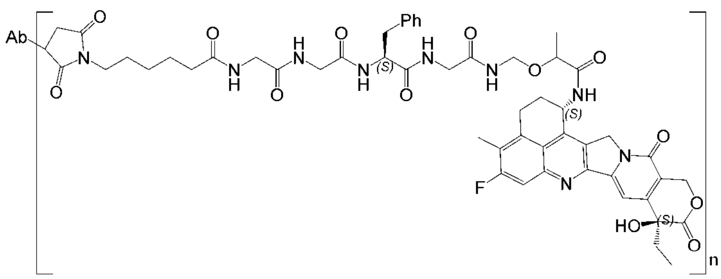

Formula I-1

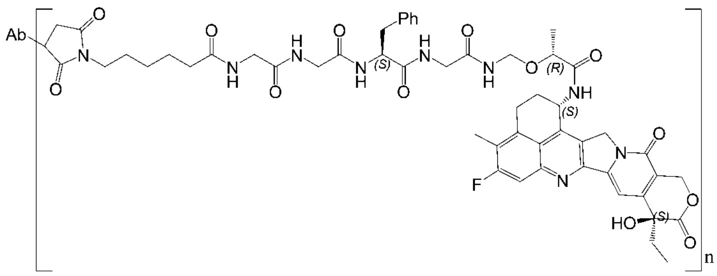

Formula Ia-1

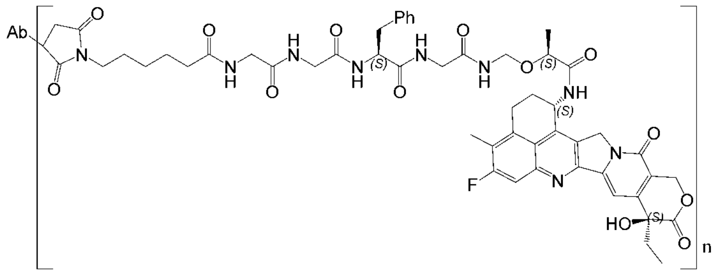

Formula Ib-1

wherein in formula I-1, formula Ia-1 and formula Ib-1, Ab and n are defined as in formula I.

In a preferable example of the present invention, in the above-mentioned antibody-drug conjugate, in case that the Ab is an antibody targeting HER2 or an antigen-binding fragment thereof, it comprises a variable region of a heavy chain (HV) and a variable region of a light chain (LV), wherein, a sequence of the variable region of the heavy chain comprises a heavy chain complementarity determining region 1 (HCDR1), a heavy chain complementarity determining region 2 (HCDR2) and a heavy chain complementarity determining region 3 (HCDR3), a sequence of the variable region of the light chain comprises a light chain complementarity determining region 1 (LCDR1), a light chain complementarity determining region 2 (LCDR2) and a light chain complementarity determining region 3 (LCDR3), wherein the amino acid sequence of HCDR1 is represented by SEQ ID NO:1, the amino acid sequence of HCDR2 is represented by SEQ ID NO:2, the amino acid sequence of HCDR3 is represented by SEQ ID NO:3, and/or the amino acid sequence of LCDR1 is represented by SEQ ID NO:4, the amino acid sequence of LCDR2 is represented by SEQ ID NO:5, the amino acid sequence of LCDR3 is represented by SEQ ID NO:6.

Further preferably, in case that the Ab is an antibody targeting HER2 or an antigen-binding fragment thereof, it comprises a variable region of a heavy chain (HV) and a variable region of a light chain (LV), wherein the amino acid sequence of HV is represented by SEQ ID NO:7, and the amino acid sequence of LV is represented by SEQ ID NO:8.

More preferably, in case that the Ab is an antibody targeting HER2 or an antigen-binding fragment thereof, it comprises a heavy chain (HC) and a light chain (LC), wherein the amino acid sequence of HC is represented by SEQ ID NO:9, and the amino acid sequence of of LC is represented by SEQ ID NO:10.

In a preferable example of the present invention, in case that the Ab is an antibody targeting HER3 or an antigen-binding fragment thereof, it comprises a variable region of a heavy chain (HV) and a variable region of a light chain (LV), wherein, a sequence of the variable region of the heavy chain comprises a heavy chain complementarity determining region 1 (HCDR1), a heavy chain complementarity determining region 2 (HCDR2) and a heavy chain complementarity determining region 3 (HCDR3), a sequence of the variable region of the light chain comprises a light chain complementarity determining region 1 (LCDR1), a light chain complementarity determining region 2 (LCDR2) and a light chain complementarity determining region 3 (LCDR3), wherein the amino acid sequence of HCDR1 is represented by SEQ ID NO:11, the amino acid sequence of HCDR2 is represented by SEQ ID NO:12, the amino acid sequence of HCDR3 is represented by SEQ ID NO:13, and/or the amino acid sequence of LCDR1 is represented by SEQ ID NO:14, the amino acid sequence of LCDR2 is represented by SEQ ID NO:15, the amino acid sequence of LCDR3 is represented by SEQ ID NO:16.

Further preferably, in case that the Ab is an antibody targeting HER3 or an antigen-binding fragment thereof, it comprises a variable region of a heavy chain (HV) and a variable region of a light chain (LV), wherein the amino acid sequence of HV is represented by SEQ ID NO:17, and the amino acid sequence of LV is represented by SEQ ID NO:18.

More preferably, in case that the Ab is an antibody targeting HER3 or an antigen-binding fragment thereof, it comprises a heavy chain (HC) and a light chain (LC), wherein the amino acid sequence of HC is represented by SEQ ID NO:19, the amino acid sequence of LC is represented by SEQ ID NO:20. In a preferable example of the present invention, in case that the Ab is an antibody targeting EGFR or an antigen-binding fragment thereof, it comprises a variable region of a heavy chain (HV) and a variable region of a light chain (LV), wherein, a sequence of the variable region of the heavy chain comprises a heavy chain complementarity determining region 1 (HCDR1), a heavy chain complementarity determining region

region 2 (HCDR2) and a heavy chain complementarity determining region 3 (HCDR3), a sequence of the variable region of the light chain comprises a light chain complementarity determining region 1 (LCDR1), a light chain complementarity determining region 2 (LCDR2) and a light chain complementarity determining region 3 (LCDR3), wherein the amino acid sequence of HCDR1 is represented by SEQ ID NO:21, the amino acid sequence of HCDR2 is represented by SEQ ID NO:22, the amino acid sequence of HCDR3 is represented by SEQ ID NO:23, and/or the amino acid sequence of LCDR1 is represented by SEQ ID NO:24, the amino acid sequence of LCDR2 is represented by SEQ ID NO:25, the amino acid sequence of LCDR3 is represented by SEQ ID NO:26.

In a preferable example of the present invention, in case that the Ab is an antibody targeting EGFR or an antigen-binding fragment thereof, it comprises a variable region of a heavy chain (HV) and a variable region of a light chain (LV), wherein the amino acid sequence of HCDR1 is represented by SEQ ID NO:31 or SEQ ID NO:35 or SEQ ID NO:38, the amino acid sequence of HCDR2 is represented by SEQ ID NO:22, the amino acid sequence of HCDR3 is represented by SEQ ID NO:23 or SEQ ID NO:32, and/or the amino acid sequence of LCDR1 is represented by SEQ ID NO:24, the amino acid sequence of LCDR2 is represented by SEQ ID NO:25, the amino acid sequence of LCDR3 is represented by SEQ ID NO:26.

In a preferable example of the present invention, in case that the Ab is an antibody targeting EGFR or an antigen-binding fragment thereof, it comprises a variable region of a heavy chain (HV) and a variable region of a light chain (LV), wherein the amino acid sequence of HCDR1 is represented by SEQ ID NO:31 or SEQ ID NO:35 or SEQ ID NO:38, the amino acid sequence of HCDR2 is represented by SEQ ID NO:22, the amino acid sequence of HCDR3 is represented by SEQ ID NO:32, and the amino acid sequence of LCDR1 is represented by SEQ ID NO:24, the amino acid sequence of LCDR2 is represented by SEQ ID NO:25, the amino acid sequence of LCDR3 is represented by SEQ ID NO:26.

Further preferably, in case that the Ab is an antibody targeting EGFR or an antigen-binding fragment thereof, it comprises a variable region of a heavy chain (HV) and a variable region of a light chain (LV), wherein the amino acid sequence of HV is represented by SEQ ID NO:27, the amino acid sequence of LV is represented by SEQ ID NO:28.

Further preferably, in case that the Ab is an antibody targeting EGFR or an antigen-binding fragment thereof, it comprises a variable region of a heavy chain (HV) and a variable region of a light chain (LV), wherein the amino acid sequence of HV is represented by SEQ ID NO:33 or SEQ ID NO:36 or SEQ ID NO:39, the amino acid sequence of LV is represented by SEQ ID NO:28.

More preferably, in case that the Ab is an antibody targeting EGFR or an antigen-binding fragment thereof, it comprises a heavy chain (HC) and a light chain (LC), wherein the amino acid sequence of HC is represented by SEQ ID NO:29, the amino acid sequence of LC is represented by SEQ ID NO:30. More preferably, in case that the Ab is an antibody targeting EGFR or an antigen-binding fragment thereof, it comprises a heavy chain (HC) and a light chain (LC), wherein the amino acid sequence of HC is represented by SEQ ID NO:34 or SEQ ID NO:37 or SEQ ID NO:40, the amino acid sequence of LC is represented by SEQ ID NO:30.

In case that Ab is an antibody targeting HER2, HER3 or EGFR, the antibody is preferably a monoclonal antibody, and the monoclonal antibody is preferably selected from human-source antibody, humanized antibody, and chimeric antibody.

In case that Ab is an antigen-binding fragment targeting HER2, HER3 or EGFR, the antigen-binding fragment is preferably selected from Fab', (Fab')2, Fab, Fv, scFv, dAb.

In the present invention, the numbering rule of the antibody amino acid sequence adopts the Kabat numbering rule.

Further, the antibody-drug conjugate represented by formula I has a structure as shown below:

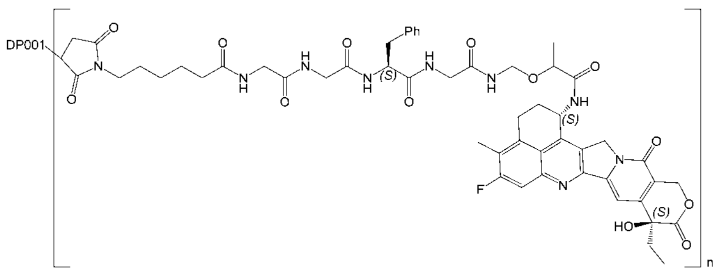

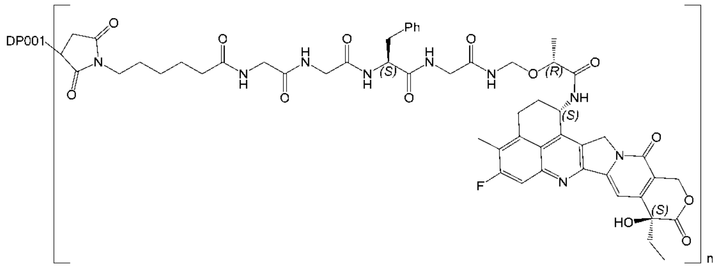

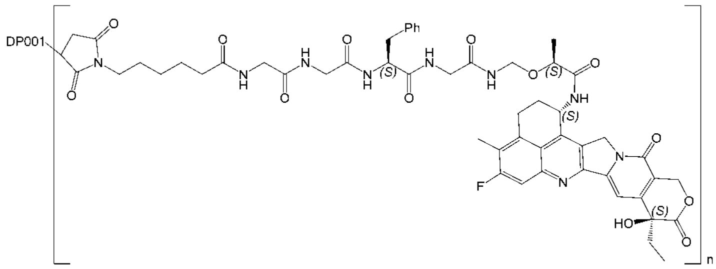

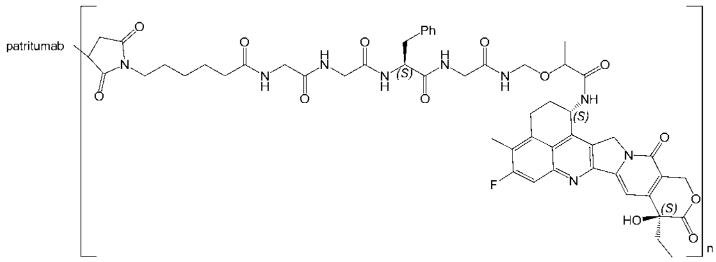

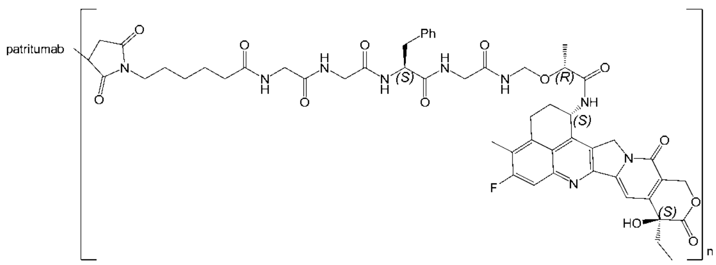

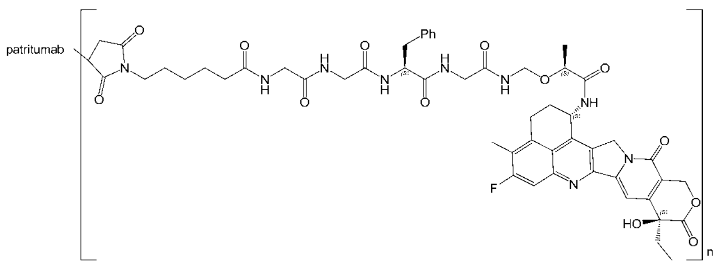

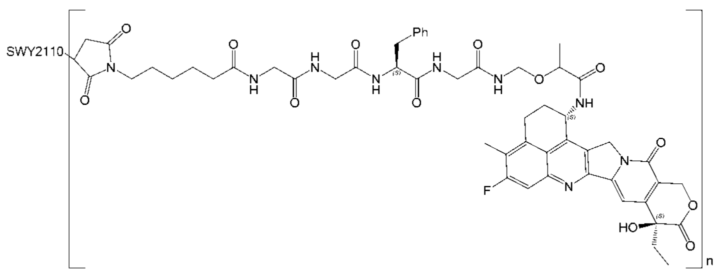

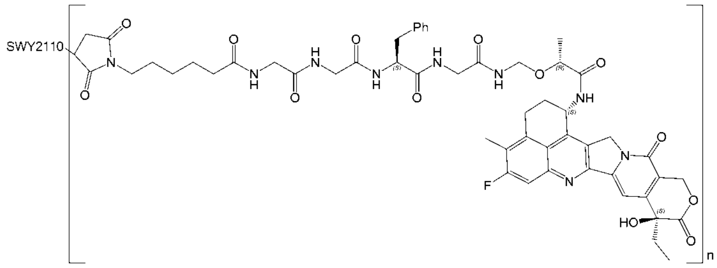

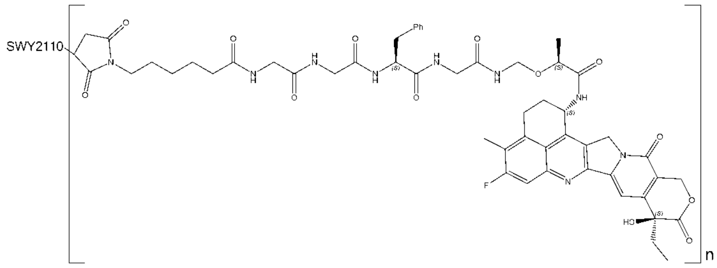

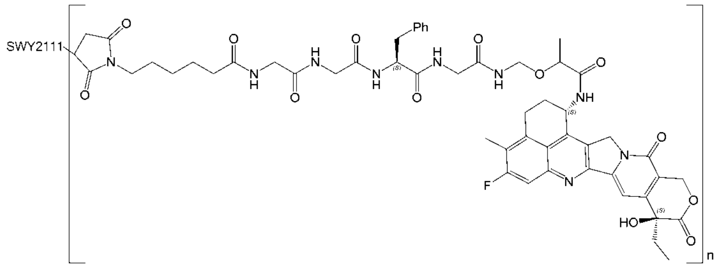

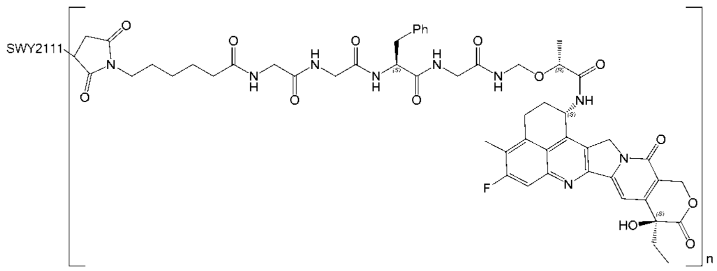

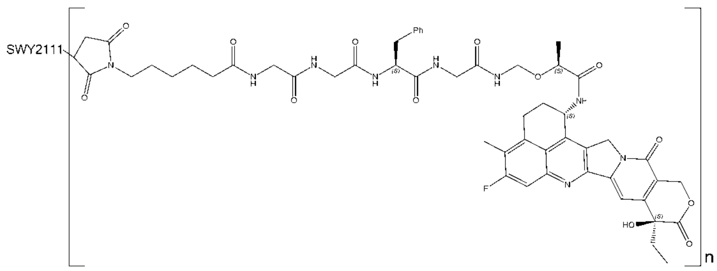

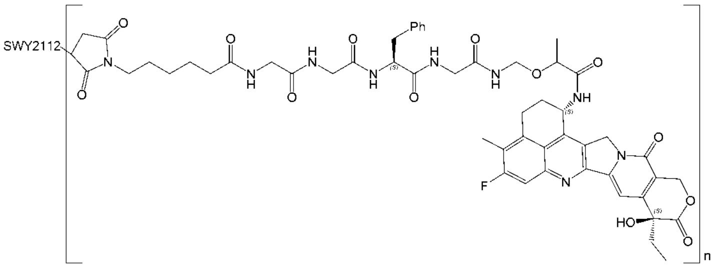

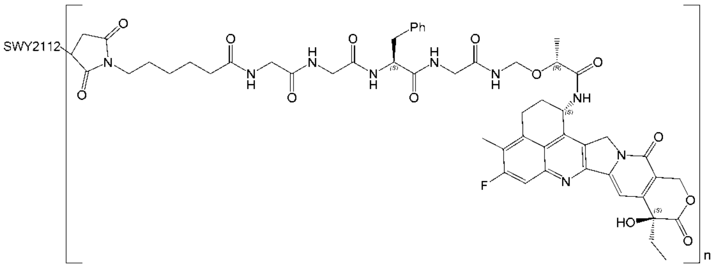

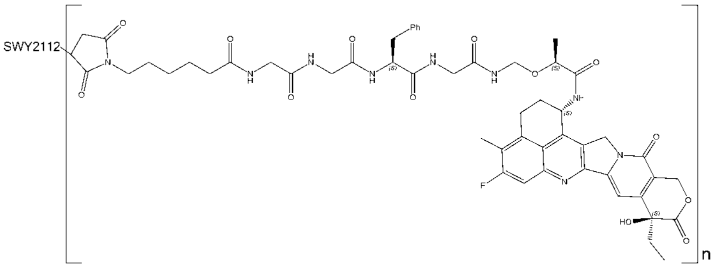

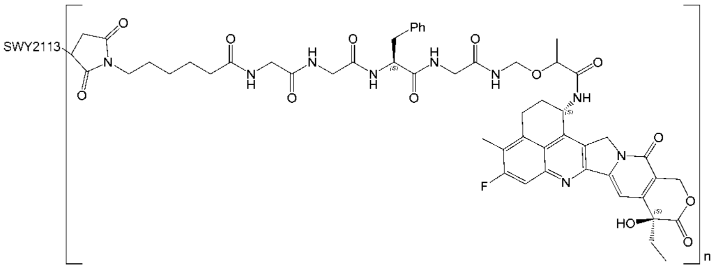

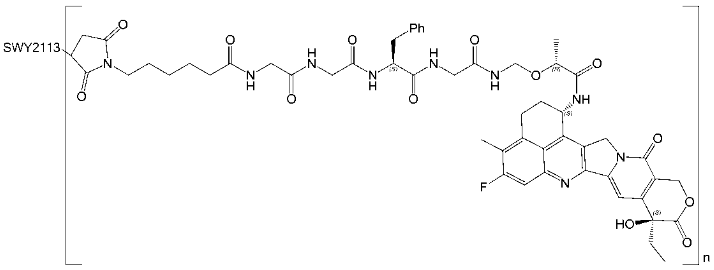

or

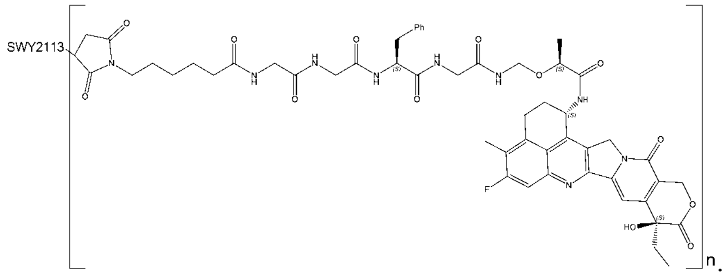

Further, the antibody-drug conjugate represented by formula I has a structure as shown below:

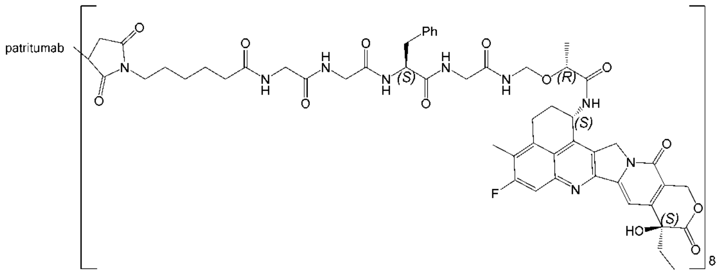

ADC-1

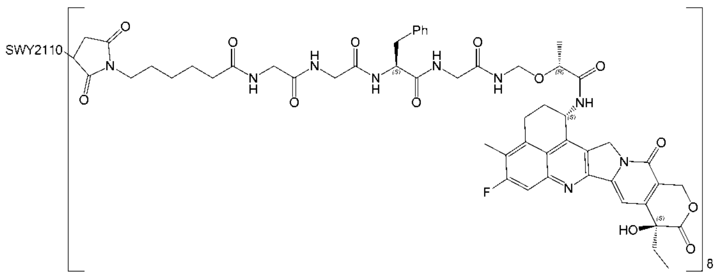

ADC-2

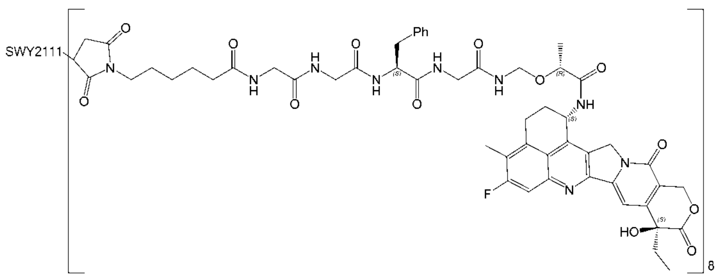

ADC-3

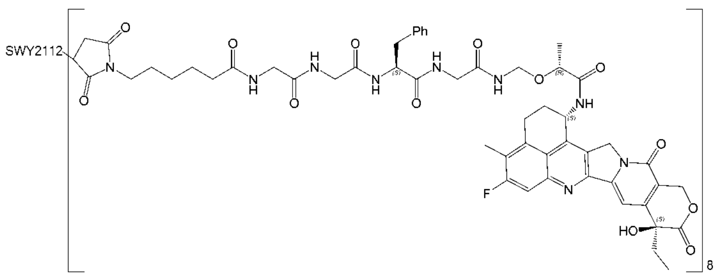

ADC-4

or

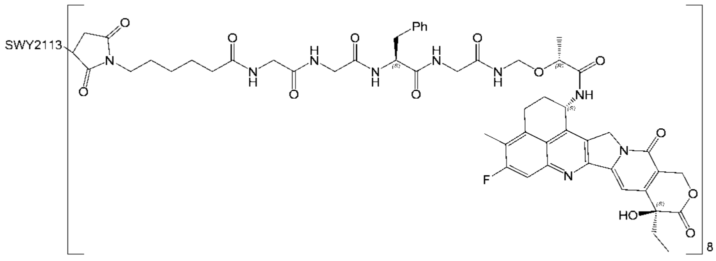 ADC-5.

In the above-mentioned antibody-drug conjugates:

DP001 is an antibody having two heavy chains with the amino acid sequence represented by SEQ ID NO:9 and two light chains with the amino acid sequence represented by SEQ ID NO:10;
Patritumab is an antibody having two heavy chains with the amino acid sequence represented by SEQ ID NO:19 and two light chains with the amino acid sequence represented by SEQ ID NO:20;
SWY2110 is an antibody having two heavy chains with the amino acid sequence represented by SEQ ID NO:29 and two light chains with the amino acid sequence represented by SEQ ID NO:30;
SWY2111 is an antibody having two heavy chains with the amino acid sequence represented by SEQ ID NO:34 and two light chains with the amino acid sequence represented by SEQ ID NO:30;
SWY2112 is an antibody having two heavy chains with the amino acid sequence represented by SEQ ID NO:37 and two light chains with the amino acid sequence represented by SEQ ID NO:30;
SWY2113 is an antibody having two heavy chains with the amino acid sequence represented by SEQ ID NO:40 and two light chains with the amino acid sequence represented by SEQ ID NO:30;
n is an integer of 1-8 or a decimal of 1-8.

Further, n is an integer of 4-8 or a decimal of 4-8.

Further, n is 8.

Further, when n is an integral 1-8, it can be 1, 2, 3, 4, 5, 6, 7, or 8; when n is an integral of 4-8, it can be 4, 5, 6, 7, or 8.

Furthermore, the pharmaceutical composition is an injection, preferably an injection solution or a lyophilized injection, more preferably a lyophilized injection.

The lyophilized injection is a solid injection (i.e., lyophilized powder), which can be used by dissolving in water (preferably water for injection) at the time of use, and which is obtained by lyophilizing an aqueous solution containing a predetermined amount of the drug component.

In the present invention, water for injection refers to water that meets the specifications under the section "water for injection" of the Chinese Pharmacopoeia (2020).

Furthermore, the pharmaceutical composition comprises the antibody-drug conjugate represented by formula I and a buffering agent.

Furthermore, the pharmaceutical composition further comprises a stabilizer.

Furthermore, the pharmaceutical composition further comprises a surfactant.

Furthermore, the pharmaceutical composition may further comprise water.

In some embodiments, the pharmaceutical composition comprises the antibody-drug conjugate represented by formula I, a buffering agent, a stabilizer, and a surfactant.

In some embodiments, the pharmaceutical composition further comprises water.

In some embodiments, in the pharmaceutical composition, the antibody-drug conjugate represented by formula I is selected from ADC-1, ADC-2, ADC-3, ADC-4, and ADC-5.

In some embodiments, the buffering agent includes, but is not limited to, acetate, succinate, gluconate, histidine salt, oxalate, lactate, phosphate, citrate, tartrate, fumarate, glycylglycine, tris(hydroxymethyl)aminomethane (Tris), 2-(N-morpholino)ethanesulfonic acid (MES), and other organic acid buffering agents.

In some embodiments, the histidine salt buffering agent is a buffering agent containing histidine ions. Examples of histidine salt buffering agents include histidine-histidine hydrochloride, histidine-acetate, histidine-phosphate, histidine-sulfate, and other buffering agents, wherein the histidine-histidine hydrochloride buffering agent is prepared from histidine and histidine hydrochloride, and the histidine-acetate buffering agent is prepared from histidine and acetic acid. Similarly, the succinate buffering agent may be succinic acid-sodium succinate, and the citrate buffering agent may be citric acid-sodium citrate.

In some embodiments, the histidine-histidine hydrochloride buffering agent is preferred.

In some embodiments, the stabilizer includes, but is not limited to, saccharides (such as sucrose and trehalose), polyols (such as mannitol and sorbitol), amino acids (such as L-serine, sodium glutamate, alanine, glycine, and sarcosine).

In some embodiments, the surfactant includes, but is not limited to, polysorbates (such as polysorbate 20 and

polysorbate 80).

In the following embodiments describing pH, weight percentage content (in weight%), and content (in weight/-volume such as mg/ml), in the absence of contrary teaching, the pH value of the pharmaceutical composition is the pH value of the aqueous solution of the pharmaceutical composition (wherein the water is water for injection); in the absence of contrary teaching, when describing the weight percentage content, it is based on the total weight of the pharmaceutical composition being 100 wt% and on the premise that the pharmaceutical composition does not include water for injection; in the absence of contrary teaching, when describing the content, the pharmaceutical composition includes water for injection and is based on the volume of the pharmaceutical composition.

In some embodiments, the pH of the pharmaceutical composition is 4.0-7.5 (e.g. about 4.0, about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.5, about 7.0), preferably 4.5-7.0, further preferably 4.5-5.5, further preferably 5.0-5.5, most preferably about 5.0.

In the above embodiments and similar embodiments below, "about" before a point value includes the value itself and values within an error range understandable by those skilled in the art.

In some embodiments, in the pharmaceutical composition, the weight percentage content of the antibody-drug conjugate represented by formula I (such as ADC-1, ADC-2, ADC-3, ADC-4, and ADC-5) is 1-30% (e.g. about 2%, about 3%, about 4%, about 5%, about 6%, about 7%, about 8%, about 8.5%, about 9%, about 9.5%, about 10%, about 10.5%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%), preferably 5-25%, 8-20%, 8-15%, 8-10%, 15-25%.

In some embodiments, in the pharmaceutical composition, the content of the antibody-drug conjugate represented by formula I (such as ADC-1, ADC-2, ADC-3, ADC-4, and ADC-5) is 1-30 mg/ml (e.g. about 1 mg/ml, about 2 mg/ml, about 3 mg/ml, about 4 mg/ml, about 5 mg/ml, about 6 mg/ml, about 7 mg/ml, about 8 mg/ml, about 8.5 mg/ml, about 9 mg/ml, about 9.5 mg/ml, about 10 mg/ml, about 10.5 mg/ml, about 11 mg/ml, about 12 mg/ml, about 13 mg/ml, about 14 mg/ml, about 15 mg/ml, about 16 mg/ml, about 17 mg/ml, about 18 mg/ml, about 19 mg/ml, about 20 mg/ml, about 21 mg/ml, about 22 mg/ml, about 23 mg/ml, about 24 mg/ml, about 25 mg/ml, about 26 mg/ml, about 27 mg/ml, about 28 mg/ml, about 29 mg/ml, about 30 mg/ml), preferably 5-25 mg/ml, further preferably 5-20 mg/ml, 5-15 mg/ml, 15-25 mg/ml.

In some embodiments, in the pharmaceutical composition, the weight percentage content of the buffering agent (such as histidine-histidine hydrochloride) is 1-10% (e.g. about 1%, about 2%, about 2.8%, about 2.9%, about 3%, about 3.1%, about 3.2%, about 4%, about 4.5%, about 4.6%, about 4.7%, about 4.8%, about 4.9%, about 5%, about 5.1%, about 5.2%, about 5.3%, about 5.4%, about 5.5%, about 6%, about 6.5%, about 7%, about 8%, about 9%, about 9.9%, about 10%), preferably 1-9.9%, 2-6%, 2-5%, 4-6%.

In some embodiments, in the pharmaceutical composition, the content of the buffering agent (such as histidine-histidine hydrochloride) is 1-10 mg/ml (e.g. about 1 mg/ml, about 2 mg/ml, about 3 mg/ml, about 4 mg/ml, about 4.5 mg/ml, about 4.6 mg/ml, about 4.7 mg/ml, about 4.8 mg/ml, about 4.9 mg/ml, about 5 mg/ml, about 5.1 mg/ml, about 5.2 mg/ml, about 5.3 mg/ml, about 5.4 mg/ml, about 5.5 mg/ml, about 6 mg/ml, about 6.5 mg/ml, about 7 mg/ml, about 8 mg/ml, about 9 mg/ml, about 10 mg/ml), preferably 2-6 mg/ml, 2-4 mg/ml, 3-6 mg/ml, 4-6 mg/ml.

In some embodiments, in the pharmaceutical composition, the buffering agent is histidine-histidine hydrochloride, wherein the weight percentage content of histidine is 0.1-1% (e.g. about 0.1%, about 0.2%, about 0.25%, about 0.26%, about 0.27%, about 0.28%, about 0.29%, about 0.3%, about 0.31%, about 0.32%, about 0.33%, about 0.34%, about 0.35%, about 0.4%, about 0.45%, about 0.5%, about 0.55%, about 0.6%, about 0.7%, about 0.8%, about 0.9%, about 1%), preferably 0.1-0.5%, 0.2-0.4%.

In some embodiments, in the pharmaceutical composition, the buffering agent is histidine-histidine hydrochloride, wherein the content of histidine is 0.1-1mg/ml (e.g. about 0.1 mg/ml, about 0.2 mg/ml, about 0.21 mg/ml, about 0.22 mg/ml, about 0.23 mg/ml, about 0.24 mg/ml, about 0.25 mg/ml, about 0.26 mg/ml, about 0.27 mg/ml, about 0.28 mg/ml, about 0.29 mg/ml, about 0.3 mg/ml, about 0.31 mg/ml, about 0.32 mg/ml, about 0.33 mg/ml, about 0.34 mg/ml, about 0.35 mg/ml, about 0.4 mg/ml, about 0.45 mg/ml, about 0.5 mg/ml, about 0.55 mg/ml, about 0.6 mg/ml, about 0.7 mg/ml, about 0.8 mg/ml, about 0.9 mg/ml, about 1mg/ml), preferably 0.1-0.5 mg/ml, 0.1-0.3 mg/ml, 0.2-0.4 mg/ml.

In some embodiments, in the pharmaceutical composition, the buffering agent is histidine-histidine hydrochloride, wherein the weight percentage content of histidine hydrochloride is 1-10% (e.g. about 1%, about 2%, about 2.5%, about 2.6%, about 2.7%, about 2.8%, about 2.9%, about 3%, about 3.5%, about 4%, about 4.5%, about 4.6%, about 4.7%, about 4.8%, about 4.9%, about 5%, about 5.1%, about 5.2%, about 5.3%, about 5.4%, about 5.5%, about 6%, about 6.5%, about 7%, about 8%, about 9%, about 9.9%, about 10%), preferably 1-9.9%, 2-6%, 2-4%, 3-6%, 3.5-5.5%, 4-5%.

In some embodiments, in the pharmaceutical composition, the buffering agent is histidine-histidine hydrochloride, wherein the content of histidine hydrochloride is 1-10 mg/ml (e.g. about 1 mg/ml, about 2 mg/ml, about 2.5 mg/ml, about 2.6 mg/ml, about 2.7 mg/ml, about 2.8 mg/ml, about 2.9 mg/ml, about 3 mg/ml, about 3.5 mg/ml, about 4 mg/ml, about 4.5 mg/ml, about 4.6 mg/ml, about 4.7 mg/ml, about 4.8 mg/ml, about 4.9 mg/ml, about 5 mg/ml, about 5.1 mg/ml, about 5.2 mg/ml, about 5.3 mg/ml, about 5.4 mg/ml, about 5.5 mg/ml, about 6 mg/ml, about 6.5 mg/ml, about 7 mg/ml,

about 8 mg/ml, about 9 mg/ml, about 10 mg/ml), preferably 2-6 mg/ml, 2-4 mg/ml, 3-6 mg/ml, about 4-5.5 mg/ml, about 4.5-5 mg/ml.

In some embodiments, in the pharmaceutical composition, the weight percentage content of the stabilizer (such as trehalose, sucrose) is 50-90% (e.g. about 50%, about 60%, about 70%, about 75%, about 80%, about 81%, about 82%, about 83%, about 84%, about 85%, about 86%, about 87%, about 88%, about 89%, about 90%), preferably 60-90%, 70-90%, 80-90%.

In some embodiments, in the pharmaceutical composition, the content of the stabilizer (such as trehalose, sucrose) is 50-110 mg/ml (e.g. about 50 mg/ml, about 60 mg/ml, about 70 mg/ml, about 75 mg/ml, about 80 mg/ml, about 81 mg/ml, about 82 mg/ml, about 83 mg/ml, about 84 mg/ml, about 85 mg/ml, about 86 mg/ml, about 87 mg/ml, about 88 mg/ml, about 89 mg/ml, about 90 mg/ml, about 91 mg/ml, about 92 mg/ml, about 93 mg/ml, about 94 mg/ml, about 95 mg/ml, about 100 mg/ml, about 105 mg/ml, about 110 mg/ml), preferably 70-100 mg/ml, 80-100 mg/ml, 75-95 mg/ml.

In some embodiments, in the pharmaceutical composition, the weight percentage content of the surfactant (such as polysorbate 80) is 0.1-1% (e.g. about 0.1%, about 0.15%, about 0.16%, about 0.17%, about 0.18%, about 0.19%, about 0.2%, about 0.25%, about 0.26%, about 0.27%, about 0.28%, about 0.29%, about 0.3%, about 0.31%, about 0.32%, about 0.33%, about 0.34%, about 0.35%, about 0.4%, about 0.45%, about 0.5%, about 0.55%, about 0.6%, about 0.7%, about 0.8%, about 0.9%, about 1%), preferably 0.2-0.4%, further preferably 0.15-0.4%, 0.15-0.35%, 0.25-0.35%.

In some embodiments, in the pharmaceutical composition, the content of the surfactant (such as polysorbate 80) is 0.1-1mg/ml (e.g. about 0.1 mg/ml, about 0.15 mg/ml, about 0.16 mg/ml, about 0.17 mg/ml, about 0.18 mg/ml, about 0.19 mg/ml, about 0.2 mg/ml, about 0.25 mg/ml, about 0.3 mg/ml, about 0.35 mg/ml, about 0.4 mg/ml, about 0.45 mg/ml, about 0.5 mg/ml, about 0.55 mg/ml, about 0.6 mg/ml, about 0.7 mg/ml, about 0.8 mg/ml, about 0.9 mg/ml, about 1mg/ml), preferably 0.1-0.4 mg/ml, 0.15-0.25 mg/ml, 0.2-0.4 mg/ml, 0.25-0.35 mg/ml.

In some embodiments, the pharmaceutical composition, in terms of weight percentage content, contains 1-30% (e.g. about 2%, about 3%, about 4%, about 5%, about 6%, about 7%, about 8%, about 8.5%, about 9%, about 9.5%, about 10%, about 10.5%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%) of the antibody-drug conjugate represented by formula I (such as ADC-1, ADC-2, ADC-3, ADC-4, and ADC-5), and 0.1-1% (e.g. about 0.1%, about 0.2%, about 0.25%, about 0.26%, about 0.27%, about 0.28%, about 0.29%, about 0.3%, about 0.31%, about 0.32%, about 0.33%, about 0.34%, about 0.35%, about 0.4%, about 0.45%, about 0.5%, about 0.55%, about 0.6%, about 0.7%, about 0.8%, about 0.9%, about 1%) of histidine, 1-10% (e.g. 1-9.9%, or about 1%, about 2%, about 2.5%, about 2.6%, about 2.7%, about 2.8%, about 2.9%, about 3%, about 3.5%, about 4%, about 4.5%, about 4.6%, about 4.7%, about 4.8%, about 4.9%, about 5%, about 5.1%, about 5.2%, about 5.3%, about 5.4%, about 5.5%, about 6%, about 6.5%, about 7%, about 8%, about 9%, about 9.9%, about 10%) of histidine hydrochloride, 50-90% (e.g. about 50%, about 60%, about 70%, about 75%, about 80%, about 81%, about 82%, about 83%, about 84%, about 85%, about 86%, about 87%, about 88%, about 89%, about 90%) of trehalose, 0.1-1% (e.g. about 0.1%, about 0.15%, about 0.16%, about 0.17%, about 0.18%, about 0.19%, about 0.2%, about 0.25%, about 0.26%, about 0.27%, about 0.28%, about 0.29%, about 0.3%, about 0.31%, about 0.32%, about 0.33%, about 0.34%, about 0.35%, about 0.4%, about 0.45%, about 0.5%, about 0.55%, about 0.6%, about 0.7%, about 0.8%, about 0.9%, about 1%) of polysorbate 80.

In some embodiments, the pharmaceutical composition, in terms of weight percentage content, contains 1-30% (e.g. about 2%, about 3%, about 4%, about 5%, about 6%, about 7%, about 8%, about 8.5%, about 9%, about 9.5%, about 10%, about 10.5%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%) of the antibody-drug conjugate represented by formula I (such as ADC-1, ADC-2, ADC-3, ADC-4, and ADC-5), and 0.1-1% (e.g. about 0.1%, about 0.2%, about 0.25%, about 0.26%, about 0.27%, about 0.28%, about 0.29%, about 0.3%, about 0.31%, about 0.32%, about 0.33%, about 0.34%, about 0.35%, about 0.4%, about 0.45%, about 0.5%, about 0.55%, about 0.6%, about 0.7%, about 0.8%, about 0.9%, about 1%) of histidine, 1-10% (e.g. 1-9.9%, or about 1%, about 2%, about 2.5%, about 2.6%, about 2.7%, about 2.8%, about 2.9%, about 3%, about 3.5%, about 4%, about 4.5%, about 4.6%, about 4.7%, about 4.8%, about 4.9%, about 5%, about 5.1%, about 5.2%, about 5.3%, about 5.4%, about 5.5%, about 6%, about 6.5%, about 7%, about 8%, about 9%, about 9.9%, about 10%) of histidine hydrochloride, 50-90% (e.g. about 50%, about 60%, about 70%, about 75%, about 80%, about 81%, about 82%, about 83%, about 84%, about 85%, about 86%, about 87%, about 88%, about 89%, about 90%) of sucrose, 0.1-1% (e.g. about 0.1%, about 0.15%, about 0.16%, about 0.17%, about 0.18%, about 0.19%, about 0.2%, about 0.25%, about 0.26%, about 0.27%, about 0.28%, about 0.29%, about 0.3%, about 0.31%, about 0.32%, about 0.33%, about 0.34%, about 0.35%, about 0.4%, about 0.45%, about 0.5%, about 0.55%, about 0.6%, about 0.7%, about 0.8%, about 0.9%, about 1%) of polysorbate 80.

In some embodiments, the pharmaceutical composition contains 1-30 mg/ml (e.g. about 1 mg/ml, about 2 mg/ml, about 3 mg/ml, about 4 mg/ml, about 5 mg/ml, about 6 mg/ml, about 7 mg/ml, about 8 mg/ml, about 8.5 mg/ml, about 9 mg/ml, about 9.5 mg/ml, about 10 mg/ml, about 10.5 mg/ml, about 11 mg/ml, about 12 mg/ml, about 13 mg/ml, about 14 mg/ml, about 15 mg/ml, about 16 mg/ml, about 17 mg/ml, about 18 mg/ml, about 19 mg/ml, about 20 mg/ml, about 21 mg/ml, about 22 mg/ml, about 23 mg/ml, about 24 mg/ml, about 25 mg/ml, about 26 mg/ml, about 27 mg/ml, about 28

mg/ml, about 29 mg/ml, about 30 mg/ml) of the antibody-drug conjugate represented by formula I (such as ADC-1, ADC-2, ADC-3, ADC-4, and ADC-5), and 0.1-1mg/ml (e.g. about 0.1 mg/ml, about 0.2 mg/ml, about 0.21 mg/ml, about 0.22 mg/ml, about 0.23 mg/ml, about 0.24 mg/ml, about 0.25 mg/ml, about 0.26 mg/ml, about 0.27 mg/ml, about 0.28 mg/ml, about 0.29 mg/ml, about 0.3 mg/ml, about 0.31 mg/ml, about 0.32 mg/ml, about 0.33 mg/ml, about 0.34 mg/ml, about 0.35 mg/ml, about 0.4 mg/ml, about 0.45 mg/ml, about 0.5 mg/ml, about 0.55 mg/ml, about 0.6 mg/ml, about 0.7 mg/ml, about 0.8 mg/ml, about 0.9 mg/ml, about 1mg/ml) of histidine, 1-10 mg/ml (e.g. about 1 mg/ml, about 2 mg/ml, about 2.5 mg/ml, about 2.6 mg/ml, about 2.7 mg/ml, about 2.8 mg/ml, about 2.9 mg/ml, about 3 mg/ml, about 3.5 mg/ml, about 4 mg/ml, about 4.5 mg/ml, about 4.6 mg/ml, about 4.7 mg/ml, about 4.8 mg/ml, about 4.9 mg/ml, about 5 mg/ml, about 5.1 mg/ml, about 5.2 mg/ml, about 5.3 mg/ml, about 5.4 mg/ml, about 5.5 mg/ml, about 6 mg/ml, about 6.5 mg/ml, about 7 mg/ml, about 8 mg/ml, about 9 mg/ml, about 10 mg/ml) of histidine hydrochloride, 50-110 mg/ml (e.g. about 50 mg/ml, about 60 mg/ml, about 70 mg/ml, about 75 mg/ml, about 80 mg/ml, about 81 mg/ml, about 82 mg/ml, about 83 mg/ml, about 84 mg/ml, about 85 mg/ml, about 86 mg/ml, about 87 mg/ml, about 88 mg/ml, about 89 mg/ml, about 90 mg/ml, about 91 mg/ml, about 92 mg/ml, about 93 mg/ml, about 94 mg/ml, about 95 mg/ml, about 100 mg/ml, about 105 mg/ml, about 110 mg/ml) of trehalose, 0.1-1mg/ml (e.g. about 0.1 mg/ml, about 0.15 mg/ml, about 0.16 mg/ml, about 0.17 mg/ml, about 0.18 mg/ml, about 0.19 mg/ml, about 0.2 mg/ml, about 0.25 mg/ml, about 0.3 mg/ml, about 0.35 mg/ml, about 0.4 mg/ml, about 0.45 mg/ml, about 0.5 mg/ml, about 0.55 mg/ml, about 0.6 mg/ml, about 0.7 mg/ml, about 0.8 mg/ml, about 0.9 mg/ml, about 1mg/ml) of polysorbate 80.

In some embodiments, the pharmaceutical composition contains 1-30 mg/ml (e.g. about 1 mg/ml, about 2 mg/ml, about 3 mg/ml, about 4 mg/ml, about 5 mg/ml, about 6 mg/ml, about 7 mg/ml, about 8 mg/ml, about 8.5 mg/ml, about 9 mg/ml, about 9.5 mg/ml, about 10 mg/ml, about 10.5 mg/ml, about 11 mg/ml, about 12 mg/ml, about 13 mg/ml, about 14 mg/ml, about 15 mg/ml, about 16 mg/ml, about 17 mg/ml, about 18 mg/ml, about 19 mg/ml, about 20 mg/ml, about 21 mg/ml, about 22 mg/ml, about 23 mg/ml, about 24 mg/ml, about 25 mg/ml, about 26 mg/ml, about 27 mg/ml, about 28 mg/ml, about 29 mg/ml, about 30 mg/ml) of the antibody-drug conjugate represented by formula I (e.g. ADC-1, ADC-2, ADC-3, ADC-4, ADC-5), and 0.1-1mg/ml (e.g. about 0.1 mg/ml, about 0.2 mg/ml, about 0.21 mg/ml, about 0.22 mg/ml, about 0.23 mg/ml, about 0.24 mg/ml, about 0.25 mg/ml, about 0.26 mg/ml, about 0.27 mg/ml, about 0.28 mg/ml, about 0.29 mg/ml, about 0.3 mg/ml, about 0.31 mg/ml, about 0.32 mg/ml, about 0.33 mg/ml, about 0.34 mg/ml, about 0.35 mg/ml, about 0.4 mg/ml, about 0.45 mg/ml, about 0.5 mg/ml, about 0.55 mg/ml, about 0.6 mg/ml, about 0.7 mg/ml, about 0.8 mg/ml, about 0.9 mg/ml, about 1mg/ml) of histidine, 1-10 mg/ml (e.g. about 1 mg/ml, about 2 mg/ml, about 2.5 mg/ml, about 2.6 mg/ml, about 2.7 mg/ml, about 2.8 mg/ml, about 2.9 mg/ml, about 3 mg/ml, about 3.5 mg/ml, about 4 mg/ml, about 4.5 mg/ml, about 4.6 mg/ml, about 4.7 mg/ml, about 4.8 mg/ml, about 4.9 mg/ml, about 5 mg/ml, about 5.1 mg/ml, about 5.2 mg/ml, about 5.3 mg/ml, about 5.4 mg/ml, about 5.5 mg/ml, about 6 mg/ml, about 6.5 mg/ml, about 7 mg/ml, about 8 mg/ml, about 9 mg/ml, about 10 mg/ml) of histidine hydrochloride, 50-110 mg/ml (e.g. about 50 mg/ml, about 60 mg/ml, about 70 mg/ml, about 75 mg/ml, about 80 mg/ml, about 81 mg/ml, about 82 mg/ml, about 83 mg/ml, about 84 mg/ml, about 85 mg/ml, about 86 mg/ml, about 87 mg/ml, about 88 mg/ml, about 89 mg/ml, about 90 mg/ml, about 91 mg/ml, about 92 mg/ml, about 93 mg/ml, about 94 mg/ml, about 95 mg/ml, about 100 mg/ml, about 105 mg/ml, about 110 mg/ml) of sucrose, 0.1-1mg/ml (e.g. about 0.1 mg/ml, about 0.15 mg/ml, about 0.16 mg/ml, about 0.17 mg/ml, about 0.18 mg/ml, about 0.19 mg/ml, about 0.2 mg/ml, about 0.25 mg/ml, about 0.3 mg/ml, about 0.35 mg/ml, about 0.4 mg/ml, about 0.45 mg/ml, about 0.5 mg/ml, about 0.55 mg/ml, about 0.6 mg/ml, about 0.7 mg/ml, about 0.8 mg/ml, about 0.9 mg/ml, about 1mg/ml) of polysorbate 80.

In some embodiments, after the pharmaceutical composition is dissolved, it contains about 10 mg/ml of the antibody-drug conjugate represented by formula I, about 0.33 mg/ml of histidine, about 4.79 mg/ml of histidine hydrochloride, about 0.3 mg/ml of polysorbate 80, about 90 mg/ml of trehalose, and has a pH of about 5.0.

In some embodiments, the pharmaceutical composition contains about 10 mg/ml of the antibody-drug conjugate represented by formula I, about 0.33 mg/ml of histidine, about 4.79 mg/ml of histidine hydrochloride, about 0.3 mg/ml of polysorbate 80, about 90 mg/ml of trehalose, and has a pH of about 5.0.

In some embodiments, the pharmaceutical composition is a lyophilized injection, and when the lyophilized injection is diluted with water for injection, the diluted pharmaceutical composition contains about 10 mg/ml of the antibody-drug conjugate represented by formula I, about 0.33 mg/ml of histidine, about 4.79 mg/ml of histidine hydrochloride, about 0.3 mg/ml of polysorbate 80, about 90 mg/ml of trehalose, based on the total volume of the diluted pharmaceutical composition, and the pH of the diluted pharmaceutical composition is about 5.0, preferably, the antibody-drug conjugate represented by formula I is selected from SWY2110-JSSW-001, SWY2111-JSSW-001, and SWY2113-JSSW-001, preferably SWY2110-JSSW-001.

In some embodiments, the pharmaceutical composition is an injection solution, and the injection solution contains about 10 mg/ml of the antibody-drug conjugate represented by formula I, about 0.33 mg/ml of histidine, about 4.79 mg/ml of histidine hydrochloride, about 0.3 mg/ml of polysorbate 80, and about 90 mg/ml of trehalose, and the pH of the injection solution is about 5.0, preferably, the antibody-drug conjugate represented by formula I is selected from SWY2110-JSSW-001, SWY2111-JSSW-001, and SWY2113-JSSW-001, preferably SWY2110-JSSW-001.

In other words, in an embodiment of the present invention, the pharmaceutical composition contains about 10

mg/ml of the antibody-drug conjugate represented by formula I, about 0.33 mg/ml of histidine, about 4.79 mg/ml of histidine hydrochloride, about 0.3 mg/ml of polysorbate 80, about 90 mg/ml trehalose, and the balance of water, and the pH of the pharmaceutical composition is about 5.0, preferably, the antibody-drug conjugate represented by formula I is selected from SWY2110-JSSW-001, SWY2111-JSSW-001, and SWY2113-JSSW-001, preferably SWY2110-JSSW-001; or the pharmaceutical composition contains or consists of about 10 parts by weight of the antibody-drug conjugate represented by formula I, about 0.33 parts by weight of histidine, about 4.79 parts by weight of histidine hydrochloride, about 0.3 parts by weight of polysorbate 80, about 90 parts by weight of trehalose, preferably, the antibody-drug conjugate represented by formula I is selected from SWY2110-JSSW-001, SWY2111-JSSW-001, and SWY2113-JSSW-001, preferably SWY2110-JSSW-001.

In some embodiments, after the pharmaceutical composition is dissolved, it contains about 20 mg/ml of the antibody-drug conjugate represented by formula I, about 0.21 mg/ml of histidine, about 2.86 mg/ml of histidine hydrochloride, about 0.2 mg/ml of polysorbate 80, and about 80 mg/ml of sucrose, and has a pH of about 5.0.

In some embodiments, the pharmaceutical composition contains about 20 mg/ml of the antibody-drug conjugate represented by formula I, about 0.21 mg/ml of histidine, about 2.86 mg/ml of histidine hydrochloride, about 0.2 mg/ml of polysorbate 80, and about 80 mg/ml of sucrose, and has a pH of 5.0. In some embodiments, the pharmaceutical composition is a lyophilized injection, and when the lyophilized injection is diluted with water for injection, the diluted pharmaceutical composition contains about 20 mg/ml of the antibody-drug conjugate represented by formula I, about 0.21 mg/ml of histidine, about 2.86 mg/ml of histidine hydrochloride, about 0.2 mg/ml of polysorbate 80, and about 80 mg/ml of sucrose, based on the total volume of the diluted pharmaceutical composition, and the pH of the diluted pharmaceutical composition is about 5.0, preferably, the antibody-drug conjugate represented by formula I is patritumab-JSSW-001.

In some embodiments, the pharmaceutical composition is an injection solution, and the injection solution contains about 20 mg/ml of the antibody-drug conjugate represented by formula I, about 0.21 mg/ml of histidine, about 2.86 mg/ml of histidine hydrochloride, about 0.2 mg/ml of polysorbate 80, and about 80 mg/ml of sucroseAnd the pH of the injection solution is about 5.0, preferably, the antibody-drug conjugate represented by formula I is patritumab-JSSW-001.

In other words, in an embodiment of the present invention, the pharmaceutical composition contains about 20 mg/ml of the antibody-drug conjugate represented by formula I, about 0.21 mg/ml of histidine, about 2.86 mg/ml of histidine hydrochloride, about 0.2 mg/ml of polysorbate 80, about 80 mg/ml of sucrose, and the balance of water, and the pH of the pharmaceutical composition is about 5.0, preferably, the antibody-drug conjugate represented by formula I is patritumab-JSSW-001; or the pharmaceutical composition contains or consists of about 20 parts by weight of the antibody-drug conjugate represented by formula I, about 0.21 parts by weight of histidine, about 2.86 parts by weight of histidine hydrochloride, about 0.2 parts by weight of polysorbate 80, and about 80 parts by weight of sucrose, preferably, the antibody-drug conjugate represented by formula I is patritumab-JSSW-001.

In some embodiments, the antibody-drug conjugate represented by formula I is one of ADC-1, ADC-2, ADC-3, ADC-4, and ADC-5.

In another aspect, the present invention also relates to a method for preparing the pharmaceutical composition, wherein the method comprises the following steps:

S1) preparing the antibody-drug conjugate represented by formula I;
S2) preparing an ultrafiltration-exchanging buffer;
S3) exchanging the antibody-drug conjugate obtained in S1 into the buffer obtained in S2.

Optionally, the method further comprises S4) freeze-drying.

Furthermore, step S2) comprises weighing a buffering agent and a stabilizer according to the amounts in a formula, adding water for injection to dilute to the target formulation volume, stirring and mixing uniformly to obtain the exchanging buffer.

Furthermore, step S3) comprises exchanging a certain amount of the antibody-drug conjugate obtained in S1 into the buffer obtained in S2 through ultrafiltration with an ultrafiltration membrane, diluting the antibody-drug conjugate to the target concentration, and then adding the surfactant according to its volume fraction to obtain a stock solution.

Furthermore, in step S3, the preparation method optionally comprises a step of sterile filtration of the obtained buffer.

The present invention provides use of the pharmaceutical composition mentioned in the present invention in manufacture of a medicament for treating proliferative diseases. The proliferative disease is preferably a disease associated with abnormal expression of HER2, HER3 or EGFR, including cancer. The cancer is selected from breast cancer, ovarian cancer, cervical cancer, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g. small cell lung cancer and non-small cell lung cancer), colon cancer, rectal cancer, colorectal cancer, leukemia (e.g. acute lymphoblastic leukemia, acute myeloid leukemia, acute promyelocytic leukemia, chronic myelocytic leukemia, chronic lymphocytic leukemia), bone cancer, skin cancer, thyroid cancer,

pancreatic cancer or lymphoma (e.g. Hodgkin lymphoma, non-Hodgkin lymphoma or relapsed anaplastic large cell lymphoma).

The present invention provides a method for treating or preventing proliferative diseases, which method includes administering a therapeutically effective dose of the pharmaceutical composition of the present invention to a patient in need thereof; the proliferative disease is preferably a disease associated with abnormal expression of HER2, HER3 or EGFR, including cancer. The cancer is selected from breast cancer, ovarian cancer, cervical cancer, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g. small cell lung cancer and non-small cell lung cancer), colon cancer, rectal cancer, colorectal cancer, leukemia (e.g. acute lymphoblastic leukemia, acute myeloid leukemia, acute promyelocytic leukemia, chronic myelocytic leukemia, chronic lymphocytic leukemia), bone cancer, skin cancer, thyroid cancer, pancreatic cancer or lymphoma (e.g. Hodgkin lymphoma, non-Hodgkin lymphoma or relapsed anaplastic large cell lymphoma).

The present invention provides use of the pharmaceutical composition mentioned in the present invention in manufacture of a medicament for treating a drug-resistant proliferative disease. The drug-resistant proliferative disease is a drug-resistant disease associated with abnormal expression of HER2, HER3 or EGFR, including cancer. The drug-resistant cancer is preferably a drug-resistant cancer caused by mutation in HER2, HER3 or EGFR genes. The cancer is preferably breast cancer, ovarian cancer, cervical cancer, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g. small cell lung cancer and non-small cell lung cancer), colon cancer, rectal cancer, colorectal cancer, leukemia (e.g. acute lymphoblastic leukemia, acute myeloid leukemia, acute promyelocytic leukemia, chronic myelocytic leukemia, chronic lymphocytic leukemia), bone cancer, skin cancer, thyroid cancer, pancreatic cancer or lymphoma (e.g. Hodgkin lymphoma, non-Hodgkin lymphoma or relapsed anaplastic large cell lymphoma), further preferably colon cancer, rectal cancer, lung cancer or pancreatic cancer. Among others, the drug resistance preferably refers to being resistant to receptor tyrosine kinase inhibitors, further preferably resistant to first-generation, second-generation or third-generation EGFR inhibitors, still further preferably resistant to gefitinib, erlotinib, icotinib, afatinib, dacomitinib, osimertinib or almonertinib, especially resistant to gefitinib, afatinib, osimertinib or almonertinib, more preferably resistant to gefitinib or osimertinib.

The present invention provides a method for treating or preventing a drug-resistant proliferative disease, which method includes administering a therapeutically effective dose of the pharmaceutical composition of the present invention to a patient in need thereof; the drug-resistant proliferative disease is a drug-resistant disease associated with abnormal expression of HER2, HER3 or EGFR, including cancer. The drug-resistant cancer is preferably a drug-resistant cancer caused by mutation in HER2, HER3 or EGFR genes. The cancer is preferably breast cancer, ovarian cancer, cervical cancer, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g. small cell lung cancer and non-small cell lung cancer), colon cancer, rectal cancer, colorectal cancer, leukemia (e.g. acute lymphoblastic leukemia, acute myeloid leukemia, acute promyelocytic leukemia, chronic myelocytic leukemia, chronic lymphocytic leukemia), bone cancer, skin cancer, thyroid cancer, pancreatic cancer or lymphoma (e.g. Hodgkin lymphoma, non-Hodgkin lymphoma or relapsed anaplastic large cell lymphoma), further preferably colon cancer, rectal cancer, lung cancer or pancreatic cancer. Among others, the drug resistance preferably refers to being resistant to receptor tyrosine kinase inhibitors, further preferably resistant to first-generation, second-generation or third-generation EGFR inhibitors, still further preferably resistant to gefitinib, erlotinib, icotinib, afatinib, dacomitinib, osimertinib or almonertinib, especially resistant to gefitinib, afatinib, osimertinib or almonertinib, more preferably resistant to gefitinib or osimertinib.

The pharmaceutical composition of the present invention can be used alone or in combination with other anti-tumor agents.

The process for preparing the pharmaceutical composition mentioned in the present invention comprises the following steps:

S1. Reduction of antibody;

Specifically, the antibody medium is exchanged with PBS6.0/EDTA to prepare a solution having an antibody concentration of 10 mg/mL. An appropriate amount of 10mM aqueous TCEP solution and 1M aqueous dipotassium hydrogen phosphate solution are added according to the reduction amount. After confirming that the pH of the solution is proper, the incubation at 37°C is performed for several hours to reduce the disulfide bonds in the antibody.

S2: Conjugation of antibody and linker-drug compound;

Specifically an appropriate amount of a solution (10mM) of the compound dissolved in dimethyl sulfoxide is

added to the above solution at room temperature, and the mixture is mixed evenly and reacted at room temperature for 0.5-4 hours to attach the linker-drug compound to the antibody. Next, 100mM aqueous NAC solution is added, and the mixture is further stirred at room temperature to terminate the reaction.

S3. Purification of antibody-drug conjugates:

Specifically, the reaction solution is purified by ultrafiltration using ultrafiltration centrifuge tubes. 25mM 2-(N-morpholine)ethanesulfonic acid (MES) is added as purification buffer to the reaction solution, pH=6.5. The resulting solution is concentrated to 1-2mL, the purification buffer is added again to make the exchanging factor greater than 1000 times. Thus, the non-conjugated linker-drug compound and other low molecular weight reagents are removed to obtain the purified antibody-drug conjugate.

Compared with known antibody-drug conjugates in the prior art such as DS-8201 or current laboratory samples with better effects on different targets as controls, the antibody-drug conjugates contained in the pharmaceutical composition of the present invention show better anti-cell proliferation effect and significant tumor inhibitory effect. For drug-resistant tumors such as those caused by mutation in HER2, HER3 or EGFR genes, especially those resistant to anti-tumor agents of receptor tyrosine kinase inhibitors, the antibody-drug conjugates of the present invention have superior inhibitory activity to DS-8201, have better stability, including plasma stability and buffer stability, have lower toxicity, have better bystander effect, and have a wider therapeutic window. The present invention carries out the pH-dependent modification of the antibody, and the ADC obtained by conjugating the modified antibody with the linker-drug compound has comparable tumor inhibitory activity to the original ADC in terms of efficacy, and it shows reduced on-target toxicity to normal tissues in terms of safety. The ADC obtained after antibody optimization further expands the therapeutic window.

The pharmaceutical composition of the present invention can be stored for an extended period at 2-8°C under a specified condition, ensuring the stability of the physicochemical properties of the antibody component while minimizing the dissociation of free drugs. Long-term stability studies showed no significant changes in the physicochemical properties of the antibody component, meeting the storage requirements for antibody formulations. Additionally, the drug-to-antibody ratio (DAR) and the distribution of DAR8 in the ADC portion exhibited no significant changes, indicating consistent quality of the conjugated drug during storage. Although some dissociation of free drugs occurred, the amount was minimal and did not pose any safety risks for clinical administration, thereby allowing for long-term storage.

Definition

Unless otherwise specified, the term "antibody-drug conjugate (ADC)" refers to the linkage of an antibody (such as a monoclonal antibody) or an antibody fragment to a biologically active cytotoxin drug through a stable chemical linker compound.

Unless otherwise specified, the term "linker-drug compound" refers to a partial structure in the "antibody-drug conjugate", which is composed of the linker compound and the drug compound.

In the present invention, the linker-drug compound and the antibody are attached by conjugation manners conventionally in the art, including lysine conjugation, reductive disulfide bond conjugation between light and heavy chains, and directional conjugation (Beck, Alain, and Janice M. Reichert. "Antibody-drug conjugates: present and future." MAbs. Vol. 6. No. 1. Taylor & Francis, 2014.; McCombs, Jessica R., and Shawn C. Owen. "Antibody drug conjugates: design and selection of linker, payload and conjugation chemistry." The AAPS journal 17 (2015): 339-351.). In the present invention, the reductive disulfide bond conjugation between the light and heavy chains is preferred, which involves the attachment through the reaction of thiol groups (sulfur atoms of cysteine residues) formed after reducing one or more of disulfide bond sites between the light and heavy chains (two sites between the heavy chains, and two sites between the heavy and light chain).

Unless otherwise specified, the term "alkyl" refers to a monovalent saturated aliphatic hydrocarbon group, including a linear or branched group containing 1-20 carbon atoms, preferably containing 1-10 carbon atoms (namely $C_{1-10}$alkyl), further preferably containing 1-8 carbon atoms ($C_{1-8}$alkyl), more preferably containing 1-6 carbon atoms (namely $C_{1-6}$alkyl). For example "$C_{1-6}$alkyl" means that the group is an alkyl group, and the number of carbon atoms in the carbon chain is between 1 and 6 (specifically 1, 2, 3, 4, 5 or 6). Examples include, but are not limited to methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl and the like. Unless otherwise specified, the term "about" means that the value falls within an acceptable error range of the specific value as determined by a person of ordinary skill in the art, which depends in part on how the value is measured or determined (i.e., the limitations of the measurement system). For example, each occurrence of "about" in the field to which the present invention belongs may imply a range of ±20%, ±15%, ±10%, ±5%, or ±1% of the specific value indicated thereafter.

**Description of the drawings**

Figure 1 shows the decection result for the DAR value of DP001-JSSW-001.
Figure 2 shows the decection result for the DAR value of DP001-JSSW-001.
Figure 3 shows the decection result for the DAR value of partitumab-JSSW-001.
Figure 4 shows the decection result for the DAR value of SWY2110-JSSW-001.
Figure 5 shows the decection result for the DAR value of SWY2111-JSSW-001.
Figure 6 shows the decection result for the DAR value of SWY2112-JSSW-001.
Figure 7 shows the decection result for the DAR value of SWY2113-JSSW-001.
Figure 8 shows the decection result for the DAR value of partitumab-Dxd.
Figure 9 shows the decection result for the DAR value of SWY2110-Dxd.
Figure 10 shows the decection result for the DAR value of SWY2110-Vc MMAE.
Figure 11 shows the effect of DP001-ADC on the tumor volume of HER2-expressing human breast cancer JIMT-1 xenograft tumor in NU/NU mice.
Figure 12 shows the effect of patritumab-ADC on the tumor volume of human lung cancer PC9-GR (gefitinib-resistant cell) xenograft tumor in NU/NU mice.
Figure 13 shows the effect of SWY2110-ADC on the tumor volume of human colorectal cancer DiFi xenograft tumor in NU/NU mice.
Figure 14 shows the effect of SWY2110-ADC on the tumor volume of human lung cancer PC9-GR (gefitinib-resistant cell) xenograft tumor in NU/NU mice.
Figure 15 shows the detection result of the inhibitory activity of SWY2110-ADC on DiFi cells.
Figure 16 shows the detection result of the inhibitory activity of SWY2110-ADC on PC9-GR cells
Figure 17 shows the detection result of the bystander effect of DP001-ADC.
Figure 18 shows the effect of SWY2110-ADC on the tumor volume of human lung cancer PC9-AR (PC9-Del19/T790M/C797S, osimertinib-resistant cell) xenograft tumor in NU/NU mice.
Figure 19 shows the effect of SWY2110-ADC, SWY2111-ADC, SWY2112-ADC, SWY2113-ADC on the tumor volume of human lung cancer NCI-H1975 xenograft tumor in NU/NU mice.
Figure 20 shows the effect of SWY2110-ADC, SWY2111-ADC, SWY2112-ADC, SWY2113-ADC on the tumor volume of human breast cancer MDA-MB-468 xenograft tumor in NOD-SCID mice.
Figure 21 shows the effect of SWY2110-ADC, SWY2111-ADC, SWY2112-ADC, SWY2113-ADC on the tumor volume of human colorectal cancer DiFi xenograft tumor in NU/NU mice.
Figure 22 shows the effect of SWY2110-ADC, SWY2111-ADC, SWY2112-ADC, SWY2113-ADC on the tumor volume of human lung cancer PC9-GR (gefitinib-resistant cell) xenograft tumor in NU/NU mice.
Figure 23 shows the effect of patritumab-ADC on the tumor volume of human lung cancer PC9-DTC (Del19/T790M/C797S, osimertinib-resistant cell) xenograft tumor in NOD-SCID mice.
Figure 24 shows the effect of patritumab-ADC on the tumor volume of human colon cancer SW620 xenograft tumor in NU/NU mice.
Figure 25 shows the detection result of the inhibitory activity of patritumab-ADC on DiFi cells.

**Detailed description**

The present invention is further described below in connection with specific examples. It should be understood that these examples are intended to illustrate the present invention only and are not intended to limit the scope of the present invention. Experimental methods for which no specific conditions are indicated in the following examples are generally in accordance with conventional conditions or in accordance with the conditions recommended by the manufacturers. Unless otherwise defined, all professional and scientific terms used herein have the same meaning as those familiar to those skilled in the art. In addition, any methods and materials similar or equivalent to those described herein may be used in the methods of the present invention. The preferred embodiments and materials shown in this disclosure are illustrative only.

The structures of the compounds according to the present invention are determined by nuclear magnetic resonance (NMR) or/and liquid chromatograph mass spectrometry (LC-MS) or/and liquid chromatography (HPLC). The instrument used for NMR is Bruker Avance III 400 MHz nuclear magnetic resonance apparatus; the instrument used for LC-MS is SHIMADZU LC-20AD-PDA-LCMS-2020; and the instrument used for HPLC is SHIMADZU LC-20AD-PDA high performance liquid chromatograph. The starting materials in the examples of the present invention are known and commercially available, or can be synthesised using those methods known in the art or according to those methods known in the art.

The antibodies of the present invention can be prepared by hybridoma technology described by Kohler et al., Nature (1975) for the first time, or by recombinant DNA method (U.S. Patent 4,816,567, etc.).

Preparation Example

An antibody having two heavy chains with the amino acid sequence represented by SEQ ID NO:9 and two light chains with the amino acid sequence represented by SEQ ID NO:10 was defined as DP001;
An antibody having two heavy chains with the amino acid sequence represented by SEQ ID NO:19 and two light chains with the amino acid sequence represented by SEQ ID NO:20 was defined as patritumab;
An antibody having two heavy chains with the amino acid sequence represented by SEQ ID NO:29 and two light chains with the amino acid sequence represented by SEQ ID NO:30 was defined as SWY2110;
An antibody having two heavy chains with the amino acid sequence represented by SEQ ID NO:34 and two light chains with the amino acid sequence represented by SEQ ID NO:30 was defined as SWY2111;
An antibody having two heavy chains with the amino acid sequence represented by SEQ ID NO:37 and two light chains with the amino acid sequence represented by SEQ ID NO:30 was defined as SWY2112;
An antibody having two heavy chains with the amino acid sequence represented by SEQ ID NO:40 and two light chains with the amino acid sequence represented by SEQ ID NO:30 was defined as SWY2113.

Antibody Sequence Description

1. HER2 antibody DP001 (trastuzumab)

Light chain (SEQ ID NO:10):

DIQMTQSPSSLSASVGDRVTITCRASQDVNTAVAWYQQKPGKAPKLLIYSASFLYSGVPSRFSGS
RSGTDFTLTISSLQPEDFATYYCQQHYTTPPTFGQGTKVEIKRTVAAPSVFIFPPSDEQLKSGTASV

VCLLNNFYPREAKVQWKVDNALQSGNSQESVTEQDSKDSTYSLSSTLTLSKADYEKHKVYACE
VTHQGLSSPVTKSFNRGEC

Heavy chain (SEQ ID NO:9):

EVQLVESGGGLVQPGGSLRLSCAASGFNIKDTYIHWVRQAPGKGLEWVARIYPTNGYTRYADSV
KGRFTISADTSKNTAYLQMNSLRAEDTAVYYCSRWGGDGFYAMDYWGQGTLVTVSSASTKGPS
VFPLAPSSKSTSGGTAALGCLVKDYFPEPVTVSWNSGALTSGVHTFPAVLQSSGLYSLSSVVTVPS
SSLGTQTYICNVNHKPSNTKVDKKVEPKSCDKTHTCPPCPAPELLGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSREEMTKNQVSLTCLVKGFYPSDIAVEWES
NGQPENNYKTTPPVLDSDGSFFLYSKLTVDKSRWQQGNVFSCSVMHEALHNHYTQKSLSLSPGK

DP001 HCDR1: GFNIKDTYIH (SEQ ID NO:1)
DP001 HCDR2: RIYPTNGYTRYAD (SEQ ID NO:2)
DP001 HCDR3: WGGDGFYAMDY (SEQ ID NO:3)
DP001 LCDR1: RASQDVNTAVA (SEQ ID NO:4)
DP001 LCDR2: SASFLYS (SEQ ID NO:5)
DP001 LCDR3: QQHYTTPPT (SEQ ID NO:6)
DP001 HV: SEQ ID NO:7

EVQLVESGGGLVQPGGSLRLSCAASGFNIKDTYIHWVRQAPGKGLEWVARIYPTNGYTRYADSV
KGRFTISADTSKNTAYLQMNSLRAEDTAVYYCSRWGGDGFYAMDYWGQGTLVTVSS

DP001 LV: SEQ ID NO:8

DIQMTQSPSSLSASVGDRVTITCRASQDVNTAVAWYQQKPGKAPKLLIYSASFLYSGVPSRFSGS RSGTDFTLTISSLQPEDFATYYCQQHYTTPPTFGQGTKVEIK

2. HER3 antibody patritumab

Light chain (SEQ ID NO:20):

DIEMTQSPDSLAVSLGERATINCRSSQSVLYSSSNRNYLAWYQQNPGQPPKLLIYWASTRESGVP DRFSGSGSGTDFTLTISSLQAEDVAVYYCQQYYSTPRTFGQGTKVEIKRTVAAPSVFIFPPSDEQL KSGTASVVCLLNNFYPREAKVQWKVDNALQSGNSQESVTEQDSKDSTYSLSSTLTLSKADYEKH KVYACEVTHQGLSSPVTKSFNRGEC

Heavy chain (SEQ ID NO:19):

QVQLQQWGAGLLKPSETLSLTCAVYGGSFSGYYWSWIRQPPGKGLEWIGEINHSGSTNYNPSLK SRVTISVETSKNQFSLKLSSVTAADTAVYYCARDKWTWYFDLWGRGTLVTVSSASTKGPSVFPL APSSKSTSGGTAALGCLVKDYFPEPVTVSWNSGALTSGVHTFPAVLQSSGLYSLSSVVTVPSSSLG TQTYICNVNHKPSNTKVDKRVEPKSCDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDIAVEWESNGQPE NNYKTTPPVLDSDGSFFLYSKLTVDKSRWQQGNVFSCSVMHEALHNHYTQKSLSLSPGK

Patritumab HCDR1: GGSFSGYYWS SEQ ID NO:11
Patritumab HCDR2: EINHSGSTNYNPSLKS SEQ ID NO:12
Patritumab HCDR3: DKWTWYFDL SEQ ID NO:13
Patritumab LCDR1: RSSQSVLYSSSNRNYLA SEQ ID NO:14
Patritumab LCDR2: WASTRES SEQ ID NO:15
Patritumab LCDR3: QQYYSTPRT SEQ ID NO:16
Patritumab HV: SEQ ID NO:17

QVQLQQWGAGLLKPSETLSLTCAVYGGSFSGYYWSWIRQPPGKGLEWIGEINHSGSTNYNPSLK SRVTISVETSKNQFSLKLSSVTAADTAVYYCARDKWTWYFDLWGRGTLVTVSS

Patritumab LV: SEQ ID NO:18

DIEMTQSPDSLAVSLGERATINCRSSQSVLYSSSNRNYLAWYQQNPGQPPKLLIYWASTRESGVP DRFSGSGSGTDFTLTISSLQAEDVAVYYCQQYYSTPRTFGQGTKVEIK

3. EGFR antibody SWY2110

Light chain (SEQ ID NO:30):

EIVLTQSPDFQSVTPKEKVTITCRASQSIGTNIHWYQQKPDQSPKLLIKYASESISGIPSRFSGSGSG TDFTLTINSLEAEDAATYYCQQNNEWPTSFGQGTKLEIK RTVAAPSVFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC

Heavy chain (SEQ ID NO:29):

QVQLQESGPGLVKPSETLSLTCTVSGFSLSNYDVHWVRQAPGKGLEWLGVIWSGGNTDYNTPFT
SRLTISVDTSKNQFSLKLSSVTAADTAVYYCARALDYYDYEFAYWGQGTLVTVSSASTKGPSVFP
LAPSSKSTSGGTAALGCLVKDYFPEPVTVSWNSGALTSGVHTFPAVLQSSGLYSLSSVVTVPSSSL
GTQTYICNVNHKPSNTKVDKRVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAVEWESNGQ
PENNYKTTPPVLDSDGSFFLYSKLTVDKSRWQQGNVFSCSVMHEALHNHYTQKSLSLSPGK

SWY2110 HCDR1: NYDVH SEQ ID NO:21
SWY2110 HCDR2: VIWSGGNTDYNTPFTS SEQ ID NO:22
SWY2110 HCDR3: ALDYYDYEFAY SEQ ID NO:23
SWY2110 LCDR1: RASQSIGTNIH SEQ ID NO:24
SWY2110 LCDR2: YASESIS SEQ ID NO:25
SWY2110 LCDR3: QQNNEWPTS SEQ ID NO:26
SWY2110 HV: SEQ ID NO:27

QVQLQESGPGLVKPSETLSLTCTVSGFSLSNYDVHWVRQAPGKGLEWLGVIWSGGNTDYNTPFT
SRLTISVDTSKNQFSLKLSSVTAADTAVYYCARALDYYDYEFAYWGQGTLVTVSS

SWY2110 LV: SEQ ID NO:28

EIVLTQSPDFQSVTPKEKVTITCRASQSIGTNIHWYQQKPDQSPKLLIKYASESISGIPSRFSGSGSG
TDFTLTINSLEAEDAATYYCQQNNEWPTSFGQGTKLEIK

4. EGFR antibody SWY2111

Light chain (SEQ ID NO:30):

EIVLTQSPDFQSVTPKEKVTITCRASQSIGTNIHWYQQKPDQSPKLLIKYASESISGIPSRFSGSGSG
TDFTLTINSLEAEDAATYYCQQNNEWPTSFGQGTKLEIK
RTVAAPSVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC

Heavy chain (SEQ ID NO:34):

QVQLQESGPGLVKPSETLSLTCTVSGFSLSDYDVHWVRQAPGKGLEWLGVIWSGGNTDYNTPFT
SRLTISVDTSKNQFSLKLSSVTAADTAVYYCARALDDYDYEFAYWGQGTLVTVSSASTKGPSVFP
LAPSSKSTSGGTAALGCLVKDYFPEPVTVSWNSGALTSGVHTFPAVLQSSGLYSLSSVVTVPSSSL
GTQTYICNVNHKPSNTKVDKRVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAVEWESNGQ
PENNYKTTPPVLDSDGSFFLYSKLTVDKSRWQQGNVFSCSVMHEALHNHYTQKSLSLSPGK

SWY2111 HCDR1: DYDVH SEQ ID NO:31
SWY2111 HCDR2: VIWSGGNTDYNTPFTS SEQ ID NO:22
SWY2111 HCDR3: ALDDYDYEFAY SEQ ID NO:32
SWY2111 LCDR1: RASQSIGTNIH SEQ ID NO:24
SWY2111 LCDR2: YASESIS SEQ ID NO:25
SWY2111 LCDR3: QQNNEWPTS SEQ ID NO:26
SWY2111 HV: SEQ ID NO:33

QVQLQESGPGLVKPSETLSLTCTVSGFSLSDYDVHWVRQAPGKGLEWLGVIWSGGNTDYNTPFT
SRLTISVDTSKNQFSLKLSSVTAADTAVYYCARALDDYDYEFAYWGQGTLVTVSS

SWY2111 LV: SEQ ID NO:28

EIVLTQSPDFQSVTPKEKVTITCRASQSIGTNIHWYQQKPDQSPKLLIKYASESISGIPSRFSGSGSG
TDFTLTINSLEAEDAATYYCQQNNEWPTSFGQGTKLEIK

5. EGFR antibody SWY2112

Light chain (SEQ ID NO:30):

EIVLTQSPDFQSVTPKEKVTITCRASQSIGTNIHWYQQKPDQSPKLLIKYASESISGIPSRFSGSGSG
TDFTLTINSLEAEDAATYYCQQNNEWPTSFGQGTKLEIK
RTVAAPSVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC

Heavy chain (SEQ ID NO:37):

QVQLQESGPGLVKPSETLSLTCTVSGFSLSEYDVHWVRQAPGKGLEWLGVIWSGGNTDYNTPFT

SRLTISVDTSKNQFSLKLSSVTAADTAVYYCARALDDYDYEFAYWGQGTLVTVSSASTKGPSVFP
LAPSSKSTSGGTAALGCLVKDYFPEPVTVSWNSGALTSGVHTFPAVLQSSGLYSLSSVVTVPSSSL
GTQTYICNVNHKPSNTKVDKRVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAVEWESNGQ
PENNYKTTPPVLDSDGSFFLYSKLTVDKSRWQQGNVFSCSVMHEALHNHYTQKSLSLSPGK

SWY2112 HCDR1: EYDVH SEQ ID NO:35
SWY2112 HCDR2: VIWSGGNTDYNTPFTS SEQ ID NO:22
SWY2112 HCDR3: ALDDYDYEFAY SEQ ID NO:32
SWY2112 LCDR1: RASQSIGTNIH SEQ ID NO:24
SWY2112 LCDR2: YASESIS SEQ ID NO:25
SWY2112 LCDR3: QQNNEWPTS SEQ ID NO:26
SWY2112 HV: SEQ ID NO:36

QVQLQESGPGLVKPSETLSLTCTVSGFSLSEYDVHWVRQAPGKGLEWLGVIWSGGNTDYNTPFT
SRLTISVDTSKNQFSLKLSSVTAADTAVYYCARALDDYDYEFAYWGQGTLVTVSS

SWY2112 LV: SEQ ID NO:28

EIVLTQSPDFQSVTPKEKVTITCRASQSIGTNIHWYQQKPDQSPKLLIKYASESISGIPSRFSGSGSG
TDFTLTINSLEAEDAATYYCQQNNEWPTSFGQGTKLEIK

6. EGFR antibody SWY2113

Light chain (SEQ ID NO:30):

EIVLTQSPDFQSVTPKEKVTITCRASQSIGTNIHWYQQKPDQSPKLLIKYASESISGIPSRFSGSGSG
TDFTLTINSLEAEDAATYYCQQNNEWPTSFGQGTKLEIK
RTVAAPSVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC

Heavy chain (SEQ ID NO:40):

QVQLQESGPGLVKPSETLSLTCTVSGFSLSHYDVHWVRQAPGKGLEWLGVIWSGGNTDYNTPFT SRLTISVDTSKNQFSLKLSSVTAADTAVYYCARALDDYDYEFAYWGQGTLVTVSSASTKGPSVFP LAPSSKSTSGGTAALGCLVKDYFPEPVTVSWNSGALTSGVHTFPAVLQSSGLYSLSSVVTVPSSSL GTQTYICNVNHKPSNTKVDKRVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKC KVSNKALPAPIEKTISKAKGQPREPQVYTLPPSREEMTKNQVSLTCLVKGFYPSDIAVEWESNGQ PENNYKTTPPVLDSDGSFFLYSKLTVDKSRWQQGNVFSCSVMHEALHNHYTQKSLSLSPGK

SWY2113 HCDR1: HYDVH SEQ ID NO:38
SWY2113 HCDR2: VIWSGGNTDYNTPFTS SEQ ID NO:22
SWY2113 HCDR3: ALDDYDYEFAY SEQ ID NO:32
SWY2113 LCDR1: RASQSIGTNIH SEQ ID NO:24
SWY2113 LCDR2: YASESIS SEQ ID NO:25
SWY2113 LCDR3: QQNNEWPTS SEQ ID NO:26
SWY2113 HV: SEQ ID NO:39

QVQLQESGPGLVKPSETLSLTCTVSGFSLSHYDVHWVRQAPGKGLEWLGVIWSGGNTDYNTPFT SRLTISVDTSKNQFSLKLSSVTAADTAVYYCARALDDYDYEFAYWGQGTLVTVSS

SWY2113 LV: SEQ ID NO:28

EIVLTQSPDFQSVTPKEKVTITCRASQSIGTNIHWYQQKPDQSPKLLIKYASESISGIPSRFSGSGSG TDFTLTINSLEAEDAATYYCQQNNEWPTSFGQGTKLEIK

Example 1

N-((2R,10S)-10-benzyl-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-2-methyl-1,6,9,12,15-pentaoxo-3-oxa-5,8,11,14-tetraazahexadecan-16-yl)-6-(2,5-dioxo-2,5-dihydro-1H-pyrrol-1-yl)hexanamide (JSSW-001)

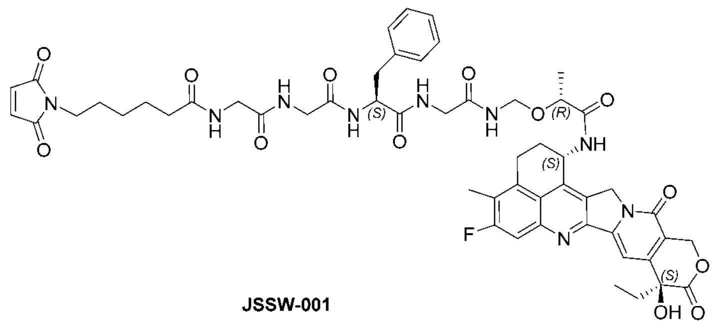

JSSW-001

Scheme I:

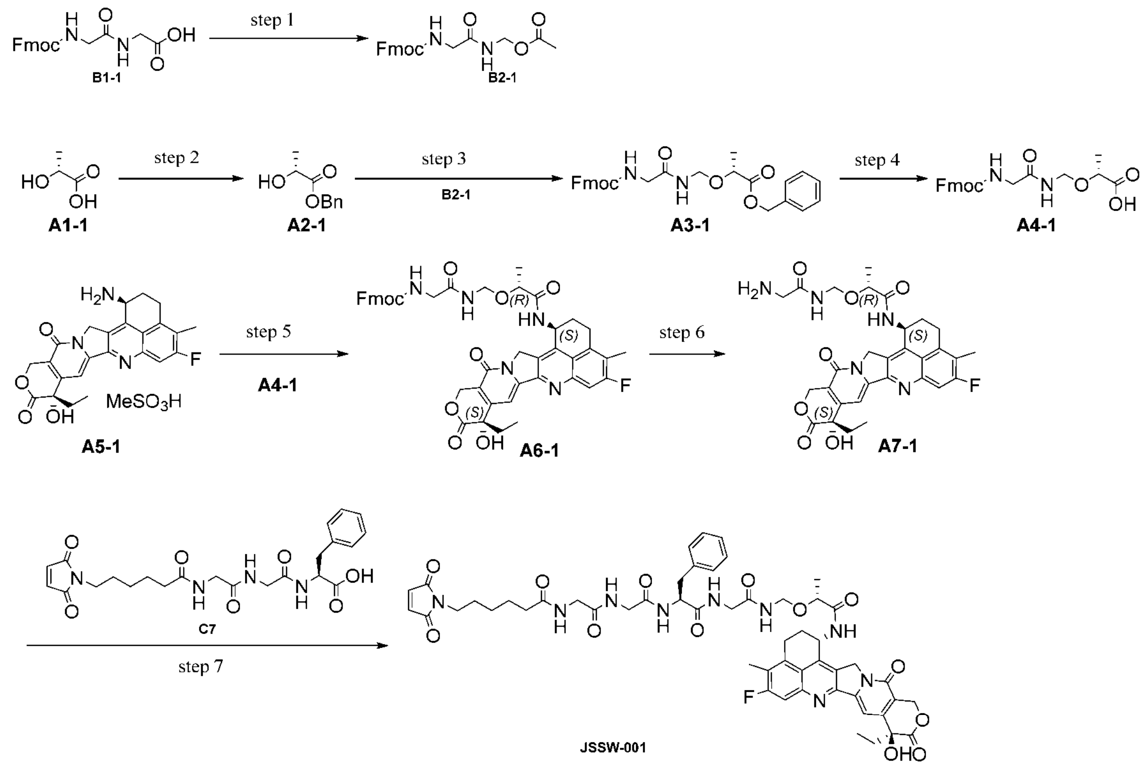


B1-1, B2-1, A1-1, A2-1, A3-1, A4-1, A5-1, A6-1, A7-1, JSSW-001

Step 1: Preparation of compound B2-1: B1-1 (1.0g, 2.28mmol), anhydrous tetrahydrofuran (30mL), toluene (10mL), pyridine (0.5mL), and lead tetraacetate (1.83g, 4.12mmol) were added to a 100mL three-neck flask. The solution turned orange and was heated under reflux. After one hour, the solution became colorless, and a white solid was formed. After a three-hour reaction, the solution was filtered using celite, the solid was rinsed with ethyl acetate, and the filtrate was dried using a rotary evaporator. The obtained crude product was purified by column chromatography to produce a solid (640mg). LC-MS$[M+Na]^+$: m/z 391.1.

Step 2: Preparation of compound A2-1: A1-1 (2.7g, 30.0mmol) and N,N-dimethylformamide (30mL) were added to a 100mL three-neck flask, followed by the addition of potassium carbonate (4.17g, 30.0mmol) and benzyl bromide (2.4mL, 20.0mmol). After reacting at room temperature for 14 hours, the reaction mixture was added dropwise into water. Extraction was carried out using ethyl acetate, and the organic layers were combined. The combined organic layers were washed with saturated brine, dried over anhydrous magnesium sulfate, filtered, and concentrated. The crude product was purified by column chromatography (PE/EA=3:1) to produce 2.61g of the target compound as a colorless liquid.

$^1$H NMR (400 MHz, $CDCl_3$): δ 7.33-7.41 (m, 5H), 5.21 (s, 2H), 4.29-4.35 (m, 1H), 2.77 (d, J = 5.6 Hz, 1H), 1.44 (d, J = 6.8 Hz, 3H).

Step 3: Preparation of compound A3-1: A2-1 (720mg, 4.0mmol), B2-1 (368mg, 1.0mmol), and dichloromethane (5mL) were added to a 100mL three-neck flask. Pyridinium 4-methylbenzenesulfonate (75mg, 0.30mmol) was then added. The reaction mixture was heated to reflux overnight. After the reaction was complete, ethyl acetate (20mL) was added to the reaction mixture, which was then washed with water three times and dried over anhydrous magnesium sulfate. The magnesium sulfate was removed by filtration, and the solution was concentrated. The obtained crude product was purified by column chromatography (PE/EA=2:1) to produce a white solid A3-1 (290mg).

LC-MS$[M+Na]^+$: m/z 511.1. $^1$H NMR (400 MHz, $CDCl_3$): δ 7.77 (d, J = 7.2 Hz, 2H), 7.58 (d, J = 7.6 Hz, 2H), 7.40 (t, J = 7.6 Hz, 2H), 7.26-7.37 (m, 6H), 6.70 (brs, 1H), 5.12-5.30(m, 3H), 4.76-4.89 (m, 2H), 4.45 (d, J = 6.8 Hz, 1H), 4.19-4.27 (m, 2H), 3.69-3.85 (m, 2H), 1.41 (d, J = 6.8 Hz, 3H).

Step 4: Preparation of compound A4-1: A3-1 (291mg, 0.60mmol) dissolved in tetrahydrofuran:ethyl acetate (4mL/2mL) was added to a 100mL three-neck flask, along with Pd/C (10% w/w, 60mg). After purging with hydrogen gas three times, a hydrogen balloon was attached, and the mixture was stirred overnight. After the reaction was complete, the mixture was filtered through celite, rinsed with methanol, and the organic layer was concentrated to produce the product A4-1. LC-MS$[M+Na]^+$: m/z 399.1.

Step 5: Preparation of compound A6-1: A4-1 (50mg, 0.13mmol, 1.3eq), exatecan mesylate (A5-1) (50mg, 0.049mmol, 1.0eq), 2-(7-azabenzotriazol-1-yl)-N,N,N',N'-tetramethyluronium hexafluorophosphate (48mg, 0.13mmol, 1.3eq), and N,N-diisopropylethylamine (36mg, 0.28mmol, 3.0eq) were dissolved in N,N-dimethylformamide (2mL) in a

25mL single-neck flask. The mixture was stirred overnight at room temperature. It was then diluted with ethyl acetate and washed with semi-saturated citric acid, followed by washing with brine, sodium bicarbonate solution, and brine again. The organic layer was dried over anhydrous sodium sulfate, and filtered. The anhydrous sodium sulfate was removed and the filtrate was concentrated. Purification was carried out using prep-TLC with a developer system of (DCM/MeOH=15:1) to produce the product A6-1 (56mg). LC-MS$[M+H]^+$: m/z 816.3.

Step 6: Preparation of compound A7-1: A6-1 (56mg, 0.069mmol), N,N-dimethylformamide (2mL), and piperidine (12mg, 0.14mmol) were added to a 25mL single-neck flask and stirred for 2 hours. The solution was then concentrated to produce a white solid. LC-MS$[M+H]^+$: m/z 594.3.

Step 7: Preparation of compound JSSW-001: A7-1 (0.069mmol), C7 (66mg, 0.14mmol), 2-(7-azabenzotriazol-1-yl)-N,N,N',N'-tetramethyluronium hexafluorophosphate (53mg, 0.14mmol), and N,N-diisopropylethylamine (36mg, 0.28mmol) were dissolved in N,N-dimethylformamide (2mL) in a 25mL single-neck flask. The mixture was stirred for 2 hours at room temperature. It was then diluted with ethyl acetate (30mL) and washed with semi-saturated citric acid, followed by washing with brine, sodium bicarbonate solution, and brine again. The organic layer was dried over anhydrous sodium sulfate, and filtered. The anhydrous sodium sulfate was removed and the filtrate was concentrated. Purification was carried out using prep-TLC with a developer system of (DCM/MeOH=15:1) to produce the product JSSW-001 (21mg).

LC-MS$[M+H]^+$: m/z 1048. $^1$H NMR (400 MHz, DMSO-d6): δ 8.63 (t, J = 6.4 Hz, 1H), 8.53 (d, J = 8.8 Hz, 1H), 8.28 (t, J = 6.0 Hz, 1H), 8.03-8.10 (m, 2H), 7.98 (t, J = 6.0 Hz, 1H), 7.77 (d, J = 11.2 Hz, 1H), 7.30 (s, 1H), 7.14-7.25 (m, 5H), 6.99 (s, 2H), 6.51 (s, 1H), 5.56-5.62 (m, 1H), 5.40 (s, 2H), 5.08-5.23 (m, 2H), 4.66-4.71 (m, 1H), 4.42-4.56 (m, 2H), 4.09-4.15 (m, 1H), 3.54-3.76 (m, 7H), 3.09-3.27 (m, 1H), 2.97-3.02 (m, 1H), 2.67-2.77 (m, 1H), 2.38 (s, 3H), 2.16-2.21 (m, 2H), 2.05-2.10 (m, 2H), 1.83-1.89 (m, 2H), 1.37-1.46 (m, 7H), 1.14-1.33 (m, 4H), 0.87 (t, J = 7.2 Hz, 3H).

Scheme II:

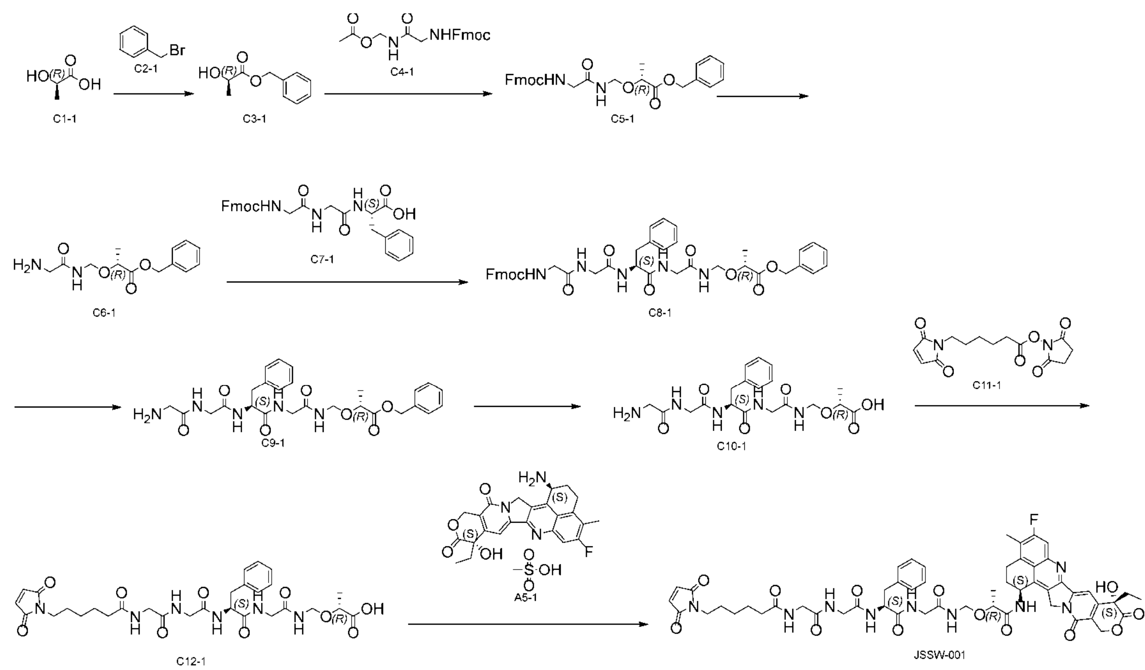
C1-1, C2-1, C3-1, C4-1, C5-1, C6-1, C7-1, C8-1, C9-1, C10-1, C11-1, C12-1, A5-1, JSSW-001

Step 1: Compound C1-1 (8g, 88.86mmol) and C2-1 (22.66g, 133.33mmol) were added to a 250mL reaction flask, followed by the addition of dimethylacetamide (DMAc) (40mL, 5 v/w). The mixture was stirred until dissolved completely, and the temperature was controlled to 0-10°C. Then, N,N-diisopropylethylamine (DIEA) (34.45g, 266.6mmol) was added dropwise. After the dropwise addition, the mixture was allowed to warm naturally to about 25°C and stirred for 16 hours at this temperature. After TLC monitoring showed the complete reaction of the starting materials, the reaction mixture was poured into 120mL of ice water. Methyl tert-butyl ether (MTBE) (40mL) was added, and the mixture was stirred, allowed to stand for phase separation. The aqueous phase was extracted four times with MTBE (40mL*4). The organic phases were combined and washed once with 0.5M hydrochloric acid solution (40mL), once with water (40mL), and twice with saturated brine (40mL*2), dried over anhydrous sodium sulfate, filtered, concentrated, and purified by wet loading and column chromatography using a gradient system of petroleum ether:ethyl acetate = 30:1→20:1→10:1 to produce a light yellow liquid C3-1. LC-MS$[M+Na]^+$: m/z 203.

Step 2: Under nitrogen protection, C4-1 (6g, 16.29mmol, 1.0eq) and C3-1 (3.23g, 17.92mmol, 1.1eq) were

dissolved in tetrahydrofuran (THF) (60mL, 10V/w). The temperature was lowered to 10-15°C, and p-toluenesulfonic acid (TsOH) (281mg, 1.63mmol, 0.1eq) was added. After the completion of the addtion, the reaction was carried out at 14-18°C for 4 hrs under TLC monitoring. After the reaction was complete, the reaction mixture was added to ice water (60mL) and extracted with ethyl acetate (EA) (60mL*3). The organic phases were combined and washed with saturated aqueous $NaHCO_3$ solution (60mL), water (60mL*2), and saturated brine (60mL), dried over anhydrous sodium sulfate, filtered, concentrated by evaporating to dryness, and purified by column chromatography using a gradient system of n-hexane:ethyl acetate = 10:1→5:1→3:1→2:1→1:1 to produce a colorless oily substance C5-1. LC-MS$[M+Na]^+$: m/z 511.

Step 3: Under nitrogen protection, C5-1 (2.8g, 5.74mmol, 1.0eq) was dissolved in DMAc (28mL, 10V/w). The temperature was lowered to 14-18°C, and 1,8-diazabicyclo[5.4.0]undec-7-ene (DBU) (436.6mg, 2.87mmol, 0.5eq) was added dropwise. The mixture was stirred at this temperature for 1.5h. TLC monitoring showed the complete reaction of the starting materials. The temperature was then lowered to 0-10°C, and pyridinium p-toluenesulfonate (PPTS) (721.23mg, 2.87mmol, 0.5eq), 3-ethyl-1-(3-dimethylaminopropyl)carbodiimide hydrochloride (EDCI) (1.1g, 5.74mmol, 1.0eq), 1-hydroxybenzotriazole (HOBT) (775mg, 5.74mmol, 1.0eq), and C7-1 (2.45g, 4.88mmol, 0.85eq) were added. After the completion of the addition, the reaction was carried out at 0-10°C for 3-4 hrs under TLC monitoring until C7-1 was completely reacted. The reaction mixture was added to ice water (100mL) and extracted with 2-methyl tetrahydrofuran (2-Me THF) (100mL*3). The organic phases were combined and washed with 0.5M hydrochloric acid (150mL*2), saturated aqueous $NaHCO_3$ solution (100mL*3), water (100mL), and saturated brine (100mL), dried over anhydrous sodium sulfate, filtered, concentrated by evaporating to dryness, and purified by column chromatography using a gradient system of DCM→DCM:MeOH = 80:1→60:1→50:1→40:1→30:1→20:1 to produce a white foamy solid C8-1 (3.1g, yield 72%). LC-MS$[M+Na]^+$: m/z 772.

Step 4: Under nitrogen protection, C8-1 (3.1g, 4.14mmol, 1.0eq) was dissolved in dichloromethane (DCM) (46.5mL, 15V/w) at a room temperature of 25-30°C. DBU (314.6mg, 2.07mmol, 0.5eq) was added dropwise, and the mixture was stirred at this temperature for 16h. TLC monitoring showed the complete reaction of the starting materials. The reaction mixture was directly subjected to wet column chromatography using a gradient system of DCM→DCM:MeOH = 50:1→30:1→20:1→10:1 to produce a white foamy solid C9-1. LC-MS$[M+H]^+$: m/z 528.

Step 5: Under nitrogen protection, C9-1 (1.7g, 3.2mmol, 1.0eq) was dissolved in water (25.5mL, 15V/w) and tert-butanol (8.5mL, 5V/w). The mixture was stirred until completely dissolved. The atmosphere was replaced with nitrogen gas once, and Pd/C (0.34g, 20% w/w) was added. The atmosphere was replaced with hydrogen gas three times. The hydrogenation reaction was carried out at a room temperature of 25-30°C for 12hrs. LCMS monitoring showed the complete reaction of the starting materials. The reaction mixture was filtered, and the filter cake was washed with water (25mL*2). The filtrate was filtered again, and the tert-butanol was removed by concentration. The aqueous phase was directly lyophilized to produce a white powdery solid C10-1 (1.5g in total, yield 100%). LC-MS$[M+H]^+$: m/z 438.

Step 6: Under nitrogen protection, C10-1 (1.4g, 3.2mmol, 1.0eq) was dissolved in acetonitrile (14mL, 10V/w) and water (28mL, 20V/w). C11-1 (1.86g, 6.097mmol, 1.1eq) was added, and the mixture was stirred until completely dissolved. The temperature was lowered to 0-10°C, and DIEA (330.24mg, 2.56mmol) was added dropwise. After the dropwise addition, the mixture was stirred to react at this temperature for 16h. LC-MS monitoring showed the complete reaction of the starting materials. A buffer solution was prepared by dissolving $Na_2HPO_4$ (260mg) and $NaH_2PO_4$ (5.6g) in 20mL of water under stirring, and the prepared buffer solution was cooled to 0-5°C. The reaction mixture was poured into the cooled buffer solution and extracted with DCM/isopropanol = 4/1 (50mL*4). The organic phases were combined, dried, filtered, concentrated by evaporating to dryness, and purified by column chromatography using a gradient system of DCM→DCM:MeOH = 50:1→30:1→20:1→15:1 to produce a yellow solid C12-1.

Step 7: Under nitrogen protection, A5-1 (547.4mg, 1.03mmol, 1.0eq) was dissolved in 5% anhydrous sodium sulfate in water (6.5mL) and THF (7.8mL). The mixture was stirred but did not dissolve completely. The temperature was lowered to 0-10°C. Then, N-methylmorpholine (NMM) (104mg, 1.03mmol, 1.0eq) was added. After the completion of the addtion, the reaction was allowed to proceed at this temperature for 1 hour. C12-1 (650mg, 1.03mmol, 1.1eq), EDCI (296.2mg, 1.545mmol, 1.5eq), and ethyl cyanoglyoxylate-2-oxime (73.9mg, 0.52mmol, 0.5eq) were added sequentially. After the completion of the addition, the reaction was allowed to proceed at 0-10°C for 4-5 hours. TLC monitoring showed the complete reaction of the starting materials. The reaction mixture was then added to 0.1M hydrochloric acid (13mL, 20V/w) in an ice bath, extracted with 2-methyl tetrahydrofuran (2-Me THF) (13mL*3), and the organic phases were combined, washed once with 0.05M hydrochloric acid (13mL, 20V/w) and twice with water (13mL*2), then dried, filtered, concentrated by evaporating to dryness, and purified by column chromatography using a gradient system of DCM→DCM:MeOH=50:1→30:1→20:1→15:1→12:1 to produce a yellow foamy solid (JSSW-001). LC-MS$[M+H]^+$: m/z 1048.

Example 2 Antibody-drug conjugate (DP001-JSSW-001 ADC, DAR=4.0)

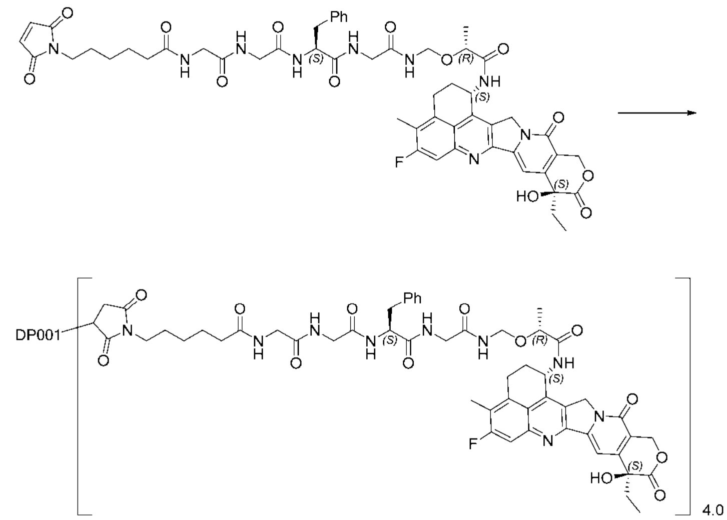

Step 1. Reduction of antibody:

The DP001 antibody medium, which was obtained by purifiction after the expression of plasmids encoding light and heavy chain genes that were transiently transfected into HEK293 cells using known technology, was exchanged with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (17μL; 2.5 equivalents per antibody molecule) and 1M aqueous dipotassium hydrogen phosphate solution (Tianjin Guangfu Science and Technology Development Co., Ltd., 50μL). After confirming that the pH of the solution was within 7.4±0.1, the solution was incubated at 37°C for 1 hour to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (31.28μL; 4.6 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution was added 25mM 2-(N-morpholine)ethanesulfonic acid (MES) as purification buffer, pH=6.5. The resulting solution was concentrated to 1-2mL, and the purification buffer was added again to make the exchanging ratio greater than 1000 times. Thus, the non-conjugated linker-drug compound and other low molecular weight reagents were removed to obtain the purified antibody-drug conjugate (DP001-JSSW-001 ADC, DAR=4.0).

Step 4. Determination of Drug-Antibody Ratio (DAR) of Antibody-Drug Conjugate:

Dithiothreitol with a final concentration of 20 mM was added to 2 mg/kg antibody-drug-conjugate, and the mixture was incubated in a 37°C water bath for 30 minutes. The sample, in which the disulfide bonds between antibody-drug conjugate chains were cleaved, was then used for HPLC analysis. The used HPLC system was the Agilent Technologies 1260 Infinity HPLC, and the used chromatograph column was the PLRP-S column (5 μm particle size; 2.1 mm×50 mm; Agilent Technologies). The column temperature was set at 80°C. The mobile phase A was 0.1% trifluoroacetic acid (TFA) in water, and the mobile phase B was 0.1% TFA in acetonitrile. The injection volume was 10μL, and the gradient program was as follows: 0-3 minutes at 27%-27%, 3-8 minutes at 27%-35%, 8-25 minutes at 35%-43%, 25-26 minutes at 43%-95%, 26-31 minutes at 95%-95%, 31-32 minutes at 95%-27%, and 32-40 minutes at 27%-27%. Compared to the unconjugated light chain (L0) and heavy chain (H0), the hydrophobicity increased with the number of conjugated drugs for the drug-conjugated light chain (L1, conjugated with one drug) and heavy chains (H1, conjugated with one drug; H2, conjugated with two drugs; H3, conjugated with three drugs). Therefore, the elution followed the order of L0, L1, H0, H1, H2, and H3.

The DAR value was calculated based on the peak area at 280 nm. A result of the DAR value is shown in Figure 1.

$$\mathrm{DAR} = \left(\frac{\mathrm{L1}}{\mathrm{L0} + \mathrm{L1}} \times 1 + \frac{\mathrm{H1}}{\mathrm{H0} + \mathrm{H1} + \mathrm{H2} + \mathrm{H3}} \times 1 + \frac{\mathrm{H2}}{\mathrm{H0} + \mathrm{H1} + \mathrm{H2} + \mathrm{H3}} \times 2 + \frac{\mathrm{H3}}{\mathrm{H0} + \mathrm{H1} + \mathrm{H2} + \mathrm{H3}} \times 3\right) \times 2$$

Example 3: Antibody-drug conjugate (DP001-JSSW-001 ADC, DAR=7.32)

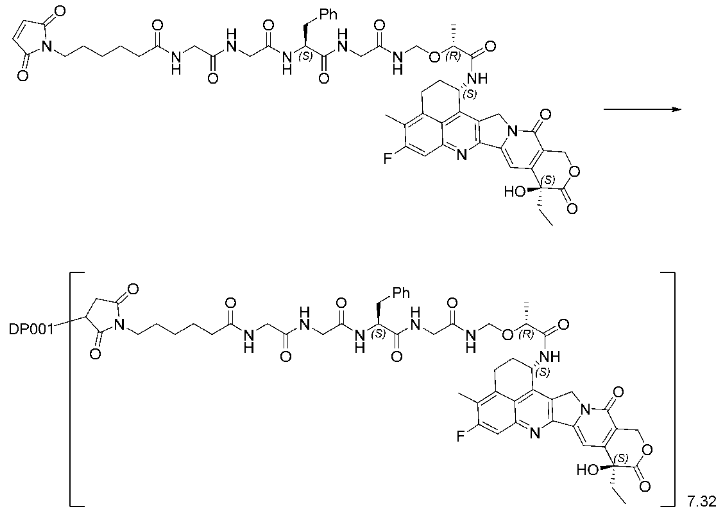

Step 1. Reduction of antibody:

The DP001 antibody medium, which was obtained by purifiction after the expression of plasmids encoding light and heavy chain genes that were transiently transfected into HEK293 cells using known technology, was exchanged with PBS6.0/EDTA to prepare an antibody solution with a concentration of 10 mg/mL. This solution (1.0mL) was put into a 1.5mL polypropylene tube, to which were added 100 mM aqueous TCEP (Bailingwei Science and Technology Co., Ltd.) solution (6.66μL; 10 equivalents per antibody molecule) and 1M dipotassium hydrogen phosphate aqueous solution (Tianjin Guangfu Science and Technology Development Co., Ltd., 18μL). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution was added 25mM 2-(N-morpholine)ethanesulfonic acid (MES) as purification buffer, pH=6.5. The resulting solution was concentrated to 1-2mL, and the purification buffer was added again to make the exchanging ratio greater than 1000 times. Thus, the non-conjugated linker-drug compound and other low molecular weight reagents were removed to obtain the purified antibody-drug conjugate (DP001-JSSW-001 ADC, DAR=7.32). The DAR value determination method was identical to that in step 4 of Example 2. The DAR value result is shown in Figure 2.

Example 4 Antibody-drug conjugate (patritumab-JSSW-001 ADC, DAR=7.36)

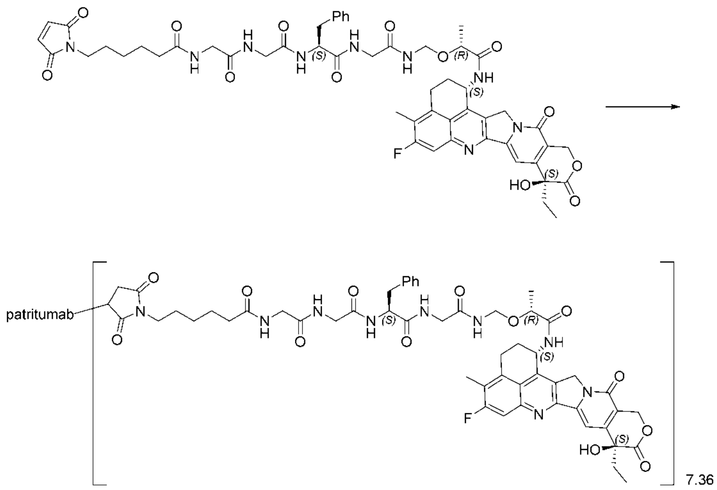

Step 1. Reduction of antibody:

The patritumab antibody medium, which was obtained by purifiction after the expression of plasmids encoding light and heavy chain genes that were transiently transfected into HEK293 cells using known technology, was exchanged with PBS6.0/EDTA to prepare a solution with a concentration of 10 mg/mL. This solution (1.0mL) was put into a 1.5mL polypropylene tube, to which were added 100 mM aqueous TCEP (Bailingwei Science and Technology Co., Ltd.) solution (6.66μL; 10 equivalents per antibody molecule) and 1M dipotassium hydrogen phosphate aqueous solution (Tianjin Guangfu Science and Technology Development Co., Ltd., 18μL). After confirming that the pH of the solution was within 7.0 ±0.1, the solution was incubated at 37°C for 3 hours to reduce the disulfide bonds in the antibody.

Step 2: Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution was added 25mM 2-(N-morpholine)ethanesulfonic acid (MES) as purification buffer, pH=6.5. The resulting solution was concentrated to 1-2mL, and the purification buffer was added again to make the exchanging factor greater than 1000 times. Thus, the non-conjugated linker-drug compound and other low molecular weight reagents were removed to obtain a purified antibody-drug conjugate (patritumab-JSSW-001 ADC, DAR=7.36). The DAR value determination method was identical to that in step 4 of Example 2. The DAR value result is shown in Figure 3.

Example 5 Antibody-drug conjugate (SWY2110-JSSW-001 ADC, DAR=7.12)

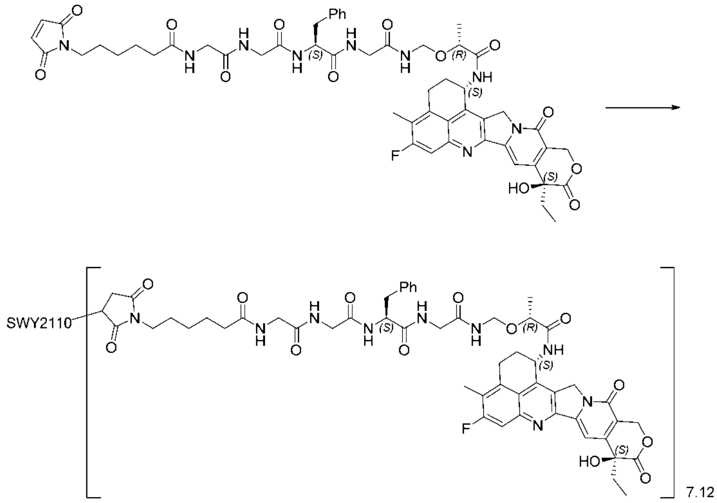

Step 1. Reduction of antibody:

The SWY2110 antibody medium, which was obtained by purifiction after the expression of plasmids encoding light and heavy chain genes that were transiently transfected into HEK293 cells using known technology, was exchanged with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (66.6μL; 10 equivalents per antibody molecule) and 1M aqueous dipotassium hydrogen phosphate solution (Tianjin Guangfu Science and Technology Development Co., Ltd.). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution were added L-histidine 0.89mg/ml and L-histidine hydrochloride 4.04mg/ml as buffer, Tween80 0.03%, and sucrose 90 mg/ml, at pH=5.5. The non-conjugated linker-drug and other low molecular weight reagents were removed to produce a purified antibody-drug conjugate (SWY2110-JSSW-001 ADC, DAR=7.12). The DAR value determination method was identical to that in step 4 of Example 2. The DAR value result is shown in Figure 4.

Example 6 Preparation of pH-dependent antibodies

SWY2110 and EGFR protein were modelled and docked using computer simulation-assisted technology to obtain an antigen-antibody complex. Amino acids within 3Å of the complex underwent D, E, and H scanning, which produced several pH-dependent antibodies that had a reduced affinity at pH=7.4 but basically had an unchanged affinity or had a lower decrease in affinity at pH=6.0. Several obtained antibody sequence genes were cloned into mammalian expression vectors and expressed through transfection of HEK-293 cells. After the completion of the expression, the expression supernatants were collected, and the antibodies were purified using an AKTA system equipped with Protein A pre-packed column to produce the desired antibodies.

Example 7: Antibody affinity determination

MDA-MB-468 cells were digested into single cells using 0.25% trypsin-EDTA and divided equally into multiple aliquots, with each aliquot containing more than $10^6$ cells. SWY2110 (control) and antibodies obtained from Example 9

(diluted with PBS buffer solutions at pH=7.4 and pH 6.0, respectively) were added to the cells at 300μL with serial 5-fold gradient dilutions. The mixtures were incubated at 4°C for 1 hour and then centrifuged at 1000 rpm for 4 minutes to remove the supernatant. The cells were washed twice with PBS solution, followed by the addition of 200μL of FITC-labelled mouse anti-human Fc secondary antibody with a dilution factor of 1:200. The mixtures were incubated at 4°C for another hour, and then centrifuged at 1000 rpm for 4 minutes to remove the supernatant. Fluorescence intensity was detected using flow cytometry, and data were saved and analyzed using relevant software. The results of the antibody affinity determination after screening are shown in Table 1. The affinity determination results of the optimized antibodies with target antigens after the pH-dependent optimization showed that at pH=7.4, the affinity of the optimized antibodies SWY2111, SWY2112, and SWY2113 decreased significantly (about 3.0 to 9.7 times) compared to SWY2110; and at pH=6.0, the affinity of SWY2111 and SWY2112 remained basically unchanged (1.2 to 1.3 times) compared to SWY2110, while the affinity of the antibody SWY2113 decreased by 6.6 times.

Table 1: Result of Antibody Affinity Determination

| FACS ($EC_{50}$) | SWY2110 | SWY2111 | SWY2112 | SWY2113 |
|---|---|---|---|---|
| pH7.4(nM) | 0.49 | 1.45 | 2.35 | 4.76 |
| pH6.0(nM) | 0.51 | 0.60 | 0.67 | 3.39 |

Example 8 Antibody-drug conjugate (SWY2111-JSSW-001 ADC, DAR=7.48)

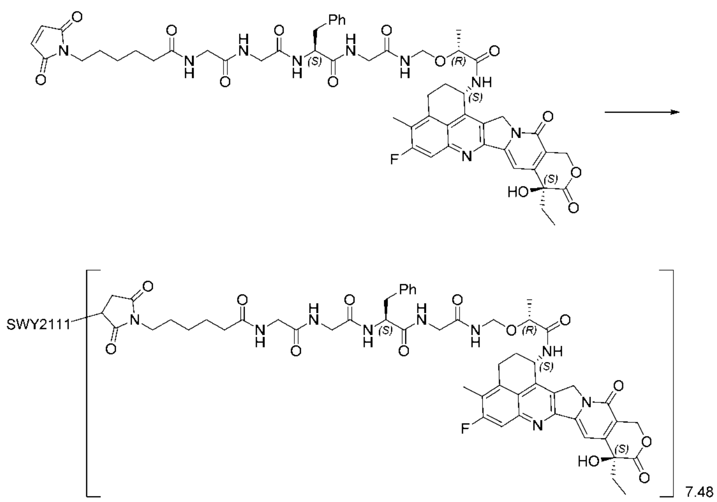

Step 1. Reduction of antibody:

The obtained SWY2111 antibody medium was exchanged with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (66.6μL; 10 equivalents per antibody molecule) and 1M aqueous dipotassium hydrogen phosphate solution (Tianjin Guangfu Science and Technology Development Co., Ltd.). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution were added L-histidine 0.89mg/ml and L-histidine hydrochloride 4.04mg/ml as buffer, Tween80 0.03%, and sucrose 90 mg/ml, at pH=5.5. The non-conjugated linker-drug and other low molecular weight reagents were removed to produce a purified antibody-drug conjugate (SWY2111-JSSW-001 ADC, DAR=7.48). The DAR value determination method was identical to that in step 4 of Example 2. The DAR value result is shown in Figure 5.

Example 9 Antibody-drug conjugate (SWY2112-JSSW-001 ADC, DAR=7.51)

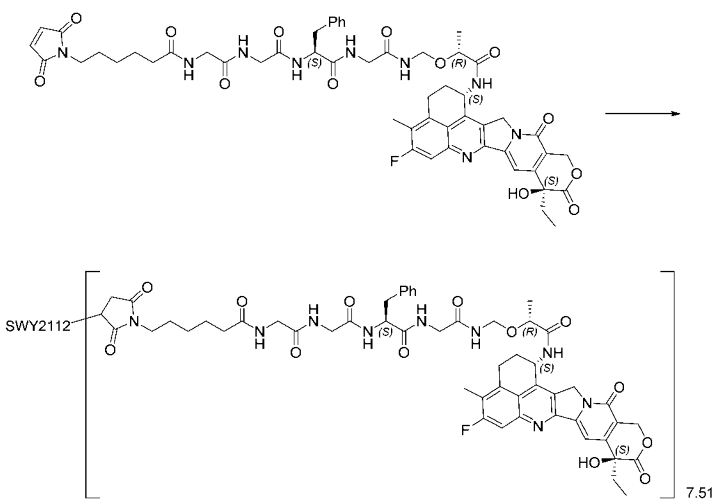

Step 1. Reduction of antibody:

The obtained SWY2112 antibody medium was exchanged with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (66.6μL; 10 equivalents per antibody molecule) and 1M aqueous dipotassium hydrogen phosphate solution (Tianjin Guangfu Science and Technology Development Co., Ltd.). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution were added L-histidine 0.89mg/ml and L-histidine hydrochloride 4.04mg/ml as buffer, Tween80 0.03%, and sucrose 90 mg/ml, at pH=5.5. The non-conjugated linker-drug and other low molecular weight reagents were removed to produce a purified antibody-drug conjugate (SWY2112-JSSW-001 ADC, DAR=7.51). The DAR value determination method was identical to that in step 4 of Example 2. The DAR value result is shown in Figure 6.

Example 10 Antibody-drug conjugate (SWY2113-JSSW-001 ADC, DAR=7.50)

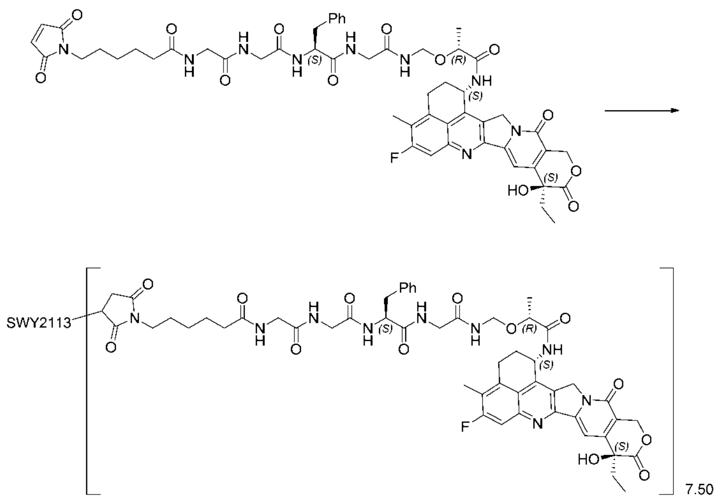

Step 1. Reduction of antibody:

The obtained SWY2113 antibody medium was exchanged with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (66.6μL; 10 equivalents per antibody molecule) and 1M aqueous dipotassium hydrogen phosphate solution (Tianjin Guangfu Science and Technology Development Co., Ltd.). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution were added L-histidine 0.89mg/ml and L-histidine hydrochloride 4.04mg/ml as buffer, Tween80 0.03%, and sucrose 90 mg/ml, at pH=5.5. The non-conjugated linker-drug and other low molecular weight reagents were removed to produce a purified antibody-drug conjugate (SWY2113-JSSW-001 ADC, DAR=7.50). The DAR value determination method was identical to that in step 4 of Example 2. The DAR value result is shown in Figure 7.

Reference Example 1 Antibody-drug conjugate (DP001-DXD ADC, DAR=4.3)

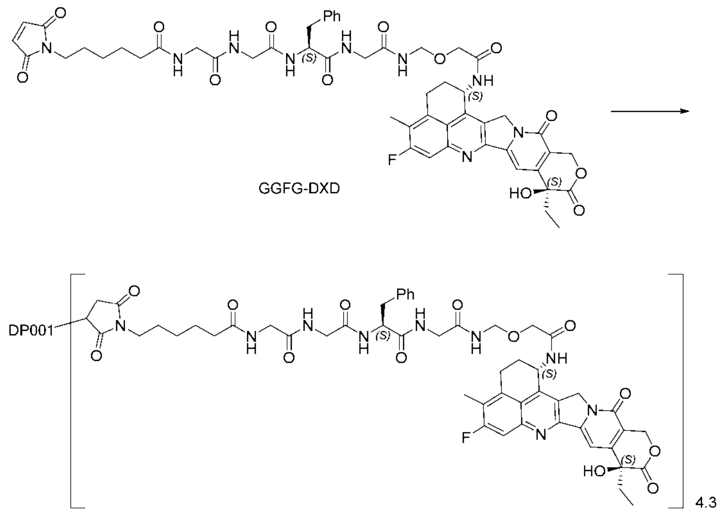

GGFG-DXD

Step 1. Reduction of antibody:

The DP001 antibody medium, which was obtained by purifiction after the expression of plasmids encoding light and heavy chain genes that were transiently transfected into HEK293 cells using known technology, was exchanged with PBS6.0/EDTA to prepare a solution with a concentration of 10 mg/mL. This solution (1.0mL) was put into a 1.5mL polypropylene tube, to which were added 10 mM aqueous TCEP (Bailingwei Science and Technology Co., Ltd.) solution (17μL; 2.5 equivalents per antibody molecule) and 1M aqueous dipotassium hydrogen phosphate solution (Tianjin Guangfu Science and Technology Development Co., Ltd., 50μL). After confirming that the pH of the solution was within 7.4 ±0.1, the solution was incubated at 37°C for 1 hour to reduce the disulfide bonds in the antibody.

Step 2. Conjugation of antibody and linker-drug compound:

To the above solution was added a 10 mM solution of commercially available Deruxtecan (CAS No.:1599440-13-7) in dimethylsulfoxide (31.28μL; 4.6 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution was added 25mM 2-(N-morpholine)ethanesulfonic acid (MES) as purification buffer, pH=6.5. The resulting solution was concentrated to 1-2mL, and the purification buffer was added again to make the exchanging ratio greater than 1000 times. Thus, the non-conjugated linker-drug compound and other low molecular weight reagents were removed to obtain the purified antibody-drug conjugate (DP001-DXD ADC, DAR=4.3). The method for measuring the drug-antibody ratio (DAR) of the antibody-drug conjugate was identical to that in step 4 of Example 2.

Reference Example 2 Antibody-drug conjugate (DP001-DXD ADC, DAR=7.0)

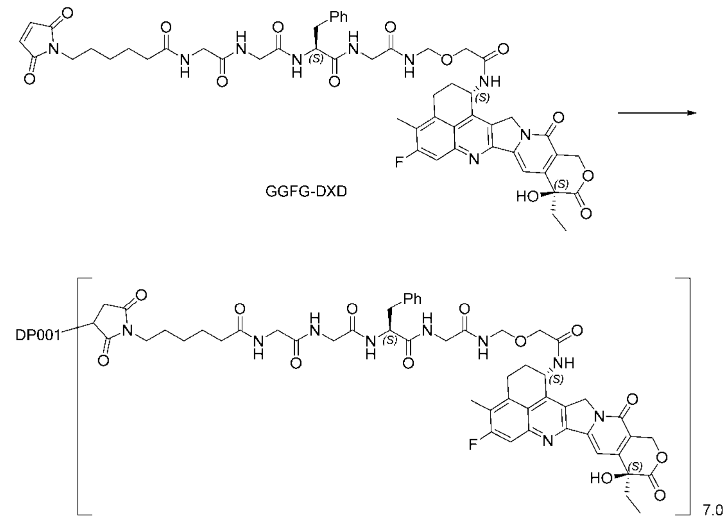

GGFG-DXD

Step 1. Reduction of antibody:

The DP001 antibody medium was exchanged with PBS6.0/EDTA to prepare a solution having an antibody concentration of 10 mg/mL. This solution (1.0mL) was put into a 1.5mL polypropylene tube, to which were added 100 mM aqueous TCEP (Bailingwei Science and Technology Co., Ltd.) solution (6.66μL; 10 equivalents per antibody molecule) and 1M dipotassium hydrogen phosphate aqueous solution (Tianjin Guangfu Science and Technology Development Co., Ltd., 18μL). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of commercially available Deruxtecan (CAS No.:1599440-13-7) in dimethylsulfoxide (87μL; 13 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution was added 25mM 2-(N-morpholine)ethanesulfonic acid (MES) as purification buffer, pH=6.5. The resulting solution was concentrated to 1-2mL, and the purification buffer was added again to make the exchanging ratio greater than 1000 times. Thus, the non-conjugated linker-drug compound and other low molecular weight reagents were removed to obtain the purified antibody-drug conjugate (DP001-DXD ADC, DAR=7.0). The method for measuring the drug-antibody ratio (DAR) of the antibody-drug conjugate was identical to that in step 4 of Example 2.

Reference Example 3 Antibody-drug conjugate (patritumab-DXD ADC, DAR=7.3)

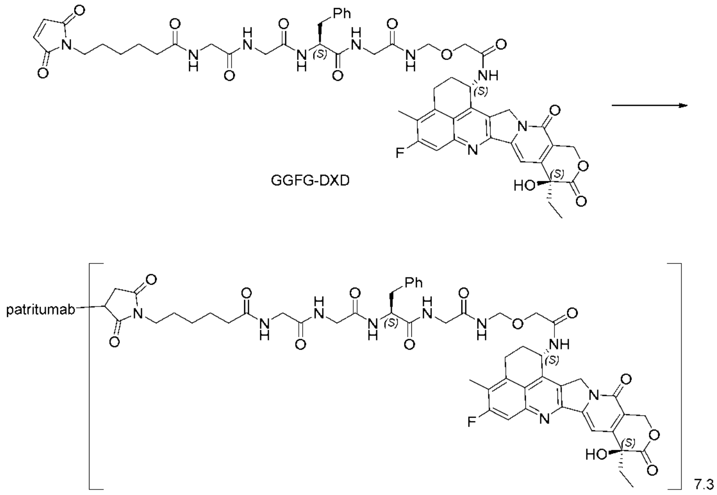

GGFG-DXD

Step 1. Reduction of antibody:

The patritumab antibody medium, which was obtained by purifiction after the expression of plasmids encoding light and heavy chain genes that were transiently transfected into HEK293 cells using known technology, was exchanged with PBS6.0/EDTA to prepare an antibody solution with a concentration of 10 mg/mL. This solution (1.0mL) was put into a 1.5mL polypropylene tube, to which were added 100 mM aqueous TCEP (Bailingwei Science and Technology Co., Ltd.) solution (6.66μL; 10 equivalents per antibody molecule) and 1M dipotassium hydrogen phosphate aqueous solution (Tianjin Guangfu Science and Technology Development Co., Ltd., 18μL). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of commercially available Deruxtecan (CAS No.:1599440-13-7) in dimethylsulfoxide (87μL; 13 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution was added 25mM 2-(N-morpholine)ethanesulfonic acid (MES) as purification buffer, pH=6.5. The resulting solution was concentrated to 1-2mL, and the purification buffer was added again to make the exchanging ratio greater than 1000 times. Thus, the non-conjugated linker-drug compound and other low molecular weight reagents were removed to obtain the purified antibody-drug conjugate (patritumab-DXD ADC, DAR=7.3). The DAR value determination method was identical to that in step 4 of Example 2. The DAR value result is shown in Figure 8.

Reference Example 4 Antibody-drug conjugate (SWY2110-DXD ADC, DAR=6.93)

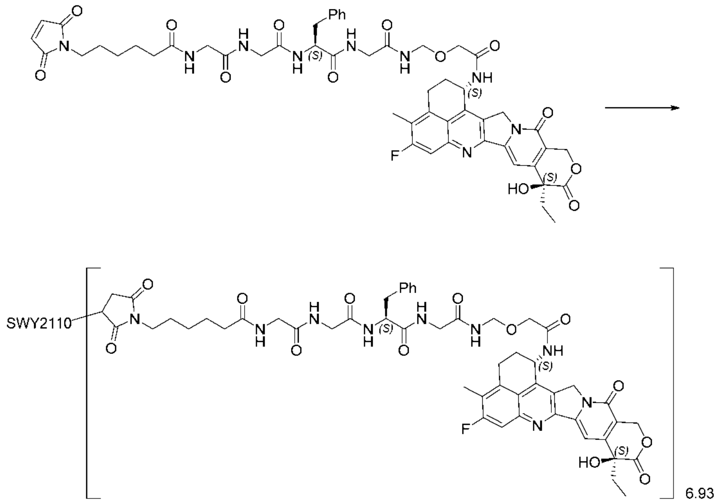

Step 1. Reduction of antibody:

The SWY2110 antibody medium, which was obtained by purifiction after the expression of plasmids encoding light and heavy chain genes that were transiently transfected into HEK293 cells using known technology, was exchanged with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (66.6μL; 10 equivalents per antibody molecule) and 1M aqueous dipotassium hydrogen phosphate solution (Tianjin Guangfu Science and Technology Development Co., Ltd.). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of commercially available Deruxtecan (CAS No.:1599440-13-7) in dimethylsulfoxide (87μL; 13 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution was added 25mM 2-(N-morpholine)ethanesulfonic acid (MES) as purification buffer, pH=6.5. The resulting solution was concentrated to 1-2mL, and the purification buffer was added again to make the exchanging ratio greater than 1000 times. Thus, the non-conjugated linker-drug compound and other low molecular weight reagents were removed to obtain the purified antibody-drug conjugate (SWY2110-DXD ADC, DAR=6.93). The DAR value determination method was identical to that in step 4 of Example 2. The DAR value result is shown in Figure 9.

Reference Example 5 Antibody-drug conjugate (SWY2110-VC MMAE ADC, DAR=4.53)

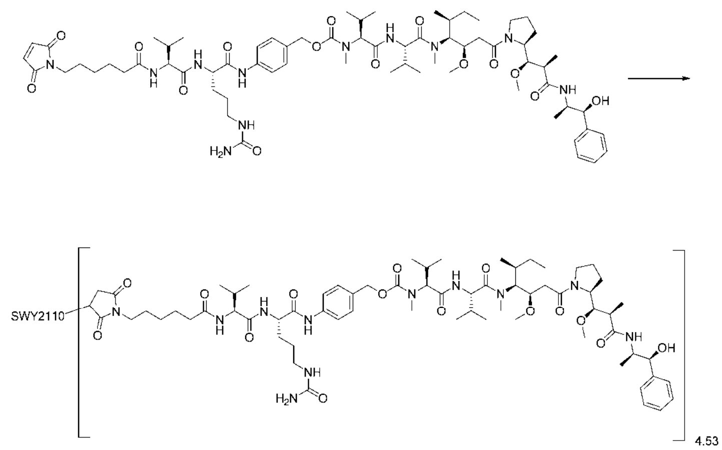

Step 1. Reduction of antibody:

The SWY2110 antibody medium, which was obtained by purifiction after the expression of plasmids encoding light and heavy chain genes that were transiently transfected into HEK293 cells using known technology, was exchanged with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (17μL; 2.5 equivalents per antibody molecule) and 1M aqueous dipotassium hydrogen phosphate solution (Tianjin Guangfu Science and Technology Development Co., Ltd.). After confirming that the pH of the solution was within 7.4±0.1, the solution was incubated at 37°C for 1 hour to reduce the disulfide bonds in the antibody.

Step 2. Conjugation of antibody and linker-drug compound:

To the above solution was added a 10 mM solution of commercially available mc-vc-PAB-MMAE (CAS No.:646502-53-6, abbrev. Vc MMAE) in dimethylsulfoxide (31.28μL; 4.6 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel® Regenerated Cellulose (30kDa MWCO), 15mL sample volume). To the reaction solution was added 25mM 2-(N-morpholine)ethanesulfonic acid (MES) as purification buffer, pH=6.5. The resulting solution was concentrated to 1-2mL, and the purification buffer was added again to make the exchanging ratio greater than 1000 times. Thus, the non-conjugated linker-drug compound and other low molecular weight reagents were removed to obtain the purified antibody-drug conjugate (SWY2110-VC MMAE ADC, DAR=4.53).

Step 4. Determination of Drug-Antibody Ratio (DAR) of Antibody-Drug Conjugate:

10 μg SWY2110-Vc MMAE ADC sample was loaded onto the chromatographic column (2.5 μm particle size; 4.6 mm × 10 cm; TSKgel Butyl-NPR). The elution mobile phase A was 20 mM PB, pH 7.0, 1.5 M $(NH_4)_2SO_4$ aqueous solution, and the mobile phase B was 20 mM PB, pH 7.0, 25% isopropanol solution. The flow rate was controlled at 0.8mL/min, the column temperature was 30°C, the detection wavelength was 280 nm, and the gradient program was set as follows: 0-20 minutes, mobile phase B from 0 to 100%; 20-25 minutes, 100% mobile phase B; and 25-30 minutes, 100% mobile phase A. Being non-conjugated was DAR0, being conjugated with 2 small molecules was DAR2, being conjugated with 4 small molecules was DAR4, being conjugated with 6 small molecules was DAR6, and being conjugated with 8 small molecules was DAR8. Therefore, elution was performed in the order of DAR0, DAR2, DAR4, DAR6, and DAR8. The DAR value was calculated based on the peak area at 280nm, where A represents the percentage of each peak area. A result of the DAR value is shown in Figure 10.

$$DAR=2\times A_{DAR2}+4\times A_{DAR4}+6\times A_{DAR6}+8\times A_{DAR8}$$

Biological Examples

Assay 1: Activity of antibody-drug conjugate in cells in vitro

1. Assay object:

The object of this assay was to detect the inhibitory activity of the antibody-drug conjugate of the present invention on the in-vitro proliferation of SK-BR-3 (ATCC/HTB-30) tumor cells, human colorectal cancer cells DiFi, human lung cancer gefitinib resistant cell PC9-GR, or human lung cancer mutant cell PC-9 (Dell9-T790M-C797S). The cells were treated in vitro with compounds of different concentrations. After culture, resazurin was added to read the fluorescence values of ex550nm/em610nm, and the data were fitted with four parameters to obtain the $IC_{50}$ values, thereby calculating the biological activities of the compound.

2. Assay materials and equipments:

Materials

| Product Name | Manufacturer/Item Number |
|---|---|
| SK-BR-3 cell line | ATCC/HTB-30 |
| DiFi cell line | Shanghai Jinmante Biotechnology Co., Ltd |
| PC9-GR cell line | Nanjing Cobioer Biosciences Co., Ltd |
| MTT Assay Kit | Abcam |
| DMEM, high glucose | Hyclone/SH30022.01 |
| 0.25% Trypsin-EDTA | Gibco/25200-072 |
| fetal bovine serum (FBS) | Gibco/16000-044 |
| 100×bispecific antibody | Gibco/15240-062 |
| black-walled, transparent-bottom tissue culture plate | Corning/3603 |
| resazurin sodium | Sigma Aldrich/199303-25G |

Equipments

| Name | Model | Manufacturer |
|---|---|---|
| Biosafety cabinet | 1300 series A2 model | Thermo Fisher Scientific |
| $CO_2$ incubator | 3111 model | Thermo Fisher Scientific |
| Inverted microscope | CKX31 | Olympus |
| Microplate reader | Infinite M200 | Tecan Company |
| Micro-oscillator | MM-I model | Shanghai Yarong Biochemistry Instrument Factory |

3. Assay operation procedure 1:

3.1 Culture medium: DMEM, 10% FBS, 1×bispecific antibody

3.2 Cell culturing: Frozen SK-BR-3/MDA-MB-468 cells were taken out of liquid nitrogen and revived in a $75cm^2$ culture vessel for culturing. The cells were grown until the confluency of cells reached >75%, and had undergone at least 3 passages.

3.2.1 If cell passage was not performed, the culture medium should be replaced every 3-4 days.
3.2.2 If the cell expansion was needed, the cells were passaged and inoculated into a larger culture vessel, ensuring that the confluency of cells reached >75% before use in assays.
3.3 Cell Collection: SK-BR-3/MDA-MB-468 cells were collected when the culture vessel was nearly full.
3.3.1 The culture medium was discarded, and the cells were washed with PBS to remove dead cells and residual culture medium.
3.3.2 2-3mL of 0.25% Trypsin-EDTA was added, the culture vessel was gently shaken, and then incubated at 37°C for 2-3 minutes to digest the cells.
3.3.3 5mL of the culture medium was immediately added to the culture vessel and gently pipetted up and down to spread the cells.
3.3.4 The cell suspension was transferred to a sterile centrifuge tube and centrifuged at 200×g for 3 minutes.
3.3.5 The culture medium in the tube was discarded, and 5-10mL of the fresh culture medium was added to resuspend the cells.
3.4 Cell density determination: The cells were counted with a cell counting plate under a microscope.
3.5 Assay plate inoculation
3.5.1 Cells were diluted to $1\times10^5$ cells/mL with the culture medium and seeded at 100μL/well in the assay plate (except rows A and H).
3.5.2 120μL of the culture medium was added to each well in rows A and H as a blank.
3.5.3 The incubation was performed at 37°C with 5% $CO_2$ for 4-6 hrs to allow the cells to adhere to the wall.

3.6 Preparation of diluted compound samples:

The compound was diluted to an initial concentration of 18 μg/ml, and then further diluted by a factor of 3 to obtain a total of 11 gradient solutions. The 12th column was left as a blank control.

3.7 Drug treatment

3.7.1 20μL of drug diluent from each of the 1st to 11th columns of the dilution plate was taken and added to the corresponding column of the assay plate.
3.7.2 20μL of fresh culture medium was added to each well in the 12th column and the A and H rows.
3.7.4 The plate was gently shaken on a plate shaker for 10-15 seconds, and then the culture plate was incubated at 37°C for 3 days.

3.8 Analyses

3.8.1 After incubation, 20μL of 0.03% resazurin (1×PBS diluted) was added to each well and gently shaken for 10-15 seconds.
3.8.2 After incubating at 37°C for 3-4 hours, the plate was read using a microplate reader with the following parameters:

Exciting light: 550nm
Emitting light: 610nm
Integration time: 50
Shaking: 15 seconds, vortex
Reading: top reading
Number of reading/well: 1
Gain: optimization setting (between 35 and 42)

If reading multiple plates, it was ensured that the gain setting was consistent for all plates.

3.8.3 EXCEL was used to plot the data and fit the $IC_{50}$ values for the reference standards and samples.
3.8.3.1 Model 201 was used to plot the data points.
3.8.3.2 For Fit parameters, the following commands were used:

a. A: Pre-fitted
b. B: Pre-fitted
c. C: Pre-fitted
d. D: Pre-fitted
e. No constraints for all

3.8.3.3 The output parameter C represented the $IC_{50}$ in unit of ng/mL.

The results are shown in Table 2.

Assay operation procedure 2:

This assay verified the inhibitory effects of SWY2110 monoclonal antibody (SWY2110 mAb), SWY2110-DXD ADC (DAR=6.93), and SWY2110-JSSW-001 ADC (DAR=7.12) on the proliferation of two cell lines, DiFi (human colorectal cancer cells) and PC9-GR (gefitinib-resistant human lung cancer cells). The specific concentration settings were as follows:

DiFi: SWY2110 mAb, SWY2110-DXD ADC (DAR=6.93), and SWY2110-JSSW-001 ADC (DAR=7.12) started at a concentration of 3000 ng/ml, with a 3x serial dilution of the drug, resulting in 10 concentrations: 3000, 1000, 333.33, 111.11, 37.04, 12.35, 4.12, 1.37, 0.46, 0.15 ng/ml, respectively. The drug exposure lasted for 144 hours.
PC9-GR: SWY2110 mAb, SWY2110-DXD ADC (DAR=6.93), SWY2110-JSSW-001 ADC (DAR=7.12) started at a concentration of 1000 ng/ml, with a 5x serial dilution of the drug, resulting in 8 concentrations: 1000, 200, 40, 8, 1.6, 0.32, 0.64, 0.013 ng/ml, respectively. The drug exposure lasted for 144 hours.

Cells in logarithmic growth phase were seeded in a 96-well plate (100μL/well) at a certain number. After the adherent cells had adhered for 24 hours, 100μL of culture medium containing different concentrations of SWY2110 mAb, SWY2110-DXD ADC (DAR=6.93), or SWY2110-JSSW-001 ADC (DAR=7.12) was added to each well. Three replicate wells were set for each drug concentration, along with corresponding blank wells (only containing culture medium) and normal wells (drug concentration of 0). After 144 hours of drug exposure, a MTT working solution (5 mg/mL) was added to each well (20μL per well). The plates were incubated at 37°C for 4 hours, and then the supernatant was discarded. DMSO (analytical grade) was added (150μL per well), and the plates were shaken on a microplate shaker to mix thoroughly. The plates were then wiped clean, and the optical density (OD) was measured at 550 nm using the microplate reader.
The inhibition rate of the cell growth was calculated with the following equation:

$$\text{Inhibition Rate}(\%) = (\text{OD Value}_{\text{normal well}} - \text{OD Value}_{\text{drug well}})/(\text{OD Value}_{\text{normal well}} - \text{OD Value}_{\text{blank well}}) \times 100\ \%$$

Based on the inhibition rates at different concentrations, the half-maximal inhibitory concentration ($IC_{50}$) of the drug was calculated using SPSS 19.0. The results are shown in Table 3, and Figures 15 and 16.

Assay data:

Table 2: $IC_{50}$ value of DP001-ADC against SK-BR-3 cells

| Compound No. | $IC_{50}$ value (ng/mL) |
|---|---|
| DP001-DXD ADC (DAR=7.0) | 7.2481 |
| DP001-JSSW-001 ADC (DAR=7.32) | 5.1824 |

It can be seen from the data recorded in Table 2 that the antibody-drug DP001-JSSW-001 ADC (DAR=7.12) of the present invention had a lower $IC_{50}$ value against SK-BR-3 cells than DP001-DXD ADC (DAR=6.93), showing better anti-cell proliferation effect.

Table 3: $IC_{50}$ value of SWY2110-ADC against two cell lines (ng/mL)

| Cell | SWY2110 mAb | SWY2110-DXD ADC (DAR=6.93) | SWY2110-JSSW-001 ADC (DAR=7.12) |
|---|---|---|---|
| DiFi | 43.94 | 14.85 | 15.84 |

(continued)

| Cell | SWY2110 mAb | SWY2110-DXD ADC (DAR=6.93) | SWY2110-JSSW-001 ADC (DAR=7.12) |
|---|---|---|---|
| PC9-GR | - | 1.00 | 1.12 |
| wherein "--" represents not showing anti-cell proliferation effect. | | | |

It can be seen from the data in Table 3 that the antibody-drug conjugates SWY2110-JSSW-001 ADC (DAR=7.12) and SWY2110-DXD ADC (DAR=6.93) of the present invention had similar $IC_{50}$ values for EGFR overexpressing cell line DiFi and gefitinib-resistant cell line PC9-GR, and showed better anti-cell proliferation effect compared to SWY2110 mAb.

Assay 2: Efficiency assay of antibody-drug conjugates on HER2-expressing human breast cancer JIMT-1 xenograft tumor in NU/NU mice

1. Assay animals

NU/NU mice, 5-6 weeks old, 24 mice

2. Assay object

To investigate the antitumor effect of antibody-drug conjugates in vivo using a HER2-expressing human breast cancer JIMT-1 cell xenograft tumor model in NU/NU mice.

3. Drug dosage and grouping

Table 4. Table of animal grouping and dosage

| Group | Animal Number | Dosage (mg/kg) | Administration Route/Frequency |
|---|---|---|---|
| Vehicle | 6 | 0 | i.v./once |
| DP001-DXD ADC (DAR=4.30) | 6 | 3 | i.v./once |
| DP001-JSSW-001 ADC (DAR=4.0) | 6 | 3 | i.v./once |
| DP001-ZW-002 ADC (DAR=5.78) | 6 | 3 | i.v./once |
| Note: i.v.: intravenous injection. | | | |

4. Assay method

In this assay, human breast cancer JIMT-1 cells were used to construct a xenograft tumor model in nude mice. When the tumor volume reached about 127 $mm^3$, the animals were evenly divided into 4 groups (d0) according to Table 4 based on tumor volume with 6 animals in each group. Intravenous administration was given once, with an administration volume of 10mL/kg. The vehicle control group (Vehicle) received a 0.9% sodium chloride injection. Observations were made for 13 days after administration to compare the inhibitory effects of three antibody-drug conjugates in the table on HER2-expressing human breast cancer JIMT-1 xenograft tumors in nude mice.

5. Observation indicator

5.1 Evaluation indicator

(1)

$$\text{Tumor volume: } V = 1/2 \times A \times B^2$$

(2)

$$\text{Relative tumor volume: RTV} = \frac{TV_{nd}}{TV_{0d}}$$

(3)

$$\text{Relative tumor volume proliferation rate: } \frac{T}{C}\% = \frac{\text{Drug Group RTV}_{xnd}}{\text{Vehicle Group RTV}_{xnd}} \times 100\%$$

(4)

$$\text{Tumor (growth) inhibition rate: TGI\%} = [1 - \frac{TV_{Xn} - TV_{X0}}{TV_{Mn} - TV_{M0}}] \times 100\%$$

Note: V: Tumor volume
A: tumor length
B: tumor width
RTV: relative tumor volume
$TV_{nd}$: tumor volume on day n
$TV_{0d}$: tumor volume on day 0
$RTV_{xnd}$: average relative tumor volume on day n
$TV_{Xn}$: average tumor volume of drug group on day n
$TV_{X0}$: average tumor volume of drug group on day 0
$TV_{Mn}$: average tumor volume of vehicle group on day n
$TV_{M0}$: average tumor volume of vehicle group on day 0

6 Result

6.1 Tumor volume

At the end of the assay, compared with the vehicle group, DP001-DXD ADC (DAR=4.3) (P<0.05), DP001-JSSW-001 ADC (DAR=4.0) (P<0.01) and DP001-ZW-002 ADC (DAR=5.78) (P<0.05) all could significantly inhibit the volume of transplantation tumor (see Figure 11 and Table 5 for details).

Table 5 Tumor parameters of each group 13 days after administration of antibody-drug conjugates

| Group | Animal Number | Dosage (mg/kg) | Tumor Volume ($mm^3$) | TGI (%) Day13 |
|---|---|---|---|---|
| Vehicle | 6 | 0 | 322.7±82.2 | - |
| DP001-DXD ADC (DAR=4.30) | 6 | 3 | 192.7±70.2* | 67.3 |
| DP001-JSSW-001 ADC (DAR=4.0) | 6 | 3 | 202.9±38.1** | 61.4 |
| DP001-ZW-002 ADC (DAR=5.78) | 6 | 3 | 194.8+68.2* | 65.3 |
| *: compared with the vehicle group P<0.05, **: compared with the vehicle group P<0.01 | | | | |

Assay 3: Efficacy assay of antibody-drug conjugate on human lung cancer PC9-GR (gefitinib-resistant cell) xenograft tumor in NU/NU mice

1. Assay animals

NU/NU mice, 5-6 weeks old, 16 mice

2. Assay object

To investigate the anti-tumor effect of two patritumab-ADC drugs in vivo using a human lung cancer PC-9/GR cell (gefitinib-resistant cell) xenograft tumor model in NU/NU mice.

3. Drug dosage and grouping

Table 6. Table of animal grouping and dosage

| Group | Animal Number | Dosage (mg/kg) | Administration Route/Frequency |
|---|---|---|---|
| Vehicle | 6 | 0 | i.v./qw*4w |
| patritumab-DXD ADC (DAR=7.3) | 5 | 3 | i.v./qw*4w |
| patritumab-JSSW-001 ADC (DAR=7.36) | 5 | 3 | i.v./qw*4w |
| Note: i.v.: intravenous injection, qw*4w: administration once weekly for 4 weeks. | | | |

4. Assay method

In this assay, human lung cancer PC9-GR cells (gefitinib-resistant cells) were used to construct a xenograft tumor model in nude mice. When the tumor volume reached approximately 110 $mm^3$, the animals were divided into 3 groups (d0) based on tumor volume according to Table 6, with 6 animals in vehicle group, each 5 animals in patritumab-DXD ADC (DAR=7.3) group and patritumab-JSSW-001 ADC (DAR=7.36) group. Intravenous administration was given once weekly for 4 weeks, with an administration volume of 10mL/kg. The vehicle control group (Vehicle) received a 0.9% sodium chloride injection. The assay was terminated on day 28 after the administration. The inhibitory effects of patritumab-DXD ADC (DAR=7.3) and patritumab-JSSW-001 ADC (DAR=7.36) on human lung cancer PC9-GR (Gefitinib-resistant cell) xenograft tumor in nude mice were compared.

5. Observation indicator

5.1 Evaluation indicator

See the section of Assay 2: 5.1 Evaluation indicator

6 Result

6.1 Tumor volume

At the end of the assay, the patritumab-JSSW-001 ADC (DAR=7.36) group ($P<0.001$) exhibited the superior effect of inhibiting the volume of xenograft tumor compared to the patritumab-DXD ADC (DAR=7.3) ($P<0.01$) group. For details, see Figure 12 and Table 7.

Table 7 Tumor parameters of each group 28 days after administration of patritumab-ADC

| Group | Animal Number | Dosage (mg/kg) | Administration Route/Frequency | Tumor Volume ($mm^3$) | TGI (%) Day 28 |
|---|---|---|---|---|---|
| Vehicle | 6 | 0 | i.v./qw*4w | 761.4±272.6 | - |
| patritumab-DXD ADC (DAR=7.3) | 5 | 3 | i.v./qw*4w | 373.4+102.8** | 59.5 |
| patritumab-JSSW-001 ADC (DAR=7.36) | 5 | 3 | i.v./qw*4w | 312.7+86.7*** | 68.9 |
| **: compared with the vehicle group P<0.01, ***: compared with the vehicle group P<0.001 | | | | | |

Assay 4: Efficacy Assay of SWY2110-ADC on human colorectal cancer DiFi xenograft tumor in NU/NU mice

1. Assay animals

NU/NU mice, 5-6 weeks old, 14 mice

2. Assay object

To investigate the anti-tumor effect of two SWY2110-ADC drugs in vivo using a human colorectal cancer DiFi cell xenograft tumor model in NU/NU mice.

3. Drug dosage and grouping

Table 8. Table of animal grouping and dosage

| Group | Animal Number | Dosage (mg/kg) | Administration Route/Frequency |
|---|---|---|---|
| Vehicle | 5 | 0 | i.v./once |
| SWY2110-Vc MMAE ADC (DAR=4.53) | 4 | 0.5 | i.v./once |
| SWY2110-JSSW-001 ADC (DAR=7.12) | 5 | 0.5 | i.v./once |
| Note: i.v.: intravenous injection. | | | |

4. Assay method

In this assay, human colorectal cancer DiFi cells were used to construct a xenograft tumor model in nude mice. When the tumor volume reached approximately 110 $mm^3$, the animals were evenly divided into 3 groups (d0) based on tumor volume according to Table 8, with each 5 animals in vehicle group and SWY2110-JSSW-001 ADC (DAR=7.12) group and 4 animals in SWY2110-Vc MMAE ADC (DAR=4.53) group. Intravenous administration was given once with an administration volume of 10mL/kg. The vehicle control group (Vehicle) received a 0.9% sodium chloride injection. Observation was made for 27 days after the administration. The inhibitory effects of two SWY2110-ADC drugs in the table on human colorectal cancer DiFi xenograft tumor in nude mice were compared.

5. Observation indicator

5.1 Evaluation indicator

See the section of Assay 2: 5.1 Evaluation indicator

6 Result

6.1 Tumor volume

At the end of the assay, compared with the vehicle group, both SWY2110-Vc MMAE ADC (DAR=4.53) ($P<0.05$) and SWY2110-JSSW-001 ADC (DAR=7.12) ($P<0.05$) could significantly inhibit the volume of the xenograft tumor. The tumor inhibition rate on day 27 of the SWY2110-JSSW-001 ADC (DAR=7.12) group reached as high as 74.6%, followed by the SWY2110-Vc MMAE ADC (DAR=4.53) group with a tumor inhibition rate of 69.7%. Since the toxicity of the JSSW-001 small molecule was lower than that of Vc MMAE, it was suggested that SWY2110-JSSW-001 ADC (DAR=7.12) could achieve a more efficient and less toxic therapeutic effect. For details, see Figure 13 and Table 9.

Table 9 Tumor parameters of each group 27 days after administration of SWY2110-ADC

| Group | Animal Number | Dosage (mg/kg) | Tumor Volume ($mm^3$) | TGI (%) Day 27 |
|---|---|---|---|---|
| Vehicle | 5 | 0 | 763.1±293.8 | - |
| SWY2110-Vc MMAE ADC (DAR=4.53) | 4 | 0.5 | 306.5±332.4* | 69.7 |
| SWY2110-JSSW-001 ADC (DAR=7.12) | 5 | 0.5 | 275.5±297.5* | 74.6 |
| *: compared with the vehicle group P<0.05 | | | | |

Assay 5 Efficacy assay of SWY2110-ADC on human lung cancer PC9-GR (gefitinib-resistant cell) xenograft tumor in NU/NU mice

1. Assay animals

NU/NU mice, 5-6 weeks old, 30 mice

2. Assay object

To investigate the anti-tumor effect of SWY2110 monoclonal antibody and three SWY2110-ADC drugs in vivo using a human lung cancer PC-9-GR cell (gefitinib-resistant cell) xenograft tumor model in NU/NU mice.

3. Drug dosage and grouping

Table 10. Table of animal grouping and dosage

| Group | Animal Number | Dosage (mg/kg) | Administration Route/Frequency |
|---|---|---|---|
| Vehicle | 6 | 0 | i.v./qw*4w |
| SWY2110 mAb | 6 | 1 | i.v./qw*4w |
| SWY2110-Vc MMAE ADC (DAR=4.53) | 6 | 1 | i.v./qw*4w |
| SWY2110-DXD ADC (DAR=6.93) | 6 | 1 | i.v./qw*4w |
| SWY2110-JSSW-001 ADC (DAR=7.12) | 6 | 1 | i.v./qw*4w |
| Note: i.v.: intravenous injection, qw*4w: administration once weekly for 4 weeks. | | | |

4. Assay method

In this assay, human lung cancer PC9-GR cells (gefitinib-resistant cells) were used to construct a xenograft tumor model in nude mice. When the tumor volume reached approximately 110 $mm^3$, the animals were evenly divided into 5 groups (d0) based on tumor volume according to Table 10, with 6 animals in each group. Intravenous administration was given once weekly for 4 weeks, with an administration volume of 10mL/kg. The vehicle control group (Vehicle) received a 0.9% sodium chloride injection. The assay was terminated on day 28 after the administration. The inhibitory effects of SWY2110 monoclonal antibody and three SWY2110-ADC drugs on human lung cancer PC9-GR (Gefitinib-resistant cell) xenograft tumor in nude mice were compared.

5. Observation indicator

5.1 Evaluation indicator

See the section of Assay 2: 5.1 Evaluation indicator

6 Result

6.1 Tumor volume

At the end of the assay, compared with the vehicle group, SWY2110-Vc MMAE ADC (DAR=4.53) ($P<0.05$), SWY2110-DXD ADC (DAR=6.93) ($P<0.05$), and SWY2110-JSSW-001 ADC (DAR=7.12) ($P<0.001$) all exhibited inhibitory effects on the volume of the xenograft tumor. The antibody SWY2110 showed poor tumor inhibitory effect, with a tumor inhibition rate of only 20.3%. The SWY2110-JSSW-001 ADC (DAR=7.12) group had the most significant inhibitory effect on the xenograft tumor volume of PC9-GR, with a tumor inhibition rate as high as 77.7%. The tumor inhibition rates of SWY2110-Vc MMAE ADC (DAR=4.53) and SWY2110-DXD ADC (DAR=6.93) were 40.4% and 50.2%, respectively. SWY2110-JSSW-001 ADC (DAR=7.12) showed 154% of the tumor inhibitory effect of SWY2110-DXD ADC (DAR=6.93) on the PCR-GR, and SWY2110-JSSW-001 ADC (DAR=7.12) showed 192% of the tumor inhibitory effect of SWY2110-Vc MMAE ADC (DAR=4.53) on PCR-GR, suggesting that SWY2110-JSSW-001 ADC (DAR=7.12) still had a significant

inhibitory effect on gefitinib-resistant tumor cells. For details, see Figure 14 and Table 11.

Table 11 Tumor parameters of each group 28 days after administration of SWY2110-ADC

| Group | Animal Number | Dosage (mg/kg) | Administration Route/Frequency | Tumor Volume (mm3) | TGI (%) Day 28 |
|---|---|---|---|---|---|
| Vehicle | 6 | 0 | i.v./qw*4w | 761.4±272.6 | - |
| SWY2110 mAb | 6 | 1 | i.v./qw*4w | 628.9±290.0 | 20.3 |
| SWY2110-Vc MMAE ADC (DAR=4.53) | 6 | 1 | i.v./qw*4w | 498.0±179.1* | 40.4 |
| SWY2110-DXD ADC (DAR=6.93) | 6 | 1 | i.v./qw*4w | 434.6±136.3* | 50.2 |
| SWY2110-JSSW-001 ADC (DAR=7.12) | 6 | 1 | i.v./qw*4w | 255.1±117.7*** | 77.7 |
| *: compared with the vehicle group P<0.05, ***: compared with the vehicle group P<0.001 | | | | | |

Assay 6: In Vitro Plasma Stability of Antibody-Drug Conjugates

DP001-DXD ADC (DAR=7.0) and DP001-JSSW-001 ADC (DAR=7.32) were incubated with 0.5% BSA-PBS and ICR mouse plasma, respectively, at concentrations of DP001-DXD ADC (DAR=7.0) (91.7μg/mL) and DP001-JSSW-001 ADC (DAR=7.32) (80μg/mL). Samples were collected after incubation for 0h, 24h, 48h, 72h, 96h, 120h, 144h, 168 h, 336h and 504h, respectively, under sterile conditions at 37°C. The concentration of dropped molecules in the plasma samples was detected using LC-MS/MS. The results are as follows:

Table 12 In vitro plasma stability

| | DP001-DXD ADC (DAR=7.0) | | DP001-JSSW-001 ADC (DAR=7.32) | |
|---|---|---|---|---|
| Time | 0.5% BSA-PBS | ICR | 0.5% BSA-PBS | ICR |
| (h) | Dropping rate (%) | Dropping rate (%) | Dropping rate (%) | Dropping rate (%) |
| 0 | 0.02 | 0.02 | 0.03 | 0.01 |
| 24 | - | - | 0.13 | 0.15 |
| 48 | 0.13 | 0.61 | 0.16 | 1.02 |
| 72 | 0.13 | 0.65 | 0.18 | 1.13 |
| 96 | 0.13 | 0.97 | 0.20 | 0.77 |
| 120 | 0.16 | 1.18 | - | 0.82 |
| 144 | 0.16 | 1.29 | 0.19 | 1.18 |
| 168 | 0.14 | 1.43 | 0.20 | 1.36 |
| 336 | 0.41 | 2.50 | 0.27 | 1.54 |
| 504 | 3.90 | 4.07 | 1.09 | 2.75 |
| Note: 1. Calculation of the dropping rate: according to the DAR value, the mass ratio of DXD/ADC was calculated to determine DP001-DXD ADC (DAR=7.0) (91.7μg/mL) and DP001-JSSW-001 ADC (DAR=7.32) (80μg/mL). Dropping rate (%)= Ct/ Ctotal*100%, wherein Ctotal represents the total small molecule concentration, and Ct represents the free small molecule concentration at each time point. "-" indicates no sampling. | | | | |

The results showed that ADCs generated free small molecules in both 0.5% BSA-PBS and ICR mouse plasma after incubation at 37°C for 0h, 24h, 48h, 72h, 96h, 120h, 144h, 168 h, 336h and 504h. After 504 hours of incubation, the dropping rates in 0.5% BSA-PBS were DP001-DXD ADC (DAR=7.0) (3.90%) and DP001-JSSW-001 ADC (DAR=7.32) (1.09%) respectively, and in ICR mouse plasma, the dropping rates were DP001-DXD ADC (DAR=7.0) (4.07%) and DP001-JSSW-001 ADC (DAR=7.32) (2.75%) respectively. In 0.5% BSA-PBS, the dropping rates of small molecules from the antibodies were all less than 4.0%: DP001-DXD ADC (DAR=7.0) > DP001-JSSW-001 ADC (DAR=7.32), indicating that DP001-JSSW-001 ADC (DAR=7.32) was more stable in 0.5% BSA-PBS. In ICR mouse plasma, the dropping rates of

small molecules from the antibodies were all less than 5.0%: DP001-DXD ADC (DAR=7.0) > DP001-JSSW-001 ADC (DAR=7.32), indicating that DP001-JSSW-001 ADC (DAR=7.32) was more stable in ICR mouse plasma than DP001-DXD ADC (DAR=7.0).

Assay 7: Study on in vitro bystander effect of antibody-drug

For the study on the bystander effect, positive cell line SK-BR-3 and negative control cell MDA-MB-468 were co-cultured with DP001-DXD ADC (DAR=7.0) and DP001-JSSW-001 ADC (DAR=7.32), respectively. The released small molecule payloads could cause apoptosis of co-cultured negative cells in the periphery, known as the bystander effect. In this study, reporter gene cells HEK293-Luc were co-cultured with positive cell line SK-BR-3 and negative control cell MDA-MB-468 for four days. The effect of released small molecule payloads on the growth of HEK293-Luc was determined by adding a chemiluminescent substrate. The study results showed that DP001-DXD ADC (DAR=7.0) and DP001-JSSW-001 ADC (DAR=7.32) could bind to HER2 receptors on the surface of tumor cells, enter the cells through endocytosis and release small molecule payloads. Meanwhile, DP001-JSSW-001 ADC (DAR=7.32) showed a stronger inhibitory effect on the proliferation of bystander cells.

2. Assay preparation

2.1 Assay equipments:

| Apparatus and equipment | Manufacturer | Model |
|---|---|---|
| $CO_2$ incubator | Thermo Fisher | 3111 model |
| Biosafety cabinet | ThermoFisher | 1300 Series A2 6 inches |
| Desktop high speed freezing centrifuge | Thermo Fisher | SORVALL Stratos |
| Inverted microscope | Olympus | CKX31 inverted microscope |
| Pipettor | Eppendorf | Research Plus |
| Microplate reader | Tecan | M200 |

2.2 Assay materials:

| Name | Manufacturer | Item Number |
|---|---|---|
| 96-well transparent flat-bottom culture plate | Corning | 3599 |
| cell culture plate | Corning | 3917 |
| 50mL centrifuge tube | Corning | 430828 |
| 15mL centrifuge tube | Corning | 430790 |
| T75 cell culture flask | Corning | 430641 |
| T25 cell culture flask | Corning | 430639 |

2.3 Assay reagents:

| Name | Manufacturer | Item Number |
|---|---|---|
| DPBS | BBI | E607009-0500 |
| Fetal Bovine Serum | Gibco | 10099-141C |

(continued)

| Name | Manufacturer | Item Number |
|---|---|---|
| DMEM culture medium | HyClone | SH30243.01 |
| 0.25% Trypsin-EDTA | Gibco | 25200-072 |
| Bright-GloTM Luciferase assay system | Promega | E2620 |

2.4 Cell information

| Name | Manufacturer | Culture condition |
|---|---|---|
| SK-BR-3 | ATCC | DMEM culture medium +10% |
| MDA-MB-468 | Nanjing Cobioer | DMEM culture medium +10% |
| HEK293-Luc | self-made in the laboratory | DMEM culture medium +10% FBS+400 μg/ml G418+100 μg/ml HygromycinB |

3. Assay method

3.1 Reagent preparation

3.1.1 Preparation of cell recovery medium

DMEM complete medium: Adding to a DMEM basic culture medium so that the final system contained 10% of Fetal Bovine Serium and 1% of Penicilin/Streptomycin, fully mixing uniformly, and storing at 2-8°C.

3.1.2 preparation of cell culture medium (containing a screening agent)

Adding to a DMEM basic culture medium so that the final system contained 10% of Fetal Bovine Serium and 1% of Penicilin/Streptomycin and adding 200μg/mL of G418, fully mixing uniformly, and storing at 2-8°C.

3.2 Cell preparation

3.2.1 Cell recovery

A cell cryopreservation tube was placed in a water bath at 37°C and shaken quickly until it was completely thawed, then transferred to an ultra-clean workbench or a biosafety cabinet. The cell suspension was diluted with a cell recovery medium. The mixture was centrifuged, and the centrifugation supernatant was discarded. Cells were resuspended by using the cell recovery medium, and the cell density and viability were measured. The cell density was adjusted with the cell culture medium. The cell suspension was uniformly mixed, transferred to a cell culture flask. After the microscopic examining, it was transferred to an incubator for culturing at 37°C with 5% $CO_2$.

3.2.2 cell passages

The cell culture flask was removed from the $CO_2$ incubator and transferred to the ultra-clean workbench or biosafety cabinet. After the cells were washed with PBS, digested with trypsin, and the digestion was terminated with cell growth medium, the cell suspension was collected, and cell density and viability were measured. An appropriate amount of the cell suspension was added to a suitable amount of the fresh medium (with or without a screening agent), and the cells were passaged and expanded according to their growth characteristics and experimental needs. After passaging, the cells were placed in an incubator for continued culture.

3.3 Bystander effect test procedure

3.3.1 When the cell confluence reached 90%, SK-BR-3, MDA-MB-468, and HEK293-Luc cells were digested with trypsin and collected separately, and the cells were resuspended in their respective media.

3.3.2 The resuspended target cells were gently pipetted several times to form a single-cell suspension. Cell viability and cell count were identified using the trypan blue staining method, and the cell density was adjusted to 2.0×105 cells/mL.

3.3.3 The density-adjusted SK-BR-3 and MDA-MB-468 cells were evenly mixed with HEK293-Luc cell suspension at a 1:1 ratio. Fifty microliters per well were added to a 96-well transparent flat-bottom culture plate. The positive cell group was a mixture of SK-BR-3 and HEK293-Luc cells, while the negative cell group was a mixture of MDA-MB-468 and HEK293-Luc cells.

3.3.4 DP001-DXD ADC (DAR=7.0) and DP001-JSSW-001 ADC (DAR=7.32) groups (drug administration groups) were provided at three concentrations with final concentrations of 100 ng/ml, 500 ng/ml, and 1000 ng/ml. Fifty microliters per well were added to the cell-inoculated 96-well transparent flat-bottom culture plate and placed in a 37°C $CO_2$ incubator for 4 days.

3.3.5 After incubation, the plate was removed from the incubator, and the temperature was allowed to equilibrate to room temperature. Bright-Glo™ Luciferase Assay System was added to the plate at 100μL per well, and chemiluminescence was read after 5-15 minutes in the dark.

4. Test result: DP001-DXD ADC (DAR=7.0) and DP001-JSSW-001 ADC (DAR=7.32) could bind to HER2 receptors on the surface of tumor cells, enter the cells through endocytosis and release small molecule payloads. Meanwhile, DP001-JSSW-001 ADC (DAR=7.32) showed a stronger inhibitory effect on the proliferation of bystander cells than DP001-DXD ADC (DAR=7.0). See Table 13 and Figure 17 for the test result.

Table 13: Inhibition rate of the antibody-drug conjugates of the present invention on fluorescent cells

| Concentration/ng/ml | SKBR3+HEK293-GLP1/DP001-DXD ADC((DAR=7.0) | MDA-MB-468+HEK293-GLP1/DP001-DXD ADC (DAR=7.0) | SKBR3+HEK293-GLP1/DP001-JSSW-001 ADC (DAR=7.32) | MDA-MB-468+HEK293-GLP1/DP001-JSSW-001 ADC (DAR=7.32) |
|---|---|---|---|---|
| | Inhibition rate | | | |
| 1000 | 17.1% | 2.7% | 18.7% | 3.1% |
| 500 | 16.4% | 2.6% | 18.1% | -0.4% |
| 100 | 10.0% | 3.4% | 16.8% | 2.7% |

Assay 8: Study on the stability of linker-drug compounds

Different linker-drug compounds such as Deruxtecan and JSSW-001 were studied under high temperature (40°C, 60°C), high humidity (25°C/75% RH and 25°C/92.5% RH), illumination (illuminance 4500Lux, near-UV energy 90μw/cm$^2$), or 0.5% BSA-PBS conditions. Samples were collected at different times after exposure to these conditions, and the concentration changes of the linker-drug compounds in the samples were detected using the LC-MS/MS method. The test results showed that the compound JSSW-001 had better stability.

Assay 9: Efficacy assay of SWY2110-ADC on human lung cancer PC9-AR (PC9-Del19/T790M/C797S, osimertinib-resistant cell) xenograft tumor

1. Assay animals

Balb/c nude mice, 5-6 weeks old, 24 mice

2. Assay object

To investigate the anti-tumor effect of three SWY2110-ADC drugs in vivo using a human lung cancer PC9-Del19/T790M/C797S cells (osimertinib-resistant cell, hereinafter referred to as PC9-AR) xenograft tumor model in NU/NU mice.

3. Drug dosage and grouping

Table 14. Table of animal grouping and dosage

| Group | Animal Number | Dosage (mg/kg) | Administration Route/Frequency |
|---|---|---|---|
| Vehicle | 6 | 0 | i.v./once |
| SWY2110-Vc MMAE ADC (DAR=4.53) | 6 | 3 | i.v./once |
| SWY2110-DXD ADC (DAR=6.93) | 6 | 3 | i.v./once |
| SWY2110-JSSW-001 ADC (DAR=7.12) | 6 | 3 | i.v./once |
| Note: i.v.: intravenous injection, i.v./once: administration once. | | | |

4. Assay method

In this assay, human lung cancer PC9-Del19/T790M/C797S cells (osimertinib-resistant cell) were used to construct a xenograft tumor model in nude mice. When the tumor volume reached approximately 110 $mm^3$, the animals were evenly divided into 4 groups (d0) based on tumor volume according to Table 14, with 6 animals in each group. Intravenous administration was given once, with an administration volume of 10mL/kg. The vehicle control group (Vehicle) received a 0.9% sodium chloride injection. The assay was terminated on day 24 after the administration. The inhibitory effects of three SWY2110-ADC drugs on human lung cancer PC9-Del19/T790M/C797S (osimertinib-resistant cell) xenograft tumor in nude mice were compared.

5. Observation indicator

5.1 Evaluation indicator

See the section of Assay 2: 5.1 Evaluation indicator

6 Result

6.1 Tumor volume

At the end of the assay, compared with the vehicle group, SWY2110-Vc MMAE ADC (DAR=4.53) (P<0.001), SWY2110-DXD ADC (DAR=6.93) (P<0.001) and SWY2110-JSSW-001 ADC (DAR=7.12) (P<0.001) all exhibited inhibitory effects on the volume of the xenograft tumor. The SWY2110-JSSW-001 ADC (DAR=7.12) group demonstrated the most significant inhibitory effect on the PC9-AR xenograft tumor volume, achieving a tumor inhibition rate of as high as 97.5%. The tumor inhibition rates of SWY2110-Vc MMAE ADC (DAR=4.53) and SWY2110-DXD ADC (DAR=6.93) were 89.7% and 76.2% respectively. SWY2110-JSSW-001 ADC (DAR=7.12) showed 128% of the tumor inhibitory effect of SWY2110-DXD ADC (DAR=6.93), and SWY2110-JSSW-001 ADC (DAR=7.12) showed 109% of the tumor inhibitory effect of SWY2110-Vc MMAE ADC (DAR=4.53), suggesting that SWY2110-JSSW-001 ADC (DAR=7.12) still had a significant inhibitory effect on osimertinib-resistant tumor cells. For details, see Figure 18 and Table 15.

Table 15 Tumor parameters of each group 24 days after administration of SWY2110-ADC

| Group | Animal Number | Dosage (mg/kg) | Administration Route/Frequency | Tumor Volume ($mm^3$) | TGI (%) Day 24 |
|---|---|---|---|---|---|
| Vehicle | 6 | 0 | i.v./once | 1138.3 | --- |
| SWY2110-Vc MMAE ADC (DAR=4.53) | 6 | 3 | i.v./once | 212.3*** | 89.7 |
| SWY2110-DXD ADC (DAR=6.93) | 6 | 3 | i.v./once | 352.0*** | 76.2 |

(continued)

| Group | Animal Number | Dosage (mg/kg) | Administration Route/Frequency | Tumor Volume ($mm^3$) | TGI (%) Day 24 |
|---|---|---|---|---|---|
| SWY2110-JSSW-001 ADC (DAR=7.12) | 6 | 3 | i.v./once | 131.3*** | 97.5 |
| ***: statistical significance relative to the vehicle group p<0.001 | | | | | |

Assay 10 Efficacy Assay of SWY2110-ADC, SWY2111-ADC, SWY2112-ADC, SWY2113-ADC on human lung cancer NCI-H1975 (human lung cancer cell) xenograft tumor

1. Assay animals

NU/NU mice, 5-6 weeks old, 30 mice

2. Assay object

To investigate the anti-tumor effect of SWY2110-JSSW-001, SWY2111-JSSW-001, SWY2112-JSSW-001, SWY2113-JSSW-001 drugs in vivo using a human lung cancer NCI-H1975 cell (human lung cancer cell) xenograft tumor model in NU/NU mice.

3. Drug dosage and grouping

Table 16. Table of animal grouping and dosage

| Group | Animal Number | Dosage (mg/kg) | Administration Route/Frequency |
|---|---|---|---|
| Vehicle | 6 | - | - |
| SWY2110-JSSW-001 ADC (DAR=7.12) | 6 | 1 | i.v./once |
| SWY2111-JSSW-001 ADC (DAR=7.48) | 6 | 1 | i.v./once |
| SWY2112-JSSW-001 ADC (DAR=7.51) | 6 | 1 | i.v./once |
| SWY2113-JSSW-001 ADC (DAR=7.50) | 6 | 1 | i.v./once |
| Note: i.v.: intravenous injection, i.v./once: administration once; | | | |

4. Assay method

In this assay, human lung cancer NCI-H1975 cells (human lung cancer cells) were used to construct a xenograft tumor model in nude mice. When the tumor volume reached approximately 110 $mm^3$, the animals were evenly divided into 5 groups (d0) based on tumor volume according to Table 16, with 6 animals in each group. Each dosage group received a single administration at 1mg/kg. Intravenous administration was given once, with an administration volume of 10mL/kg. The vehicle control group (Vehicle) received a 0.9% sodium chloride injection. The assay was terminated on day 21 after the administration. The inhibitory effects of SWY2110-JSSW-001, SWY2111-JSSW-001, SWY2112-JSSW-001, SWY2113-JSSW-001 drugs on human lung cancer NCI-H1975 xenograft tumor in nude mice were compared.

5. Observation indicator

5.1 Evaluation indicator

See Assay 2: the part of 5.1 Evaluation indicator

6 Result

6.1 Tumor volume

At the end of the assay, compared to the vehicle group, SWY2110-JSSW-001 ADC (DAR=7.12), SWY2112-JSSW-001 ADC (DAR=7.51), SWY2111-JSSW-001 ADC (DAR=7.48), SWY2113-JSSW-001 ADC (DAR=7.50) (P<0.001) all demonstrated significant tumor inhibitory effects (P<0.001), achieving tumor inhibition rates of 84.1%, 83.0%, 80.9% and 74.2% (P<0.001) respectively. These results indicated that all four ADCs had significant inhibitory effects on human lung cancer NCI-H1975 tumor cells. For details, see Figure 19 and Table 17.

Table 17 Tumor parameters of each group 21 days after administration of SWY2110 to SWY2113-ADCs

| Group | Animal Number | Dosage (mg/kg) | Administration Route/Frequency | Tumor volume ($mm^3$) | TGI (%) Day 21 |
|---|---|---|---|---|---|
| Vehicle | 6 | 0 | i.v./once | 852.4±97.8 | --- |
| SWY2110-JSSW-001 ADC (DAR=7.12) | 6 | 1 | i.v./once | 223.3±71.7*** | 84.2 |
| SWY2111-JSSW-001 ADC (DAR=7.48) | 6 | 1 | i.v./once | 248.4+80.3*** | 80.9 |
| SWY2112-JSSW-001 ADC (DAR=7.51) | 6 | 1 | i.v./once | 231.8±106.5*** | 83.0 |
| SWY2113-JSSW-001 ADC (DAR=7.50) | 6 | 1 | i.v./once | 298.4±108.4*** | 74.2 |
| ***: compared with the vehicle group P<0.001 | | | | | |

Assay 11: Efficacy Assay of SWY2110-ADC, SWY2111-ADC, SWY2112-ADC, SWY2113-ADC on human breast cancer MDA-MB-468 (human breast cancer cell) xenograft tumor

1. Assay animals

NOD-SCID mice, 5-6 weeks old, 28 mice

2. Assay object

To investigate the anti-tumor effect of SWY2110-JSSW-001, SWY2111-JSSW-001, SWY2112-JSSW-001, SWY2113-JSSW-001 drugs in vivo using a human breast cancer MDA-MB-468 cell (human breast cancer cell) xenograft tumor model in NOD-SCID mice.

3. Drug dosage and grouping

Table 18. Table of animal grouping and dosage

| Group | Animal Number | Dosage (mg/kg) | Administration Route/Frequency |
|---|---|---|---|
| Vehicle | 8 | 0 | i.v./once |
| SWY2110-JSSW-001 ADC (DAR=7.12) | 5 | 2 | i.v./once |
| SWY2111-JSSW-001 ADC (DAR=7.48) | 5 | 2 | i.v./once |
| SWY2112-JSSW-001 ADC (DAR=7.51) | 5 | 2 | i.v./once |
| SWY2113-JSSW-001 ADC (DAR=7.50) | 5 | 2 | i.v./once |
| Note: i.v.: intravenous injection, i.v./once: administration once; | | | |

4. Assay method

In this assay, human breast cancer MDA-MB-468 cells (human breast cancer cells) were used to construct a xenograft tumor model in mice. When the tumor volume reached approximately 150 $mm^3$, the animals were divided into 5 groups (d0) based on tumor volume according to Table 18, with 8 animals in the control group and 5 animals in the

experimental group. Intravenous administration was given once, with an administration volume of 10mL/kg. The vehicle control group (Vehicle) received a 0.9% sodium chloride injection. The assay was terminated on day 28 after the administration. The anti-tumor effects of four ADC drugs in vivo were investigated.

5. Observation indicator

5.1 Evaluation indicator

See the section of Assay 2: 5.1 Evaluation indicator

6 Result

6.1 Tumor volume

At the end of the assay, compared to the vehicle group, the tumor inhibition rates of SWY2110-JSSW-001 (DAR=7.12), SWY2111-JSSW-001 ADC (DAR=7.48), SWY2112-JSSW-001 ADC (DAR=7.51) and SWY2113-JSSW-001 ADC (DAR=7.50) experimental groups reached 65.8%, 65.1%, 58.3%, 47.1% (P<0.001) respectively, all demonstrating good tumor inhibition activity. It was suggested that four ADC drugs had significant inhibitory effect on human breast cancer MDA-MB-468 tumor cells. For details, see Figure 20 and Table 19.

Table 19 Tumor parameters of each group 28 days after administration of SWY2110 to SWY2113-ADCs

| Group | Animal Number | Dosage (mg/kg) | Administration Route/Frequency | Tumor Volume ($mm^3$) | TGI (%) Day 28 |
|---|---|---|---|---|---|
| Vehicle | 8 | - | - | 525.0±28.4 | --- |
| SWY2110-JSSW-001 ADC (DAR=7.12) | 5 | 2 | i.v./once | 275.9±50.6*** | 65.8 |
| SWY2111-JSSW-001 ADC (DAR=7.48) | 5 | 2 | i.v./once | 278.8±22.9*** | 65.1 |
| SWY2112-JSSW-001 ADC (DAR=7.51) | 5 | 2 | i.v./once | 304.3±40.6*** | 58.3 |
| SWY2113-JSSW-001 ADC (DAR=7.50) | 5 | 2 | i.v./once | 346.9±46.2*** | 47.1 |
| ***: compared with the vehicle group P<0.001 | | | | | |

Assay 12 Efficacy Assay of SWY2110-ADC, SWY2111-ADC, SWY2112-ADC, SWY2113-ADC on human colorectal cancer DiFi (human colorectal cancer cell) xenograft tumor

1. Assay animals

NU/NU mice, 5-6 weeks old, 35 mice

2. Assay object

To investigate the anti-tumor effect of SWY2110-JSSW-001, SWY2111-JSSW-001, SWY2112-JSSW-001, SWY2113-JSSW-001 drugs in vivo using a human colorectal cancer DiFi (human colorectal cancer cell) xenograft tumor model in NU/NU mice.

3. Drug dosage and grouping

Table 20. Table of animal grouping and dosage

| Group | Animal Number | Dosage (mg/kg) | Administration Route/Frequency |
|---|---|---|---|
| Vehicle | 7 | - | - |
| SWY2110-JSSW-001 ADC (DAR=7.12) | 7 | 1 | i.v./qw*twice |

(continued)

| Group | Animal Number | Dosage (mg/kg) | Administration Route/Frequency |
|---|---|---|---|
| SWY2111-JSSW-001 ADC (DAR=7.48) | 7 | 1 | i.v./qw*twice |
| SWY2112-JSSW-001 ADC (DAR=7.51) | 7 | 1 | i.v./qw*twice |
| SWY2113-JSSW-001 ADC (DAR=7.50) | 7 | 1 | i.v./qw*twice |
| Note: i.v.: intravenous injection, i.v./qw*twice: administration twice weekly; | | | |

4. Assay method

In this assay, human colorectal cancer DiFi cells (human colorectal cancer cells) were used to construct a xenograft tumor model in mice. When the tumor volume reached approximately 100 $mm^3$, the animals were divided into 5 groups (d0) based on tumor volume according to Table 20, with 7 animals in each group. Intravenous administration was given twice, with an administration volume of 10mL/kg. The vehicle control group (Vehicle) received a 0.9% sodium chloride injection. The assay was terminated on day 21 after the administration. The anti-tumor effects of four ADC drugs in vivo were investigated.

5. Observation indicator

5.1 Evaluation indicator

See the section of Assay 2: 5.1 Evaluation indicator

6 Result

6.1 Tumor volume

At the end of the assay, compared to the vehicle group, the tumor inhibition rates of SWY2110-JSSW-001 (DAR=7.12), SWY2111-JSSW-001 ADC (DAR=7.48), SWY2112-JSSW-001 ADC (DAR=7.51), SWY2113-JSSW-001 ADC (DAR=7.50) experimental groups reached 86.5%, 82.3%, 83.5%, 76.8% (P<0.001) respectively, all demonstrating good tumor inhibition activity. It was suggested that four ADC drugs had significant inhibitory effect on human colorectal cancer DiFi tumor cells. For details, see Figure 21 and Table 21.

Table 21 Tumor parameters of each group 21 days after administration of SWY2110 to SWY2113-ADCs

| Group | Animal Number | Dosage (mg/kg) | Administration Route/Frequency | Tumor Volume ($mm^3$) | TGI (%) Day 28 |
|---|---|---|---|---|---|
| Vehicle | 7 | 0 | - | 562.2±272.8 | --- |
| SWY2110-JSSW-001 ADC (DAR=7.12) | 7 | 1 | i.v./qw*twice | 161.9±41.2*** | 86.5 |
| SWY2111-JSSW-001 ADC (DAR=7.48) | 7 | 1 | i.v./qw*twice | 180.3+98.8*** | 82.3 |
| SWY2112-JSSW-001 ADC (DAR=7.51) | 7 | 1 | i.v./qw*twice | 175.3±36.7*** | 83.5 |
| SWY2113-JSSW-001 ADC (DAR=7.50) | 7 | 1 | i.v./qw*twice | 205.3+155.6*** | 76.8 |
| ***: compared with the vehicle group P<0.001 | | | | | |

Assay 13: Efficacy Assay of SWY2110-ADC, SWY2111-ADC, SWY2112-ADC, SWY2113-ADC on human lung cancer PC9-GR (human lung cancer gefitinib-resistance acquired cell line) xenograft tumor

1. Assay animals

NU/NU mice, 5-6 weeks old, 30 mice

2. Assay object

To investigate the anti-tumor effect of SWY2110-JSSW-001, SWY2111-JSSW-001, SWY2112-JSSW-001, SWY2113-JSSW-001 drugs in vivo using a human lung cancer cell PC9-GR xenograft tumor model in NU/NU mice.

3. Drug dosage and grouping

Table 22. Table of animal grouping and dosage

| Group | Animal Number | Dosage (mg/kg) | Administration Route/Frequency |
|---|---|---|---|
| Vehicle | 6 | - | - |
| SWY2110-JSSW-001 ADC (DAR=7.12) | 6 | 3 | i.v./once |
| SWY2111-JSSW-001 ADC (DAR=7.48) | 6 | 3 | i.v./once |
| SWY2112-JSSW-001 ADC (DAR=7.51) | 6 | 3 | i.v./once |
| SWY2113-JSSW-001 ADC (DAR=7.50) | 6 | 3 | i.v./once |
| Note: i.v.: intravenous injection, i.v./once: administration once; | | | |

4. Assay method

In this assay, human lung cancer cell PC9-GR cells were used to construct a xenograft tumor model in mice. When the tumor volume reached approximately 110 $mm^3$, the animals were divided into 5 groups (d0) based on tumor volume according to Table 22, with 6 animals in each group. Intravenous administration was given once, with an administration volume of 10mL/kg. The vehicle control group (Vehicle) received a 0.9% sodium chloride injection. The assay was terminated on day 28 after the administration. The anti-tumor effects of four ADC drugs in vivo were investigated.

5. Observation indicator

5.1 Evaluation indicator

See the section of Assay 2: 5.1 Evaluation indicator

6 Result

6.1 Tumor volume

At the end of the assay, compared to the vehicle group, the tumor inhibition rates of SWY2110-JSSW-001 (DAR=7.12), SWY2111-JSSW-001 ADC (DAR=7.48), SWY2112-JSSW-001 ADC (DAR=7.51), SWY2113-JSSW-001 ADC (DAR=7.50) experimental groups reached 99.0%, 100.1%, 92.4%, 89.3% (P<0.001) respectively, all demonstrating good tumor inhibitory effect. It was suggested that four ADC drugs had significant inhibitory effect on human lung cancer cell PC9-GR tumor cells. For details, see Figure 22 and Table 23.

Table 23 Tumor parameters of each group 28 days after administration of SWY2110 to SWY2113-ADCs

| Group | Animal Number | Dosage (mg/kg) | Administration Route/Frequency | Tumor Volume ($mm^3$) | TGI (%) Day 28 |
|---|---|---|---|---|---|
| Vehicle | 6 | - | - | 1159.3 | - |
| SWY2110-JSSW-001 ADC (DAR=7.12) | 6 | 3 | i.v./once | 124.3*** | 99.0 |
| SWY2111-JSSW-001 ADC (DAR=7.48) | 6 | 3 | i.v./once | 112.9*** | 100.1 |
| SWY2112-JSSW-001 ADC (DAR=7.51) | 6 | 3 | i.v./once | 193.0*** | 92.4 |

(continued)

| Group | Animal Number | Dosage (mg/kg) | Administration Route/Frequency | Tumor Volume ($mm^3$) | TGI (%) Day 28 |
|---|---|---|---|---|---|
| SWY2113-JSSW-001 ADC (DAR=7.50) | 6 | 3 | i.v./once | 225.4*** | 89.3 |
| ***: compared with the vehicle group P<0.001 | | | | | |

Assay 14 Efficacy Assay of antibody-drug conjugate on human lung cancer PC9-DTC (Del19/T790M/C797S, osimertinib-resistant cell) xenograft tumor in NOD-SCID mice

1. Assay animals

NOD-SCID mice, 5-6 weeks old, 18 mice

2. Assay object

To investigate the anti-tumor effect of two patritumab-ADC drugs in vivo using a human lung cancer PC9-DTC cell (Del19/T790M/C797S, osimertinib-resistant cell) xenograft tumor model in NU/NU mice.

3. Drug dosage and grouping

Table 24. Table of animal grouping and dosage

| Group | Animal Number | Dosage (mg/kg) | Administration Route/Frequency |
|---|---|---|---|
| Vehicle | 6 | 0 | i.v./qw*3w |
| patritumab-DXD ADC (DAR=7.3) | 6 | 10 | i.v./qw*3w |
| patritumab-JSSW-001 ADC (DAR=7.36) | 6 | 10 | i.v./qw*3w |
| Note: i.v.: intravenous injection, qw*3w: administration once weekly for three weeks. | | | |

4. Assay method

In this assay, human lung cancer PC9-DTC cells (Dell9/T790M/C797S, osimertinib-resistant cells) were used to construct a xenograft tumor model in nude mice. When the tumor volume reached approximately 115 $mm^3$, the animals were divided into 3 groups (d0) based on tumor volume according to Table 24, with each 6 animals in the vehicle group, the patritumab-DXD ADC (DAR=7.3) group and patritumab-JSSW-001 ADC (DAR=7.36) group. Intravenous administration was given once weekly for 3 weeks, with an administration volume of 10mL/kg. The vehicle control group (Vehicle) received a 0.9% sodium chloride injection. The assay was terminated on day 26 after the administration. The inhibitory effects of patritumab-DXD ADC (DAR=7.3) and patritumab-JSSW-001 ADC (DAR=7.36) on human lung cancer PC9-DTC (De119/T790M/C797S, osimertinib-resistant cell) xenograft tumor in nude mice were compared.

5. Observation indicator

5.1 Evaluation indicator

See the section of Assay 2: 5.1 Evaluation indicator

6 Result

6.1 Tumor volume

At the end of the assay, the patritumab-JSSW-001 ADC (DAR=7.36) group (P<0.001) exhibited the superior effect of inhibiting the volume of xenograft tumor compared to the patritumab-DXD ADC (DAR=7.3) (P<0.001) group. For

details, see Figure 23 and Table 25

Table 25 Tumor parameters of each group 26 days after administration of patritumab-ADC

| Group | Animal Number | Dosage (mg/kg) | Administration Route/Frequency | Tumor Volume ($mm^3$) | TGI (%) Day 26 |
|---|---|---|---|---|---|
| Vehicle | 6 | 0 | i.v./qw*3w | 465.1±78.9 | - |
| patritumab-DXD ADC (DAR=7.3) | 6 | 10 | i.v./qw*3w | 132.9±18.8*** | 71.3 |
| patritumab-JSSW-001 ADC (DAR=7.36) | 6 | 10 | i.v./qw*3w | 82.9±14.4*** | 82.3 |
| ***: compared with the vehicle group P<0.001 | | | | | |

Assay 15 Efficacy Assay of antibody-drug conjugates on human colon cancer SW620 xenograft tumor in NU/NU mice

1. Assay animals

NU/NU mice, 5-6 weeks old, 18 mice

2. Assay object

To investigate the anti-tumor effect of two patritumab-ADC drugs in vivo using a human colon cancer SW620 cell xenograft tumor model in NU/NU mice.

3. Drug dosage and grouping

Table 26. Table of animal grouping and dosage

| Group | Animal Number | Dosage (mg/kg) | Administration Route/Frequency |
|---|---|---|---|
| Vehicle | 6 | 0 | i.v./single administration |
| patritumab-DXD ADC (DAR=7.3) | 6 | 7 | i.v./single administration |
| patritumab-JSSW-001 ADC (DAR=7.36) | 6 | 7 | i.v./single administration |

4. Assay method

In this assay, human colon cancer SW620 cells were used to construct a xenograft tumor model in nude mice. When the tumor volume reached approximately 110 $mm^3$, the animals were divided into 3 groups (d0) based on tumor volume according to Table 26, with each 6 animals in the vehicle group, the patritumab-DXD ADC (DAR=7.3) group and the patritumab-JSSW-001 ADC (DAR=7.36) group. Intravenous administration was given once, with an administration volume of 10mL/kg. The vehicle control group (Vehicle) received a 0.9% sodium chloride injection. The assay was terminated on day 26 after the administration. The inhibitory effects of patritumab-DXD ADC (DAR=7.3) and patritumab-JSSW-001 ADC (DAR=7.36) on human colon cancer SW620 xenograft tumor in nude mice were compared.

5. Observation indicator

5.1 Evaluation indicator

See the section of Assay 2: 5.1 Evaluation indicator

6 Result

6.1 Tumor volume

At the end of the assay, the patritumab-JSSW-001 ADC (DAR=7.36) group (P<0.001) exhibited the superior effect of inhibiting the volume of xenograft tumor compared to the patritumab-DXD ADC (DAR=7.3) (P<0.001) group. For details, see Figure 24 and Table 27.

Table 27 Tumor parameters of each group 21 days after administration of patritumab-ADC

| Group | Animal Number | Dosage (mg/kg) | Administration Route/Frequency | Tumor Volume ($mm^3$) | TGI (%) Day 21 |
|---|---|---|---|---|---|
| Vehicle | 6 | 0 | i.v./single administration | 1422.8+416.0 | - |
| patritumab-DXD ADC (DAR=7.3) | 6 | 7 | i.v./single administration | 461.3±278.1*** | 73.2 |
| patritumab-JSSW-001 ADC (DAR=7.36) | 6 | 7 | i.v./single administration | 187.5±91.6 *** | 94.0 |
| ***: compared to the vehicle control group P<0.001 | | | | | |

Assay 16 Efficacy Assay of antibody-drug conjugates on human colon cancer DiFi xenograft tumor in NU/NU mice

1. Assay animals

NU/NU mice, 5-6 weeks old, 18 mice

2. Assay object

To investigate the anti-tumor effect of two patritumab-ADC drugs in vivo using a human colorectal cancer DiFi cell xenograft tumor model in NU/NU mice.

3. Drug dosage and grouping

Table 28. Table of animal grouping and dosage

| Group | Animal Number | Dosage (mg/kg) | Administration Route/Frequency |
|---|---|---|---|
| Vehicle | 6 | 0 | i.v./single administration |
| patritumab-DXD ADC (DAR=7.3) | 6 | 3 | i.v./single administration |
| patritumab-JSSW-001 ADC (DAR=7.36) | 6 | 3 | i.v./single administration |
| Note: i.v.: intravenous injection. | | | |

4. Assay method

In this assay, human colorectal cancer DiFi cells were used to construct a xenograft tumor model in nude mice. When the tumor volume reached approximately 110 $mm^3$, the animals were divided into 3 groups (d0) based on tumor volume according to Table 28, with 6 animals in vehicle group, each 5 animals in patritumab-DXD ADC (DAR=7.3) group and patritumab-JSSW-001 ADC (DAR=7.36) group. Intravenous administration was given once, with an administration volume of 10mL/kg. The vehicle control group (Vehicle) received a 0.9% sodium chloride injection. The assay was terminated on day 28 after the administration. The inhibitory effects of patritumab-DXD ADC (DAR=7.3) and patritumab-JSSW-001 ADC (DAR=7.36) on human colorectal cancer DiFi xenograft tumor in nude mice were compared.

5. Observation indicator

5.1 Evaluation indicator

See the section of Assay 2: 5.1 Evaluation indicator

6 Result

6.1 Tumor volume

At the end of the assay, the patritumab-JSSW-001 ADC (DAR=7.36) group (P<0.01) exhibited the superior effect of inhibiting the volume of xenograft tumor compared to the patritumab-DXD ADC (DAR=7.3) (P<0.05) group. For details, see Figure 25 and Table 29.

Table 29 Tumor parameters of each group 28 days after administration of patritumab-ADC

| Group | Animal Number | Dosage (mg/kg) | Administration Route/Frequency | Tumor Volume ($mm^3$) | TGI (%) Day 28 |
|---|---|---|---|---|---|
| Vehicle | 6 | 0 | i.v./qw*3w | 989.8+317.6 | - |
| patritumab-DXD ADC (DAR=7.3) | 6 | 10 | i.v./qw*3w | 512.5+470.8* | 54.0 |
| patritumab-JSSW-001 ADC (DAR=7.36) | 6 | 10 | i.v./qw*3w | 257.9±189.1** | 82.9 |
| *: compared with the vehicle group P<0.05, **: compared with the vehicle group P<0.01 | | | | | |

Assay 17: Safety Evaluation Study

In this assay, six appropriately aged crab-eating macaques were selected, with three males and three females, and were intravenously injected with SWY2110-JSSW-001, SWY2111-JSSW-001, and SWY2113-JSSW-001. The administration regimen was designed as shown in the table below, with intravenous injections once every three weeks for three successive injections. After the administration, a continuous observation was made for 7 days, a general observation was made twice daily, and a detailed observation was made once daily. Changes in body weight were observed before and after administration, and hematological tests were performed. Assay results showed that compared to the ADCs prepared with pH-dependent antibodies (SWY2111-JSSW-001 and SWY2113-JSSW-001), SWY2110-JSSW-001 exhibited reduced toxicity in skin and gastrointestinal tracts, and no obvious target-related toxicity (such as skin ulceration, scabbing, loose stools, etc.), indicating that the pH-modified ADCs had reduced toxicity to normal tissues, however, there was no significant difference in the haematological toxicity caused by the three ADC molecules.

Table 30: Pre-toxicology experiment design scheme

| Administration frequency | Q3W×3 | | | |
|---|---|---|---|---|
| | Group | D1 | D22 | D43 |
| Administration dosage | Group 1: SWY2110-JSSW-001 | 6mg/kg | 30mg/kg | 30mg/kg |
| | Group 2: SWY2111-JSSW-001 | 6mg/kg | 30mg/kg | 30mg/kg |
| | Group 3: SWY2113-JSSW-001 | 6mg/kg | 30mg/kg | 30mg/kg |
| Animal number | 1 animal/sex/group | | | |

Table 31: Pre-toxicity assay results

| | |
|---|---|
| General observation | Group 1 showed loose stools, skin flakes on limbs, ulceration, and scabbing;<br>Group 2 showed soft stools, mild skin flakes, and no ulceration;<br>Group 3 showed no abnormalities. |

(continued)

| | |
|---|---|
| Body Weight | Group 1 showed a decrease in body weight within 7 days after each administration, with partial recovery before the next administration;<br>Group 2 showed a slight decrease in body weight within 7 days after each administration, with full recovery before the next administration;<br>Group 3 showed no significant decrease in body weight within 7 days after each administration. |
| Hematology | Group 1 HGB, WBC, LYMP↓;<br>Group 2 HGB, WBC, LYMP↓;<br>Group 3 HGB, WBC, LYMP↓; |
| HGB: haemoglobin; WBC: white blood count; LYMP: lymphocyte absolute number. | |

**Formulation Examples**

Formulation Example 1

The pH value is an important factor affecting the stability of biological products. It regulates the charge distribution on the protein surface, thereby affecting intermolecular and intramolecular forces of the protein, and influencing the protein conformation and colloidal stability, while also affecting the chemical stability of the protein, such as degradation reactions like deamidation. An appropriate amount of buffer salt has the function of maintaining a stable pH of the solution; the type of buffer salt plays an important role in the physicochemical properties and stability of biological products.

Histidine-histidine hydrochloride and citric acid-sodium citrate were selected as candidate buffer salts, with a buffer salt ion concentration of 25 mM. The pH value of the histidine-histidine hydrochloride buffer salt was adjusted to 5.0, 5.5, 6.0, and 6.5, and the pH of the citric acid-sodium citrate buffer salt was adjusted to 5.5, 6.0, 6.5, and 7.0. Sucrose was added to each buffer solution to prepare 8 groups of solutions (at 5% sucroce concentration) with different pH values and buffer salts. The antibody-drug conjugate (SWY2110-JSSW-001) sample was exchanged into the above 8 groups of solutions through ultrafiltration, with a final concentration of the antibody-drug conjugate of approximately 5 mg/ml. The appearance of the samples after ultrafiltration exchanging was observed. The histidine-histidine hydrochloride (pH 6.0, 6.5) groups and the citric acid-sodium citrate (pH 5.5, 6.0) groups all showed significant turbidity, and the antibody-drug conjugates precipitated; these 4 groups of samples were discarded. The remaining 4 groups of samples were placed under high temperature (40±2°C) conditions and sampled on the 7th and 14th days; they were placed under light exposure (5±3°C, white light illuminance: 5000 Lux, UV intensity: 90 $\mu$w/cm$^2$) conditions and sampled on the 10th day. The protein concentrations of the antibody-drug conjugates were detected by ultraviolet spectrophotometry, and the molecular size variants of the antibody-drug conjugates were detected by size exclusion chromatography (SEC-HPLC) to investigate the stability of the antibody-drug conjugate in the 4 groups of buffer solutions after high temperature and light exposure treatment. After 7 days at high temperature, the pH 5.5 test group showed protein precipitation with a significant decrease in protein concentration, and a significant decrease in monomer using SEC. After 10 days of light exposure, the citric acid test group showed white precipitate with a significant decrease in protein concentration, and a significant decrease in monomer using SEC. In summary, the 25 mM histidine-histidine hydrochloride at pH=5.0 was selected as the target buffer.

Detection methods:

Detection of protein concentration: the protein concentration was detected using a Nanodrop2000 microspectrophotometer, using a protein-free placebo for zero calibration, based on the extinction coefficient of the antibody-drug conjugate of 1.251.

SEC-HPLC: According to Chinese Pharmacopoeia 2020 edition, Part IV, General Rule 0512 High Performance Liquid Chromatography, analysis and detection were performed using a chromatographic column packed with a gel suitable for separating proteins having a molecular weight of 10-500 kD. The proportions of the protein monomer peak, the high molecular weight substance (HMWS) peak and the low molecular weight substance (LMWS) peak were calculated according to the area normalization method.

Detection result is shown below:

Table 31: Appearance result

| Buffer salt (containing 5% sucrose) | pH value | Appearance | | | | |
|---|---|---|---|---|---|---|
| | | 0d | 5°C, protected from light, 10d | 5°C, light exposure, 10d | 40°C, high temperature, 7d | 40°C, high temperature, 14d |
| Histidine-histidine hydrochloride | 5.0 | Clear | Clear | Clear | Clear | Clear |
| | 5.5 | Clear | Clear | Clear | Slight protein precipitate | More protein precipitate |
| Citric acid-sodium citrate | 6.5 | Clear | Clear | Slight protein precipitate | Clear | Clear |
| | 7.0 | Clear | Clear | Slight protein precipitate | Clear | Clear |

Table 32: Detection result of protein concentrations

| Buffer salt (containing 5% sucrose) | pH value | Protein concentrations (mg/ml) | | | | |
|---|---|---|---|---|---|---|
| | | 0d | 5°C, protected from light, 10d | 5°C, light exposure, 10d | 40°C, high temperature, 7d | 40°C, high temperature, 14d |
| Histidine-histidine hydrochloride | 5.0 | 5.56 | 5.30 | 5.35 | 5.32 | 5.47 |
| | 5.5 | 4.72 | 4.57 | 4.61 | 3.24 | 2.49 |
| Citric acid-sodium citrate | 6.5 | 5.58 | 5.39 | 3.49 | 5.48 | 5.44 |
| | 7.0 | 5.44 | 5.45 | 3.85 | 5.49 | 5.52 |

Table 33: Detection result of SEC-HPLC

| Buffer (containing 5% sucrose) | pH value | SEC-HPLC concentrations (%) | | | | | | | | |
|---|---|---|---|---|---|---|---|---|---|---|
| | | 0d | | | 40°C, high temperature, 7d | | | 5°C, light exposure, 10d | | |
| | | Aggrelgate | Main peak | Fraglment | Aggrelgate | Main peak | Fraglment | Aggrelgate | Main peak | Fraglment |
| Histidine-histidine hydrochloride | 5.0 | 1.57 | 97.30 | 1.13 | 3.77 | 94.41 | 1.82 | 5.17 | 93.61 | 1.23 |
| | 5.5 | 1.33 | 97.55 | 1.12 | 12.72 | 85.10 | 2.18 | 8.63 | 89.87 | 1.51 |
| Citric acid-sodium citrate | 6.5 | 3.53 | 95.30 | 1.17 | 8.39 | 89.98 | 1.65 | 23.06 | 75.08 | 1.87 |
| | 7.0 | 4.5 | 94.41 | 1.10 | 9.13 | 89.06 | 1.81 | 47.66 | 50.89 | 1.47 |

After replacing SWY2110-JSSW-001 with the antibody-drug conjugates SWY2111-JSSW-001 and SWY2113-JSSW-001, the stability of the antibody-drug conjugates in the buffer was investigated using the same method described above. The test results were substantially similar to those of SWY2110-JSSW-001. The 25 mM histidine-histidine hydrochloride at pH=5.0 was selected as the target buffer.

Formulation Example 2

Saccharides are widely used stabilizers in monoclonal antibody and ADC products, serving to stabilize monoclonal antibody drugs, and can also be used as excipients for lyophilized products, while also having the function

of adjusting the osmotic pressure of the product.

The 25 mM histidine-histidine hydrochloride buffer at pH=5.0 was prepared, and sucrose or trehalose was added separately to prepare 4 groups of solutions with different saccharides and saccharide concentrations (5% or 9%). The antibody-drug conjugate (SWY2110-JSSW-001) sample was exchanged into the above 4 groups of solutions through ultrafiltration, with a final concentration of the antibody-drug conjugate of approximately 10 mg/ml. The thermal stability of the initial samples was detected by differential scanning calorimetry (DSC). The samples were placed under high temperature (40±2°C) conditions and sampled on the 7th and 14th days; they were placed under light exposure (5 ±3°C, white light illuminance: 5000 Lux, UV intensity: 90 μw/$cm^2$) conditions and sampled on the 10th day. The molecular size variants of the antibody-drug conjugates were detected by size exclusion chromatography (SEC-HPLC), and the charge variants of the antibody-drug conjugates were detected by ion exchange chromatography (CEX-HPLC) to investigate the stability of the antibody-drug conjugates in the 8 groups of solutions after high temperature and light exposure treatments. Additionally, under the same lyophilization process, the appearances of two lyophilized powders with 9% sucrose and trehalose were compared.

DSC test results showed that $T_{onset}$ and $Tm_2$ of the test groups with 9% saccharide added were significantly higher than those of the test groups with 5% saccharide. There was no significant difference between sucrose and trehalose. The SEC test results indicated that when the saccharide concentration was raised from 5% to 9%, the level of SEC monomer content remained significantly high.. The CEX test results showed that in the high temperature test, neither the addition of saccharide nor its concentration had a significant effect on the main peak purity; whereas in the light exposure test, significant changes occurred in the acidic and basic variants of the sample, and the chromatogram thereof could no longer distinguish the acidic peak, main peak, and basic peak. Therefore, the CEX data were not statistically analyzed. Comparing the morphology of lyophilized powders with different stabilizers, the shrinkage at the bottom of lyophilized powder from the sucrose test group was significantly more severe than that from the trehalose test group. In summary, the addition of 9% trehalose was selected for the stabilizer.

Detection method

DSC: A sample to be tested was diluted with the same buffer therein to a protein concentration of 1 mg/mL. Then, 350 μL each of the test sample and the corresponding buffer were added to the designated wells of a 96-well plate. The 96-well plate was placed into a sample holder, and the temperature of the sample holder was set to 15°C. Instrument parameters were set as follows: initial temperature: 20°C, final temperature: 100°C, and heating rate: 90°C/min.

SEC-HPLC: Detection method was identical to that in Formulation Example 1.

CEX-HPLC: According to Chinese Pharmacopoeia 2020 edition, Part IV, General Rule 0512 High Performance Liquid Chromatography, analysis and detection were performed using a cation exchange column as the chromatographic column. The proportions of the acidic peak, main peak, and basic peak of the test sample were calculated according to the area normalization method.

Detection results:

Table 34: DSC detection results

| Item | $T_{onset}$(°C) | $Tm_1$(°C) | $Tm_2$(°C) | $Tm_3$(°C) |
|---|---|---|---|---|
| pH5.0 5% sucrose | 48.87 | 58.45 | 70.24 | 79.17 |
| pH5.0 9% sucrose | 49.61 | 59.55 | 70.82 | 79.70 |
| pH5.0 5% trehalose | 48.77 | 58.19 | 70.24 | 79.18 |
| pH5.0 9% trehalose | 49.22 | 59.09 | 70.82 | 79.76 |

Table 35: SEC-HPLC detection result

| Item | SEC-HPLC purity (%) | | | | | | | | | | | |
|---|---|---|---|---|---|---|---|---|---|---|---|---|
| | 0d | | | 40°C, high temperature, 7d | | | 40°C, high temperature, 14d | | | 5°C, light exposure, 10d | | |
| | Aggregate | Main peak | Fragment | Aggregate | Main peak | Fragment | Aggregate | Main peak | Fragment | Aggregate | Main peak | Fragment |
| pH5.0 5% sucrose | 2.20 | 95.83 | 1.97 | 6.21 | 91.11 | 2.67 | 11.45 | 84.75 | 3.81 | 10.75 | 86.93 | 2.32 |
| pH5.0 9% sucrose | 2.16 | 95.88 | 1.96 | 5.01 | 92.42 | 2.58 | 7.99 | 88.35 | 3.66 | 9.69 | 87.34 | 2.97 |
| pH5.0 5% trehalose | 2.28 | 95.69 | 2.02 | 7.92 | 89.51 | 2.57 | 11.29 | 84.93 | 3.77 | 10.89 | 86.18 | 2.93 |
| pH5.0 9% trehalose | 2.06 | 95.88 | 2.05 | 5.55 | 91.82 | 2.63 | 8.18 | 88.07 | 3.76 | 10.65 | 86.12 | 3.23 |

Table 36 CEX-HPLC detection result

| Item | CEX-HPLC concentration (%) | | | | | | | | |
|---|---|---|---|---|---|---|---|---|---|
| | 0d | | | 40°C, high temperature, 7d | | | 40°C, high temperature, 14d | | |
| | Acidic peak | Main peak | Basic peak | Acidic peak | Main peak | Basic peak | Acidic peak | Main peak | Basic peak |
| pH5.0 5% sucrose | 25.55 | 44.75 | 29.70 | 28.40 | 43.78 | 27.82 | 32.58 | 37.60 | 29.82 |
| pH5.0 9% sucrose | 26.44 | 43.99 | 29.57 | 28.33 | 43.89 | 27.78 | 32.84 | 37.38 | 29.78 |
| pH5.0 5% trehalose | 26.41 | 44.35 | 29.24 | 27.74 | 43.72 | 28.54 | 32.63 | 37.71 | 29.66 |
| pH5.0 9% trehalose | 26.43 | 44.80 | 28.77 | 27.74 | 44.65 | 27.61 | 33.17 | 37.53 | 29.30 |

Table 37: Detection results for basic evaluation indicators of lyophilized powders

| Stabilizer | Appearance of lyophilized powder | Moisture content (%) | Reconstitution time |
|---|---|---|---|
| 9% trehalose | White loose solid, slight shrinkage at the bottom | 1.45 | ≤1min |
| 9% sucrose | White loose solid, moderately severe shrinkage at the bottom | 1.85 | ≤1min |

After replacing SWY2110-JSSW-001 with the antibody-drug conjugates SWY2111-JSSW-001 and SWY2113-JSSW-001, the effect of the stabilizer on the antibody-drug conjugates was investigated using the same method described above. The test results were substantially similar to those of SWY2110-JSSW-001. Trehalose was selected as the stabilizer.

Formulation Example 3

25 mM histidine-histidine hydrochloride buffer at pH=5.0 was prepared, and trehalose was added at 9%. The antibody-drug conjugate (SWY2110-JSSW-001) was exchanged into the above solution through ultrafiltration and polysorbate 80 was added to prepare samples with a final concentration of the antibody-drug conjugate of approximately 10 mg/ml. The samples were dispensed into injection vials, and a stability study was conducted under freezing-thawing conditions (frozen at -20°C and thawed at room temperature for a total of 6 cycles). Changes in subvisible particles in samples under different conditions were detected using a microflow imaging instrument (Flowcam). The results showed that adding polysorbate 80 as the surfactant could effectively control the increase in the number of particles. Adding 0.01% polysorbate 80 showed a significant inhibitory effect on the formation of particles. Adding 0.03% polysorbate 80 as the surfactant was preferred.

Table 38: Effect of freezing-thawing on insoluble particles in samples with different addition amounts of polysorbate 80

| PS80 Content / Particle | | 0 | | 0.01% | | 0.03% | | 0.05% | |
|---|---|---|---|---|---|---|---|---|---|
| | | T0 | 6 cycles of freezing-thawing | T0 | 6 cycles of freezing-thawing | T0 | 6 cycles of freezing-thawing | T0 | 6 cycles of freezing-thawing |
| Particle number (P/mL) | NSP 2-10μm | 2481 | 15799 | 62 | 72 | 49 | 119 | 7 | 25 |
| | NSP 10+μm | 1008 | 15410 | 7 | 15 | 0 | 10 | 0 | 5 |

After replacing SWY2110-JSSW-001 with the antibody-drug conjugates SWY2111-JSSW-001 and SWY2113-JSSW-001, the effect of the surfactant on the stability of the antibody-drug conjugates was investigated using the same method described above. The test results were substantially similar to those of SWY2110-JSSW-001. Polysorbate 80 was selected as the surfactant.

Formulation Example 4

A formula of 10 mg/ml of the antibody-drug conjugate (SWY2110-JSSW-001), 25 mmol/L of histidine-histidine hydrochloride, 0.03% of polysorbate 80, and 9% of trehalose at pH=5.0 was selected as base formula. The glass transition temperature and collapse temperature of this base formula were detected. Low-temperature DSC detection showed the glass transition temperature of the sample was -29.28°C, and the lyophilization microscopy detection showed the collapse temperature of the sample was -25.7°C. Pre-freezing: Based on the glass transition temperature, the pre-freezing temperature was set to -45°C. First, the shelf temperature was rapidly lowered to -5°C and held at this temperature for 30 minutes. Then the product was cooled to the pre-freezing temperature of -45°C either rapidly (2°C/min) or slowly (0.5°C/min) and maintained at this temperature for at least 180 minutes. The lyophilization processes were subsequently carried out under the same primary and secondary drying conditions. No significant difference in moisture contents of the lyophilized powders was observed between two pre-freezing methods. The rapid freezing led to the formation of smaller ice crystals, but it did not increase the moisture content compared with the slow freezing; therefore, the rapid freezing was preferably selected for the pre-freezing.

Table 39: Effect of rapid and slow freezing at different positions on moisture content

| Sample Position Number | Moisture content (%) | |
|---|---|---|
| | rapid freezing (2°C/min) | slow freezing (0.5°C/min) |
| A | 1.10 | 1.19 |
| B | 1.17 | 1.16 |
| C | 1.22 | 1.25 |
| D | 1.16 | 1.20 |
| E | 1.10 | 1.19 |

(continued)

| Sample Position Number | Moisture content (%) | |
|---|---|---|
| | rapid freezing (2°C/min) | slow freezing (0.5°C/min) |
| Average | 1.44 | 1.39 |
| Note: C was the center position of the shelf, A was a corner of the shelf near the door, E was the diagonal corner of A near the isolation valve, B and D were the midpoints of AC and CE, respectively. | | |

Primary drying: The shelf temperature was set above the allowable collapse temperature of the product, ensuring that the temperature of the non-sublimed portion of the product did not exceed its collapse temperature. The rapid freezing was preferably used in the pre-freezing process. At the primary drying temperature of -20°C, the lyophilization effects under the vacuum values of the lyophilization chamber of 0.20 mbar and 0.25 mbar were compared. Both batches of lyophilized powders were white cake-like loose solids, showing no collapse or significant shrinkage, with essentially identical moisture contents and reconstitution times. With essentially the same moisture contents of the lyophilized powders, the primary drying time at -20°C and 0.20 mbar was 200 minutes shorter than that at -20°C and 0.25 mbar. Therefore, the primary drying was preferably carried out at -20°C and 0.20 mbar.

Table 40: Morphology, moisture content, and reconstitution time of lyophilized powders under different primary drying pressures

| Batch number | DP221 | DP228 |
|---|---|---|
| Primary drying condition | -20°C 0.25mbar 4000min | -20°C 0.20mbar 3800min |
| Moisture content of sample A | 0.84% | 1.10% |
| Moisture content of sample B | 1.12% | 1.17% |
| Moisture content of sample C | 1.20% | 1.22% |
| Average moisture content | 1.09% | 1.16% |
| Lyophilized powder morphology | White, cake-like, loose solid | White, cake-like, loose solid |
| Reconstitution time | ≤1min | ≤1min |
| Note: C was the center position of the shelf, A was a corner near the door, B was the midpoint of AC. Secondary drying: After the completion of primary drying, the shelf temperature was set below the maximum allowable temperature for the product. The secondary drying temperature was set to 30°C and the chamber pressure was set to 0.20 mbar, consistent with the primary drying condition. | | |

The key qualities of the lyophilized samples were compared before and after lyophilization. The results showed no significant changes in product quality after lyophilization, indicating that the lyophilization process did not have a significant impact on the product quality.

Table 41: Impact of lyophilization process on the product quality

| Item | Stock solution batch number: DS218 | | Stock solution batch number: DS228 | |
|---|---|---|---|---|
| | Stock solution (DS218) | Reconstitution solution of lyophilized powder (DP221) | Stock solution (DS228) | Reconstitution solution of lyophilized powder (DP228) |
| Moisture content (%) | - | 1.1 | - | 1.2 |
| Reconstitution time (min) | - | <3min | - | <3min |
| pH value | 5.0 | 5.0 | 5.0 | 5.0 |
| Protein content (mg/ml) | 10.1 | 9.8 | 9.9 | 10.2 |

(continued)

| Item | Stock solution batch number: DS218 | | Stock solution batch number: DS228 | |
|---|---|---|---|---|
| | Stock solution (DS218) | Reconstitution solution of lyophilized powder (DP221) | Stock solution (DS228) | Reconstitution solution of lyophilized powder (DP228) |
| SEC-HPLC | HMWS: 1.2% | 1.1% | HMWS: 1.3% | 1.3% |
| | Monomer: 97.4% | 97.5% | Monomer: 96.7% | 96.6% |
| | LMWS: 1.4% | 1.4% | LMWS: 2.0% | 2.2% |
| CEX-HPLC | Acidic peak: 24.2% | 24.3% | Acidic peak: 22.5% | 22.5% |
| | Main peak: 47.5% | 48.4% | Main peak: 47.2% | 48.5% |
| | Basic peak: 28.3% | 27.3% | Basic peak: 30.3% | 29.0% |
| NR-CE-SDS | Monomer: 97.2% | 97.6% | Monomer: 97.1% | 96.5% |
| | LMWS: 2.8% | 2.4% | LMWS: 2.9% | 3.5% |
| DAR | DAR value: 8.0 | 8.0 | DAR value: 8.0 | 8.0 |
| Antigen binding activity | 86% | 106% | 100% | 105% |
| Bioactivity | 94% | 96% | 100% | 114% |

After replacing SWY2110-JSSW-001 with the antibody-drug conjugates SWY2111-JSSW-001 and SWY2113-JSSW-001, the impact of the lyophilization process on the product quality was investigated using the same method described above. The test results were substantially similar to those of SWY2110-JSSW-001, indicating that the lyophilization process did not have a significant impact on the product quality.

Formulation Example 5

Stability studies at 5±3°C were carried out separately on the stock solution of the antibody-drug conjugate (SWY2110-JSSW-001) before lyophilization and the finished product of the antibody-drug conjugate (SWY2110-JSSW-001) after lyophilization. The study results showed that the quality of the product in the lyophilized state was more stable than that in the liquid state. The product in the liquid state showed significantly increased high molecular-weight fragments than the product in the lyophilized state. Moreover, the product in the liquid state was more prone to generating free small molecule payloads, with an obviously higher releasing rate compared to the lyophilized powder. The lyophilized dosage form was more conducive to the long-term storage of the product.

Detection methods:

SEC-HPLC: According to Chinese Pharmacopoeia 2020 edition, Part IV, General Rule 0512 High Performance Liquid Chromatography, analysis and detection were performed using a chromatographic column packed with a gel suitable for separating proteins with a molecular weight of 10-500 kD (e.g., Xbridge® BEH200Å, 7.8×300mm, 200Å, 3.5μm or other suitable chromatographic columns). The mobile phase included 100 mmol/L phosphate buffer, 100 mmol/L NaCl, and 10% isopropanol at pH=6.7±0.1, the flow rate was 0.8 mL/min, and the detection wavelength was 280 nm. An appropriate amount of the test sample solution was injected into the liquid chromatograph. The proportions of the protein monomer peak, the high molecular weight substance (HMWS) peak and the low molecular weight substance (LMWS) peak were calculated according to the area normalization method.

CEX-HPLC: According to Chinese Pharmacopoeia 2020 edition, Part IV, General Rule 0512 High Performance Liquid Chromatography, analysis and detection were performed using an ion exchange column (e.g., BioPro IEX SF, 4.6×100mm or other suitable chromatographic columns). The mobile phase A included 20 mmol/L MES (morpholinoethanesulfonic acid) at pH=6.1, and the mobile phase B included 20 mmol/L MES and 500 mmol/L NaCl at pH=6.1, the flow rate was 1 mL/min, and the detection wavelength was 280 nm. An appropriate amount of the test sample solution was injected into the liquid chromatograph for gradient elution. The proportions of the acidic peak, main peak, and basic peak of the test sample were calculated according to the area normalization method.

NR-CE-SDS: Detection was performed according to Chinese Pharmacopoeia 2020 edition, Part IV, General Rule 3127 Determination of Molecular Size Variants of Monoclonal Antibodies. The proportions of the monomer peak and

low molecular weight substance (LMWS) peak were calculated according to the area normalization method.

icIEF: Detection was performed according to Chinese Pharmacopoeia 2020 edition, Part IV, General Rule 3129, Determination of Charge Variants of Monoclonal Antibodies. Based on the isoelectric point (PI) characteristics of different charge variants, they were separated by capillary electrophoresis (General Rule 0542). The isoelectric points of the charge variants were determined and their relative percentages were calculated.

DAR distribution: According to Chinese Pharmacopoeia 2020 edition, Part IV, General Rule 0512 High Performance Liquid Chromatography, analysis and detection were performed using a hydrophobic chromatographic column (e.g., TSKgel Butyl-NPR, 4.6×100mm or other suitable columns) as the chromatographic column. The mobile phase A included 1.0M ammonium sulfate and 25mM phosphate buffer (pH=7.0), and the mobile phase B included 25% isopropanol and 75% 25mM phosphate buffer (pH=7.0). The flow rate was 0.8 ml/min, and the detection wavelength was 280 nm. An appropriate amount of the test sample solution was injected into the liquid chromatograph for gradient elution. The DAR value was calculated.

Free small molecule payload content: According to Chinese Pharmacopoeia 2020 edition, Part IV, General Rule 0512 High Performance Liquid Chromatography and 0431 Mass Spectrometry, analysis and detection were performed using a chromatographic column packed with an octadecylsilyl silica gel (e.g., Waters ACQUITY UPLC® BEH-C18, 2.1×50mm, particle size 1.7μm or other suitable chromatographic columns).The mobile phase A was an aqueous solution containing 0.1% FA, and the mobile phase B was an acetonitrile solution containing 0.1% FA. The column temperature was 40°C, and the flow rate was 0.4 ml/min. 50μL of the test sample (10 mg/mL) was taken, and an internal standard working solution was added. The mixture was precipitated with acetonitrile in the ratio of 1:3, placed in a -20°C ice bath for 30 minutes, and then centrifuged. The supernatant was taken and loaded onto the column. Different concentrations of small molecule payloads were used as reference standards and loaded onto the column. After gradient elution by liquid chromatography, the sample was sent to the mass spectrometer detector. After setting parameters such as capillary and cone voltages, ion source temperature, collision energy, and nebulizer flow rate, the analysis was performed. The free small molecule payload content in the test sample was calculated according to the standard curve.

Free small molecule payloads content (%) = Detection concentration of free small molecule payloads/ADC concentration × 100%

Antigen binding activity: Determined by enzyme-linked immunosorbent assay (ELISA).
Bioactivity: Determined by cell proliferation inhibition assay.

Detection results:

Table 42: Stability results for key quality indicators of GMP Batch 1

| Items | GMP Batch 1 | | | |
|---|---|---|---|---|
| | Stock solution (5±3°C) | | Lyophilized powder (5±3°C) | |
| | 0 months | 6 months | 0 months | 6 months |
| SEC-HPLC | HMWS: 1.4% | 2.1% | HMWS: 1.4% | 1.1% |
| | Monomer: 97.4% | 96.2% | Monomer: 97.4% | 97.4% |
| | LMWS: 1.3% | 1.7% | LMWS: 1.3% | 1.5% |
| NR-CE-SDS | LC+HC: 97.0% | 97.3% | HC+LC: 97.3% | 97.5% |
| | Other peak: 3.0% | 2.7% | Other peak: 2.7% | 2.5% |
| CEX-HPLC | Acidic peak: 25.1% | 20.6% | Acidic peak: 23.8% | 19.9% |
| | Main peak: 47.1% | 46.2% | Main peak: 49.1% | 50.8% |
| | Basic peak: 27.8% | 33.2% | Basic peak: 27.1% | 29.3% |
| icIEF | Acidic peak: 35.4% | 32.1% | Acidic peak: 35.1% | 30.8% |
| | Main peak: 40.9% | 41.3% | Main peak: 41.3% | 41.7% |
| | Basic peak: 23.6% | 26.6% | Basic peak: 23.6% | 27.6% |

(continued)

| Items | GMP Batch 1 | | | |
|---|---|---|---|---|
| | Stock solution (5±3°C) | | Lyophilized powder (5±3°C) | |
| | 0 months | 6 months | 0 months | 6 months |
| DAR | DAR value: 8.0 | 8.0 | DAR value: 8.0 | 8.0 |
| Free small molecule payloads | 0.000037% | 0.0010% | 0.00018% | 0.00050% |
| Antigen binding activity | 105% | 103% | 96% | 97% |
| Bioactivity | 102% | 101% | 93% | 103% |

Table 43: Stability results for key quality indicators of GMP Batch 2

| Items | GMP Batch 2 | | | |
|---|---|---|---|---|
| | Stock solution (5±3°C) | | Lyophilized powder (5±3°C) | |
| | 0 months | 6 months | 0 months | 6 months |
| SEC-HPLC | HMWS: 0.9% | 1.8% | HMWS: 0.9% | 1.1% |
| | Monomer: 96.8% | 96.5% | Monomer: 96.7% | 97.3% |
| | LMWS: 2.3% | 1.7% | LMWS: 2.3% | 1.6% |
| NR-CE-SDS | LC+HC: 97.0% | 97.5% | HC+LC: 97.3% | 97.2% |
| | Other peak: 3.0% | 2.5% | Other peak: 2.7% | 2.8% |
| CEX-HPLC | Acidic peak: 21.9% | 19.6% | Acidic peak: 22.3% | 20.0% |
| | Main peak: 46.4% | 46.9% | Main peak: 46.6% | 50.8% |
| | Basic peak: 31.7% | 33.5% | Basic peak: 31.2% | 29.2% |
| icIEF | Acidic peak: 33.0% | 31.9% | Acidic peak: 34.0% | 30.0% |
| | Main peak: 42.8% | 41.4% | Main peak: 42.8% | 43.4% |
| | Basic peak: 24.3% | 26.7% | Basic peak: 23.2% | 26.6% |
| DAR | DAR value: 8.0 | 8.0 | DAR value: 8.0 | 8.0 |
| Free small molecule payloads | 0.000048% | 0.0012% | 0.00078% | 0.00059% |
| Antigen binding activity | 106% | 104% | 103% | 100% |
| Bioactivity | 94% | 104% | 98% | 101% |

After replacing SWY2110-JSSW-001 with antibody-drug conjugates SWY2111-JSSW-001 and SWY2113-JSSW-001, stability studies at 5±3°C were carried out separately on the stock solutions of the antibody-drug conjugates before lyophilization and the finished products of the antibody-drug conjugates after lyophilization using the same method described above. The study results and conclusions were substantially similar to those of SWY2110-JSSW-001, i.e., the quality of the product in the lyophilized state was more stable than that in the liquid state. The product in the liquid state showed significantly increased high molecular-weight fragments than the product in the lyophilized state. Moreover, the product in the liquid state was more prone to generating free small molecule payloads, with an obviously higher releasing rate compared to the lyophilized powder. The lyophilized dosage form was more conducive to the long-term storage of the product.

Formulation Example 6

The antibody-drug conjugate (patritumab-JSSW-001) was respectively exchanged into 25mM histidine-histidine hydrochloride buffer, citric acid-sodium citrate buffer and succinic acid-sodium hydroxide buffer at pH=5.0, 5.5, 6.0 and 6.5. The dilution was carried out to the extent that the protein concentration was about 20 mg/ml. Screening tests were conducted under high temperature (40±2°C, 75%±5% RH) and light exposure (5±3°C, white light: 5000±500 Lux, UV: 90 $\mu W/cm^2$). Table 44 shows the appearance changes of the antibody-drug conjugate during the high temperature and light exposure tests. After 3 days of light exposure, the appearance of all 12 test groups changed significantly. The sodium

citrate test groups and the succinate sodium test groups at pH=5.5, 6.0, and 6.5 showed phase separation or turbidity. The histidine test groups and the succinate sodium test group at pH=5.0 appeared slightly yellow. After 5 days at high temperature, except for the sodium citrate test groups at pH=6.0 and 6.5 which became turbid, the other test groups remained clear. The histidine test groups at pH=5.0, 5.5 and 6.0 and the succinate sodium test group at pH=5.0 were selected for the determination of SEC data after light exposure and high temperature tests. Table 45 shows the SEC changes for the pH and buffer salt type screening. After 5 days at high temperature, there was no significant difference in the SEC main peak among the four test groups, whereas after 3 days of light exposure, the SEC main peak of the histidine test group at pH=5.0 was significantly higher than that of the other test groups. Compared to high temperature, the antibody-drug conjugates are more sensitive to light. Histidine-histidine hydrochloride at pH=5.0 was an optimal buffer system.

Detection methods:

SEC-HPLC: According to Chinese Pharmacopoeia 2020 edition, Part IV, General Rule 0512 High Performance Liquid Chromatography, analysis and detection were performed using a chromatographic column packed with a gel suitable for separating proteins with a molecular weight of 10-500 kD. The proportions of the protein monomer peak, the high molecular weight substance (HMWS) peak and the low molecular weight substance (LMWS) peak were calculated according to the area normalization method.

Detection results:

Table 44: Appearance result

| Buffer | 0d | Light exposure, 3d | High temperature, 5d |
|---|---|---|---|
| pH5.0H | Clear | Slightly yellow | Clear |
| pH5.5H | Clear | Slightly yellow | Clear |
| pH6.0H | Clear | Slightly yellow | Clear |
| pH6.5H | Clear | Turbid | Clear |
| pH5.0C | Clear | Upper layer colorless, lower layer yellow | Clear |
| pH5.5C | Clear | Upper layer colorless, lower layer yellow | Clear |
| pH6.0C | Clear | Upper layer turbid, lower layer yellow | Slightly turbid |
| pH6.5C | Clear | Upper layer turbid, lower layer yellow | Turbid |
| pH5.0S | Clear | Slightly yellow | Clear |
| pH5.5S | Clear | Turbid | Clear |
| pH6.0S | Clear | Turbid | Clear |
| pH6.5S | Clear | Turbid | Clear |
| Note: H: histidine, C: sodium citrate, S: sodium succinate | | | |

Table 45: SEC-HPLC detection result

| Buffer | SEC-HPLC purity (%) | | | | | | | | |
|---|---|---|---|---|---|---|---|---|---|
| | 0d | | | 5°C, light exposure, 3d | | | 40°C, high temperature, 5d | | |
| | Aggregate (%) | Main peak (%) | Fragment (%) | Aggregate (%) | Main peak (%) | Fragment (%) | Aggregate (%) | Main peak (%) | Fragment (%) |
| pH5.0H | 1.11 | 98.17 | 0.72 | 28.96 | 70.15 | 0.89 | 3.29 | 95.81 | 0.9 |
| pH5.5H | 1.41 | 97.89 | 0.7 | 42.91 | 56.22 | 0.87 | 3.26 | 96.02 | 0.72 |
| pH6.0H | 1.37 | 97.99 | 0.64 | 55.91 | 42.92 | 1.17 | 7.51 | 91.44 | 1.05 |
| pH5.0S | 1.59 | 97.69 | 0.72 | 32.95 | 63.63 | 3.42 | 3.94 | 95.43 | 0.63 |

Note: H: histidine, S: sodium succinate

Formulation Example 7

The antibody-drug conjugate (patritumab-JSSW-001) was respectively exchanged into different concentrations (15 mM, 25 mM, 35 mM) of histidine-histidine hydrochloride buffers containing 6% sucrose at pH=5.0. The dilution was carried out to the extent that the protein concentration was about 20 mg/ml. Screening tests were conducted under high temperature and light exposure conditions. SEC and CEX were determined to screen for the optimal histidine concentration. Table 46 shows the DSC data for screening the histidine concentrations. Table 47 shows the SEC data for screening the histidine concentrations. In the light exposure test, the SEC main peak decreased significantly as the histidine concentration increased; in the high temperature test, the SEC main peak decreased slightly as the histidine concentration increased; due to the relatively strong photosensitivity of the antibody-drug conjugate, the CEX chromatogram from the light exposure test could no longer distinguish the acidic peak, main peak, and basic peak, so only the CEX data from the high temperature test were statistically analyzed. Table 48 shows the CEX main peak data from the high temperature test for screening the histidine concentrations. The results indicated that histidine concentrations had little effect on the CEX main peak. Based on the SEC and CEX data, 15 mM was an optimal concentration for histidine.

Detection method

DSC: A sample to be tested was diluted with the same buffer therein to a protein concentration of 1 mg/mL. Then, 350 μL each of the test sample and the corresponding buffer were added to the designated wells of a 96-well plate. The 96-well plate was placed into a sample holder, and the temperature of the sample holder was set to 15°C. Instrument parameters were set as follows: initial temperature: 20°C, final temperature: 100°C, and heating rate: 90°C/min.

SEC-HPLC: Detection method was identical to that in Formulation Example 6.

CEX-HPLC: According to Chinese Pharmacopoeia 2020 edition, Part IV, General Rule 0512 High Performance Liquid Chromatography, analysis and detection were performed using a cation exchange column as the chromatographic column. The proportions of the acidic peak, main peak, and basic peak of the test sample were calculated according to the area normalization method.

Detection results:

Table 46: DSC detection results

| Histidine concentration | $T_{onset}$(°C) | $Tm_1$(°C) | $Tm_2$(°C) |
|---|---|---|---|
| 15mM | 51.3 | 58.3 | 78.5 |
| 25mM | 50.2 | 58.7 | 77.2 |
| 35mM | 49.7 | 60.7 | 77.2 |

Table 47: SEC-HPLC detection result

| Histidine concentration | 0d SEC | | | Light exposure 5d SEC | | | Light exposure 10d SEC | | |
|---|---|---|---|---|---|---|---|---|---|
| | Aggregate (%) | Main peak (%) | Fragment (%) | Aggregate (%) | Main peak (%) | Fragment (%) | Aggregate (%) | Main peak (%) | Fragment (%) |
| 15mM | 0.96 | 98.48 | 0.56 | 12.50 | 85.21 | 2.29 | 13.14 | 84.08 | 2.78 |
| 25mM | 1.03 | 98.39 | 0.58 | 14.89 | 83.14 | 1.97 | 15.61 | 81.89 | 2.50 |
| 35mM | 1.09 | 98.33 | 0.58 | 18.63 | 79.33 | 2.04 | 19.43 | 78.00 | 2.57 |
| Histidine concentration | 0d SEC | | | High temperature 7d SEC | | | High temperature 14d SEC | | |
| | Aggregate (%) | Main peak (%) | Fragment (%) | Aggregate (%) | Main peak (%) | Fragment (%) | Aggregate (%) | Main peak (%) | Fragment (%) |
| 15mM | 0.96 | 98.48 | 0.56 | 1.37 | 97.61 | 1.02 | 1.55 | 97.24 | 1.21 |
| 25mM | 1.03 | 98.39 | 0.58 | 1.81 | 97.10 | 1.09 | 2.58 | 96.05 | 1.37 |
| 35mM | 1.09 | 98.33 | 0.58 | 2.08 | 96.82 | 1.10 | 2.97 | 95.67 | 1.36 |

Table 48 CEX-HPLC detection result

| Histidine concentration | 0d CEX | | | High temperature 7d CEX | | | High temperature 14d CEX | | |
|---|---|---|---|---|---|---|---|---|---|
| | Acidic peak (%) | Main peak (%) | Basic peak (%) | Acidic peak (%) | Main peak (%) | Basic peak (%) | Acidic peak (%) | Main peak (%) | Basic peak (%) |
| 15mM | 38.35 | 58.3 | 3.35 | 44.26 | 52.13 | 3.61 | 51.64 | 44.42 | 3.94 |
| 25mM | 37.79 | 58.97 | 3.24 | 44.15 | 51.94 | 3.91 | 51.62 | 43.59 | 4.79 |
| 35mM | 38.05 | 57.07 | 4.88 | 43.19 | 53.4 | 3.41 | 49.25 | 46.03 | 4.72 |

Formulation Example 8

15 mM histidine-histidine hydrochloride buffer at pH=5.0 was prepared, and sucrose or trehalose was added

separately at each of 4%, 6%, 8%, and 10%. The antibody-drug conjugate (patritumab-JSSW-001) was exchanged through ultrafiltration into the above 8 buffers, with the protein concentration diluted to approximately 20 mg/ml. After filtering through a 0.22 μm microporous filter membrane, the thermal stability of the antibody-drug conjugate was detected by DSC, and stability tests were performed under high temperature and light exposure.

Table 49 shows the thermal stability data of the antibody-drug conjugate in buffer systems with different concentrations of sucrose and trehalose. The type and addition amount of the stabilizer had no significant effect on the thermal denaturation onset temperature Tonset (°C), while the test group with the highest Tm1 value was the 8% sucrose group. Table 50 shows the effect of high temperature and light exposure on the SEC monomer of the antibody-drug conjugate in buffer systems with different types and concentrations of stabilizers. After 14 days at high temperature, the SEC monomer of the 4% test group was significantly lower than that of the other test groups. Under the same saccharide concentration, there was no significant difference in SEC between the sucrose and trehalose groups in the high temperature test. Table 51 shows the effect of high temperature on the CEX main peak of the antibody-drug conjugate in buffer systems with different types and concentrations of stabilizers. After the high temperature test, there was no significant difference in the CEX main peak among the 8 test groups. In summary, 8% sucrose or trehalose could be added as a stabilizer. Considering that the cost of trehalose was higher than that of sucrose, 8% sucrose was selected for this product.

Table 49: DSC detection results

| Stabilizer | $T_{onset}$(°C) | $Tm_1$(°C) | $Tm_2$(°C) |
|---|---|---|---|
| 4% sucrose | 51.3 | 59.0 | 77.8 |
| 6% sucrose | 51.7 | 59.4 | 78.0 |
| 8% sucrose | 51.5 | 61.4 | 78.2 |
| 10% sucrose | 52.5 | 59.0 | 78.7 |
| 4% trehalose | 50.8 | 59.0 | 77.8 |
| 6% trehalose | 51.1 | 59.2 | 78.0 |
| 8% trehalose | 52.5 | 58.4 | 78.6 |
| 10% trehalose | 51.7 | 60.0 | 78.5 |

Table 50 SEC detection result

| Stabilizer | 0d SEC | | | High temperature 7d SEC | | | High temperature 14d SEC | | |
|---|---|---|---|---|---|---|---|---|---|
| | Aggregate (%) | Main peak (%) | Fragment (%) | Aggregate (%) | Main peak (%) | Fragment (%) | Aggregate (%) | Main peak (%) | Fragment (%) |
| 4% sucrose | 1.00 | 98.22 | 0.78 | 1.31 | 97.50 | 1.19 | 2.26 | 95.87 | 1.87 |
| 6% sucrose | 1.02 | 98.26 | 0.72 | 1.18 | 97.63 | 1.19 | 2.04 | 96.18 | 1.78 |
| 8% sucrose | 0.99 | 98.26 | 0.75 | 1.13 | 97.60 | 1.27 | 1.97 | 96.26 | 1.77 |
| 10% sucrose | 1.01 | 98.27 | 0.72 | 1.18 | 97.60 | 1.22 | 1.84 | 96.41 | 1.75 |
| 4% trehalose | 1.08 | 98.11 | 0.81 | 1.32 | 97.51 | 1.17 | 2.38 | 95.82 | 1.80 |
| 6% trehalose | 1.05 | 98.18 | 0.77 | 1.21 | 97.71 | 1.08 | 2.07 | 96.24 | 1.69 |
| 8% trehalose | 1.06 | 98.12 | 0.82 | 1.29 | 97.51 | 1.20 | 2.04 | 96.29 | 1.67 |
| 10% trehalose | 1.06 | 98.08 | 0.86 | 1.24 | 97.58 | 1.18 | 1.98 | 96.23 | 1.79 |
| Stabilizer | 0d SEC | | | Light exposure 5d SEC | | | Light exposure 10d SEC | | |
| | Aggregate (%) | Main peak (%) | Fragment (%) | Aggregate (%) | Main peak (%) | Fragment (%) | Aggregate (%) | Main peak (%) | Fragment (%) |
| 4% sucrose | 1.00 | 98.22 | 0.78 | 13.05 | 84.07 | 2.89 | 17.27 | 78.62 | 4.12 |
| 6% sucrose | 1.02 | 98.26 | 0.72 | 12.02 | 85.23 | 2.76 | 15.49 | 80.53 | 3.99 |
| 8% sucrose | 0.99 | 98.26 | 0.75 | 11.16 | 86.10 | 2.75 | 14.36 | 81.75 | 3.89 |
| 10% sucrose | 1.01 | 98.27 | 0.72 | 11.18 | 86.11 | 2.71 | 14.32 | 81.76 | 3.93 |
| 4% trehalose | 1.08 | 98.11 | 0.81 | 13.93 | 83.40 | 2.67 | 17.86 | 78.24 | 3.90 |
| 6% trehalose | 1.05 | 98.18 | 0.77 | 11.79 | 85.52 | 2.70 | 14.96 | 81.18 | 3.87 |
| 8% trehalose | 1.06 | 98.12 | 0.82 | 12.46 | 84.81 | 2.74 | 15.97 | 80.13 | 3.91 |
| 10% trehalose | 1.06 | 98.08 | 0.86 | 11.61 | 85.78 | 2.61 | 14.91 | 81.28 | 3.81 |

Table 51 CEX detection result

| Stabilizer | 0d | | | High temperature 7d CEX | | | High temperature 14d CEX | | |
|---|---|---|---|---|---|---|---|---|---|
| | Acidic peak (%) | Main peak (%) | Basic peak (%) | Acidic peak (%) | Main peak (%) | Basic peak (%) | Acidic peak (%) | Main peak (%) | Basic peak (%) |
| 4% sucrose | 35.03 | 61.54 | 3.43 | 47.22 | 51.18 | 1.6 | 52.09 | 45.85 | 2.06 |
| 6% sucrose | 37.48 | 59.32 | 3.2 | 46.92 | 51.74 | 1.34 | 53.74 | 44.28 | 1.98 |
| 8% sucrose | 36.05 | 59.81 | 4.14 | 47.09 | 51.37 | 1.54 | 53.09 | 45.06 | 1.85 |
| 10% sucrose | 37.62 | 59.46 | 2.92 | 47.24 | 51.11 | 1.65 | 53.16 | 44.91 | 1.93 |
| 4% trehalose | 36.71 | 59.66 | 3.63 | 45.85 | 52.37 | 1.78 | 53.36 | 44.44 | 2.20 |
| 6% trehalose | 37.82 | 58.85 | 3.33 | 46.34 | 51.89 | 1.77 | 52.68 | 45.52 | 1.80 |
| 8% trehalose | 37.97 | 58.52 | 3.51 | 45.37 | 52.95 | 1.68 | 52.94 | 45.29 | 1.77 |
| 10% trehalose | 35.85 | 60.6 | 3.55 | 46.52 | 51.74 | 1.74 | 53.47 | 44.81 | 1.72 |

Formulation Example 9

15 mM histidine-histidine hydrochloride buffer at pH=5.0 was prepared, and sucrose was added at 8%. The antibody-drug conjugate (patritumab-JSSW-001) sample was exchanged into the above solution through ultrafiltration. Different concentrations of polysorbate 80 were added to prepare samples with an antibody-drug conjugate concentration of 20 mg/ml. The samples were dispensed into injection vials, and a stability study was conducted under freezing-thawing conditions (frozen at -20°C and thawed at room temperature for a total of 6 cycles) and shaking (5°C, 200 rpm for 10 days). The changes in subvisible particles in the samples under different conditions were detected using a microflow imaging instrument (Flowcam). Table 52 shows the effect of freezing-thawing and shaking on insoluble particles in samples with different addition amounts of polysorbate 80. The results showed that the addition of 0.005% polysorbate 80 could significantly inhibit particle formation, while 0.02% polysorbate 80 was preferred for preventing aggregate formation of the antibody-drug conjugate during long-term storage.

Table 52: Effect of freezing-thawing and shaking on insoluble particles in samples with different addition amounts of polysorbate 80

| PS80 concentration | Particle number (P/mL) | | | | | |
|---|---|---|---|---|---|---|
| | T0 | | 6 cycles of freezing-thawing | | shaking 10d | |
| | NSP(2-10μm) | NSP(≥10μm) | NSP(2-10μm) | NSP(≥10μm) | NSP(2-10μm) | NSP(≥10μm) |
| 0% | 197 | 15 | 2306 | 254 | 5381 | 580 |
| 0.005% | 8 | 1 | 29 | 0 | 438 | 2 |
| 0.02% | 35 | 2 | 42 | 2 | 556 | 0 |
| 0.04% | 66 | 2 | 88 | 1 | 1225 | 0 |
| 0.06% | 38 | 1 | 86 | 0 | 416 | 0 |

Formulation Example 10: Lyophilization process development

A formula of 20 mg/ml of the antibody-drug conjugate (patritumab-JSSW-001), 15 mmol/L of histidine-histidine hydrochloride, 0.02% of polysorbate 80, and 8% of sucrose at pH=5.0 was selected as base formula. The glass transition temperature and collapse temperature of a sample having this base formula were detected. Low-temperature DSC detection showed the glass transition temperature of the sample was -28.79°C, and the lyophilization microscopy detection showed the collapse temperature of the sample was -28.1°C.

The pre-freezing temperature was pre-determined to -40°C. Before the pre-freezing temperature reached -40 °C, the shelf temperature was first brought to 5°C over 30 minutes and maintained at 5 °C for another 30 minutes to ensure

that all samples could start cooling from 5°C. Pre-freezing was performed at three cooling rates: 0.5°C/min, 2°C/min, and 1°C/min. The lyophilized powders obtained with different pre-freezing cooling rates were all white, cake-like, and loose solids. After adding 5.0 ml of water for injection, all three batches of lyophilized powders could be reconstituted to produce a clear liquid within 3 minutes. Table 53 shows the moisture content of lyophilized powders containing the antibody-drug conjugate from 5 different positions. The lyophilized powder obtained at the cooling rate of 0.5 °C/min had the lowest average moisture content.

Table 53: Comparison of moisture contents of lyophilized powders at different cooling rates of pre-freezing

| Sample position No. | Measured moisture content (%) | | |
|---|---|---|---|
| | Cooling rate: 0.5°C/min | Cooling rate: 1°C/min | Cooling rate: 2°C/min |
| A | 1.19 | 1.15 | 1.13 |
| B | 1.25 | 1.37 | 1.34 |
| C | 1.15 | 1.43 | 1.29 |
| D | 1.21 | 1.37 | 1.39 |
| E | 1.18 | 1.15 | 1.09 |
| Average | 1.19 | 1.28 | 1.24 |
| Note: C was the center position of the shelf, A was a corner of the shelf near the door, E was the diagonal corner of A near the isolation valve, B and D were the midpoints of AC and CE, respectively. | | | |

Primary drying: The shelf temperature was set above the allowable collapse temperature of the product, ensuring that the temperature of the non-sublimed portion of the product did not exceed its collapse temperature. With pre-freezing performed at a cooling rate of 0.5°C/min and secondary drying conducted at 35°C and 0.2 mbar for 900 minutes, lyophilization trials were carried out using five different shelf temperatures (-15°C, -12°C, -8°C, -5°C, and 0°C) for primary drying. The temperature curve of the sample with the probe was observed during the early stage of primary drying (before the temperature rise inflection point appears), in which the temperature of the frozen portion of the sample contacted by the probe was in the range from -28 to -30°C, none exceeding the collapse temperature (Tc: -28.1°C). All five batches ultimately yielded white loose lyophilized powder without severe collapse. After adding 5.0 ml of water for injection, all five batches of lyophilized powders could be reconstituted to produce a clear liquid within 3 minutes. Table 54 shows the primary drying time and moisture content of the lyophilized powder under different primary drying conditions. The primary drying time shortened as the set value of the shelf temperature increased, with the shortest time when the shelf temperature was set to 0°C. Under the same secondary drying condition (35°C, 0.2 mbar, 900 min), moisture contents of lyophilized powders obtained by different primary drying processes were compared. The moisture content of the lyophilized powder obtained through primary drying at 0°C (1.52%) was significantly higher than that at -5°C (1.26%). Therefore, the shelf temperature was set to -5°C for primary drying. At the primary drying temperature set at -5°C, the lyophilization effects at 0.15 mbar, 0.20 mbar, and 0.25 mbar were compared. The batch obtained under the vacuum value of the primary drying set at 0.25 mbar had the lowest moisture content. Therefore, the vacuum value of the lyophilization chamber of the primary drying was set to 0.25 mbar.

Table 54: Primary drying time and moisture content of the lyophilized powder under different primary drying conditions

| Primary drying condition | Primary drying time(min) | Moisture content(%) |
|---|---|---|
| -15°C, 0.2mbar | 2500 | 1.19 |
| -12°C, 0.2mbar | 2300 | 1.26 |
| -8°C, 0.2mbar | 2000 | 1.17 |
| -5°C, 0.2mbar | 1800 | 1.26 |
| 0°C, 0.2mbar | 1600 | 1.52 |
| -5°C, 0.15mbar | 1800 | 1.44 |
| -5°C, 0.25mbar | 1800 | 1.22 |

Secondary drying: The shelf temperature was set according to the thermal stability of the product. The thermal denaturation onset temperature ($T_{onset}$) of the stock solution of the antibody-drug conjugate determined by differential

scanning calorimetry was around 50°C, so the set value of the shelf temperature for secondary drying needed to be below 50°C. The pre-freezing adopted a cooling rate of 0.5°C/min, the primary drying adopted -5°C, and the vacuum value was 0.25 mbar. Table 55 shows the comparison data of the moisture and purity of lyophilized powders obtained by two secondary drying methods. Compared to the stock solution, the purity of the antibody-drug conjugate in the lyophilized powder obtained at both secondary drying temperatures showed no significant difference, but the lyophilized powder from secondary drying at 35°C for 900 min had a lower moisture content. In summary, the secondary drying process was determined to be performed at 35°C under the vacuum value of 0.25 mbar.

Table 55: Comparison data of the moisture and purity of lyophilized powders obtained by two secondary drying methods

| Batch number | Secondary drying conditions | SEC | | | CEX | | | Moisture (%) |
|---|---|---|---|---|---|---|---|---|
| | | Aggregate (%) | Monomer (%) | Fragment (%) | Acidic peak (%) | Main peak (%) | Basic peak (%) | |
| DS113 (Stock solution) | N/A | 0.61 | 99.39 | 0.00 | 27.46 | 66.41 | 6.13 | N/A |
| DP116 (Reconstitution solution of lyophilized powder) | 30°C, 0.25mbar, 1200min | 0.66 | 99.34 | 0.00 | 29.09 | 64.77 | 6.14 | 1.52 |
| DP129 (Reconstitution solution of lyophilized powder) | 35°C, 0.25mbar, 900min | 0.66 | 99.34 | 0.00 | 27.28 | 66.87 | 5.85 | 1.34 |

Lyophilization pilot-scale process transfer:

The lyophilization pilot-scale process transfer determined the primary drying time based on the product temperature curve and the pressure rise test (the criterion for the pressure rise at the end of primary drying was <0.01 mbar/min). The secondary drying conditions were consistent with those in the lab-scale trial. A total of four batches of lyophilization pilot-scale process transfers were conducted. For the first batch of pilot-scale process transfer, the lyophilization formula that was optimized during the lab-scale trial was used. The lyophilized powders located in the middle of the shelf showed severe pits on the sidewall, while this phenomenon was less severe at the edges of the shelf. For the second batch of pilot-scale process transfer, the shelf temperature was set to -20°C during primary drying. However, the sidewall pits of the lyophilized powders located in the middle of the shelf showed no significant improvement. For the third batch of pilot-scale process transfer, an annealing step at -10°C was introduced during pre-freezing to allow the recrystallization of the portions that did not fully crystallize during pre-freezing, resulting in a more uniform crystal form and size distribution and reducing the resistance of moisture escaping from the dried layer; the sidewall pits of the lyophilized powder in the middle of the shelf were slightly improved. For the fourth batch of pilot-scale process transfer, based on the introduction of the annealing step at -10°C during pre-freezing, the vacuum value of primary drying was reduced to 0.06 mbar to enhance the escape ability of water vapor after sublimation, reduce the accumulation of moisture in the dried layer, and avoid the thawing of the dried layer due to accumulated moisture; the pitting phenomenon in the samples located in the middle of the shelf was significantly improved. Table 56 shows the summarized data of the lyophilization pilot-scale process transfer. Table 57 shows the purity comparison data between a reconstitution solution of lyophilized powder from the pilot-scale process transfer and the stock solution before lyophilization. The reconstitution solution of lyophilized powder and the stock solution before lyophilization showed no significant change in the purity.

Table 56: Summarized data of the lyophilization pilot-scale process transfer

| Batch | Pre-freezing cooling rate (°C/min) | Primary drying condition | Secondary drying condition | Moisture (%) | Lyophilized powder morphology |
|---|---|---|---|---|---|
| 1 | 0.5 | -5°C, 0.25mbar | 35°C, 0.25mbar | 1.22 | Severe pits |
| 2 | 0.5 | -20°C, 0.25mbar | | 0.77 | Severe pits |
| 3 | 0.5 (-10°C annealing) | -5°C, 0.25mbar | | 0.90 | Improved pitting phenomenon |
| 4 | 0.5 (-10°C annealing) | -5°C, 0.06mbar | | 0.69 | No significant pitting |

Table 57: Purity comparison data between reconstitution solution of lyophilized powder from the pilot-scale process transfer and stock solution before lyophilization

| Batch | SEC | | | CEX | | | DAR |
|---|---|---|---|---|---|---|---|
| | Aggregate (%) | Monomer (%) | Fragment (%) | Acidic peak (%) | Main peak (%) | Basic peak (%) | |
| Stock solution before lyophilization | 0.65 | 99.35 | 0 | 26.81 | 66.77 | 6.42 | 8 |
| 1 | 0.71 | 99.29 | 0 | 27.51 | 65.84 | 6.65 | 8 |
| 2 | 0.70 | 99.30 | 0 | 29.03 | 64.56 | 6.41 | 8 |
| 3 | 0.67 | 99.33 | 0 | 27.77 | 65.94 | 6.29 | 8 |
| 4 | 0.67 | 99.30 | 0 | 28.18 | 65.73 | 6.09 | 8 |

Detection methods: SEC and CEX were identical to those in Formulation Example 7.

DAR distribution: According to Chinese Pharmacopoeia 2020 edition, Part IV, General Rule 0512 High Performance Liquid Chromatography, analysis and detection were performed using a hydrophobic chromatographic column (e.g., TSKgel Butyl-NPR, 4.6×100mm or other suitable columns) as the chromatographic column. The mobile phase A included 1.0M ammonium sulfate and 25mM phosphate buffer (pH=7.0), and the mobile phase B included 25% isopropanol and 75% 25mM phosphate buffer (pH=7.0). The column temperature was 30°C, the flow rate was 0.5 ml/min, and the detection wavelength was 280 nm. An appropriate amount of the test sample solution was injected into the liquid chromatograph for gradient elution. The DAR value was calculated.

Formulation Example 11

Stability studies at 5±3°C were carried out separately on the stock solution of the antibody-drug conjugate (patritumab-JSSW-001) before lyophilization and the finished product of the antibody-drug conjugate (patritumab-JSSW-001) after lyophilization. The study results showed that the quality of the products in the lyophilized state was more stable than that in the liquid state. The product in the liquid state showed significantly increased high molecular-weight fragments than the product in the lyophilized state and had increased acidic peak contents of charge variants. Moreover, the product the liquid state was more prone to generating free small molecule payloads, with an obviously higher releasing rate compared to the lyophilized powder. The lyophilized dosage form was more conducive to the long-term storage of the product.

Detection methods:

SEC-HPLC: Detection method was identical to that in Formulation Example 6.

NR-CE-SDS: Detection method was identical to that in Formulation Example 5.

CZE: Analysis and detection were performed by using the capillary zone electrophoresis separation mode with reference to capillary electrophoresis (Chinese Pharmacopoeia 2020 edition, Part IV, General Rule 0542). The components in the test sample were separated according to their respective electrophoretic and electroosmotic velocities. After separation, the proportions of the acidic peak, main peak, and basic peak were calculated according to the peak area normalization method.

DAR distribution: Detection method was identical to that in Formulation Example 10.

Free small molecule payload content: According to Chinese Pharmacopoeia 2020 edition, Part IV, General Rule 0512 High Performance Liquid Chromatography and 0431 Mass Spectrometry, analysis and detection were performed using a chromatographic column packed with an octadecylsilyl silica gel (e.g., Waters ACQUITY UPLC® BEH -C18, 2.1×50mm, particle size 1.7μm or other suitable chromatographic columns). The mobile phase A was an aqueous solution containing 0.1% FA, and the mobile phase B was an acetonitrile solution containing 0.1% FA. The column temperature was 40°C, and the flow rate was 0.4 ml/min. 50μL of the test sample (10 mg/mL) was taken, and an internal standard working solution was added. The mixture was precipitated with acetonitrile in the ratio of 1:3, placed in a -20°C ice bath for 30 minutes, and then centrifuged. The supernatant was taken and loaded onto the column. Different concentrations of small molecule payloads were used as reference standards and loaded onto the column. After gradient elution by liquid chromatography, the sample was sent to the mass spectrometer detector. After setting parameters such as capillary and cone voltages, ion source temperature, collision energy, and nebulizer flow rate, the analysis was performed. The free small molecule payload content in the test sample was calculated according to the standard curve.

Free small molecule payloads content (%) = Detection concentration of free small molecule payloads/ADC concentration × 100%

Table 58: Stability results for key quality indicators of GMP Batch 1

| Items | GMP Batch 1 | | | |
|---|---|---|---|---|
| | Stock solution (5±3°C) | | Lyophilized powder (5±3°C) | |
| | 0 months | 6 months | 0 months | 6 months |
| SEC-HPLC | HMWS: 0.4% | 1.1% | HMWS: 0.4% | 0.4% |
| | Monomer: 99.6% | 98.9% | Monomer: 99.6% | 99.6% |
| NR-CE-SDS | LC+HC: 96.5% | 95.7% | HC+LC: 96.4% | 96.4% |
| | Other peak: 3.4% | 4.3% | Other peak: 3.6% | 3.6% |
| CZE | Acidic peak: 19.2% | 25.1% | Acidic peak: 19.7% | 20.8% |
| | Main peak: 75.1% | 68.4% | Main peak: 74.8% | 73.4% |
| | Basic peak: 5.7% | 6.5% | Basic peak: 5.5% | 5.9% |
| DAR | DAR value: 8.0 | 8.0 | DAR value: 8.0 | 8.0 |
| Free small molecule payloads | 0.000038% | 0.00076% | 0.00012% | 0.00023% |
| Antigen binding activity | 96% | 94% | 93% | 99% |
| Bioactivity | 103% | 105% | 107% | 106% |

Table 59: Stability results for key quality indicators of GMP Batch 2

| Items | GMP Batch 2 | | | |
|---|---|---|---|---|
| | Stock solution (5±3°C) | | Lyophilized powder (5±3°C) | |
| | 0 months | 6 months | 0 months | 6 months |
| SEC-HPLC | HMWS: 0.5% | 1.0% | HMWS: 0.6% | 0.4% |
| | Monomer: 99.5% | 99.0% | Monomer: 99.4% | 99.6% |
| NR-CE-SDS | LC+HC: 95.9% | 95.1% | HC+LC: 95.6% | 96.3% |
| | Other peak: 4.1% | 5.0% | Other peak: 4.4% | 3.7% |
| CZE | Acidic peak: 18.6% | 25.8% | Acidic peak: 18.1% | 18.9% |
| | Main peak: 75.0% | 67.4% | Main peak: 75.4% | 74.6% |
| | Basic peak: 6.4% | 6.7% | Basic peak: 6.4% | 6.5% |
| DAR | DAR value: 8.0 | 8.0 | DAR value: 8.0 | 8.0 |
| Free small molecule payloads | 0.000031% | 0.00067% | 0.00018% | 0.00025% |

(continued)

| Items | GMP Batch 2 | | | |
|---|---|---|---|---|
| | Stock solution (5±3°C) | | Lyophilized powder (5±3°C) | |
| | 0 months | 6 months | 0 months | 6 months |
| Antigen binding activity | 100% | 95% | 105% | 92% |
| Bioactivity | 102% | 104% | 105% | 106% |

Formulation Example 12.1: Preparation Example of Formulation

(1) Buffer exchanging through ultrafiltration: A solution of 25 mM histidine-histidine hydrochloride and 9% trehalose at pH=5.0 was prepared. The buffer exchanging was performed by using a 30 kD ultrafiltration membrane pack. The antibody-drug conjugate was exchanged into the solution of 25 mM histidine-histidine hydrochloride and 9% trehalose at pH=5.0. The exchanging factor for the buffer exchanging was no less than 100-fold. The concentration of the ADC in the exchanging solution was controlled to be no less than 1.2 times the target concentration.
(2) Preparation of semi-finished product: According to the amounts in the formula, the corresponding amount of polysorbate 80 was added to the ultrafiltration exchanging solution. The ADC was diluted to the target concentration with a solution containing 25 mM histidine-histidine hydrochloride and 9% trehalose at pH=5.0. After stirring and mixing uniformly, a target solution containing 10 mg/ml ADC, 25 mM histidine-histidine hydrochloride, 9% trehalose, and 0.03% polysorbate 80 was obtained. After filtering with a two-stage 0.22 μm sterilization filter, an aqueous solution of the SWY2110-JSSW001 formulation was obtained, which contained about 10 mg/ml of the antibody-drug conjugate SWY2110-JSSW-001, about 0.33 mg/ml of histidine, about 4.79 mg/ml of histidine hydrochloride, about 0.3 mg/ml of polysorbate 80, about 90 mg/ml of trehalose, and the balance of water.
(3) Filling and lyophilization: According to the filling volume of the product, the filling of sample products and the semi-stoppering were performed on a fully automatic filling line. The semi-stoppered products were transferred via an automatic conveyor into a lyophilizer for freeze-drying. The conditions were controlled as follows: the pre-freezing was performed at -45°C for not less than 180 minutes; the primary drying was performed at -20°C and 0.20 mbar for not less than 4000 minutes; the secondary drying was performed at 30°C and 0.20 mbar for not less than 1200 minutes. After the lyophilization was completed, the stoppering was performed in the lyophilizer, then the product was transferred to the capping station via an automatic conveyor to complete the capping. After passing the lamp inspection, the final product, SWY2110-JSSW001 formulation, was obtained, which contained about 10 parts by weight of antibody-drug conjugate SWY2110-JSSW-001, about 0.33 parts by weight of histidine, about 4.79 parts by weight of histidine hydrochloride, about 0.3 parts by weight of polysorbate 80, and about 90 parts by weight of trehalose.

Formulation Example 12.2: Preparation Example of Formulation

(1) Buffer exchanging through ultrafiltration: A solution of 15 mM histidine-histidine hydrochloride and 8% sucrose at pH=5.0 was prepared. The buffer exchanging was performed by using a 30 kD ultrafiltration membrane pack. The antibody-drug conjugate was exchanged into the solution of 15 mM histidine-histidine hydrochloride and 8% sucrose at pH=5.0. The exchanging factor for the buffer exchanging was no less than 100-fold. The concentration of the ADC in the exchanging solution was controlled to be no less than 1.2 times the target concentration.
(2) Preparation of semi-finished product: According to the amounts in the formula, the corresponding amount of polysorbate 80 was added to the ultrafiltration exchanging solution. The ADC was diluted to the target concentration with a solution containing 15 mM histidine-histidine hydrochloride and 8% sucrose at pH=5.0. After stirring and mixing uniformly, a target solution containing 20 mg/ml ADC, 15 mM histidine-histidine hydrochloride, 8% sucrose, and 0.02% polysorbate 80 was obtained. After filtering with a two-stage 0.22 μm sterilization filter, an aqueous solution of the Patritumab-JSSW001 formulation was obtained, which contained about 20 mg/ml of patritumab-JSSW-001 antibody-drug conjugate, about 0.21 mg/ml of histidine, about 2.86 mg/ml of histidine hydrochloride, about 0.2 mg/ml of polysorbate 80, about 80 mg/ml of sucrose, and the balance of water.
(3) Filling and lyophilization: According to the filling volume of the product, the filling of sample products and the semi-stoppering were performed on a fully automatic filling line. The semi-stoppered products were transferred via an automatic conveyor into a lyophilizer for freeze-drying. The conditions were controlled as follows: the pre-freezing was

performed at -40°C for not less than 180 minutes; the primary drying was performed at -5°C and 0.06 mbar for not less than 3000 minutes; the secondary drying was performed at 35°C and 0.25 mbar for not less than 900 minutes. After the lyophilization was completed, the stoppering was performed in the lyophilizer, then the product was transferred to the capping station via an automatic conveyor to complete the capping. After passing the lamp inspection, the final product, patritumab-JSSW001 formulation, was obtained, which contained about 20 parts by weight of antibody-drug conjugate patritumab-JSSW-001, about 0.21 parts by weight of histidine, about 2.86 parts by weight of histidine hydrochloride, about 0.2 parts by weight of polysorbate 80, and about 80 parts by weight of sucrose.

Formulation Example 13: Bioactivity Assay

An aqueous solution of the SWY2110-JSSW001 formulation and an aqueous solution of the Patritumab-JSSW001 formulation, respectively obtained in step (2) of Formulation Examples 12.1 and 12.2, were used to determine the biological activities of two formulations according to the method for determining biological activity described in the preceding Biological Examples section. The results indicated that the biological activities of the two formulations were comparable to those of the standalone ADCs.

## Claims

**1.** A pharmaceutical composition containing an antibody-drug conjugate represented by formula I:

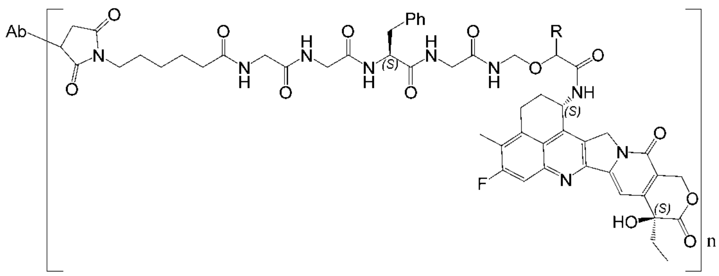

Formula I

wherein Ab is an antibody targeting HER2, HER3, or EGFR or an antigen-binding fragment thereof, R is selected from $C_{1-6}$ alkyl, and n is an integer of 1-8 or a decimal of 1-8, preferably, n is 8;
the pharmaceutical composition is an injection, preferably an injection solution or a lyophilized injection;
preferably, the antibody-drug conjugate represented by formula I has a structure represented by formula Ia or formula Ib:

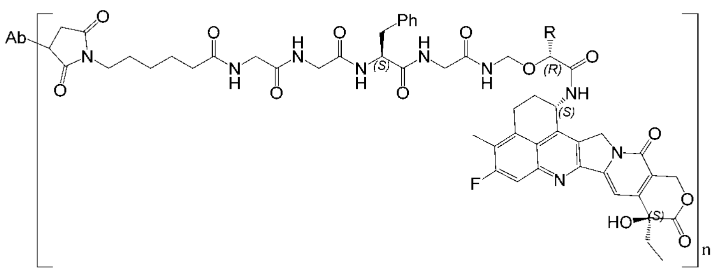

Formula Ia

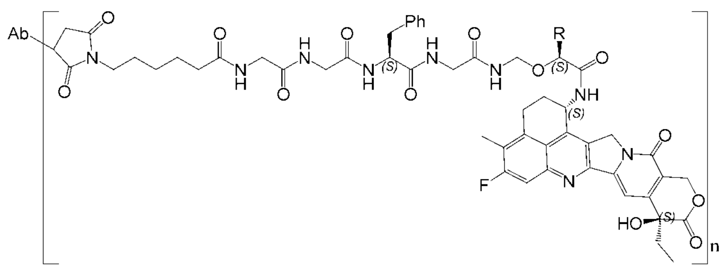

Formula Ib;

more preferably, the antibody-drug conjugate represented by formula I has a structure represented by formula I-1,

formula Ia-1, or formula Ib-1:

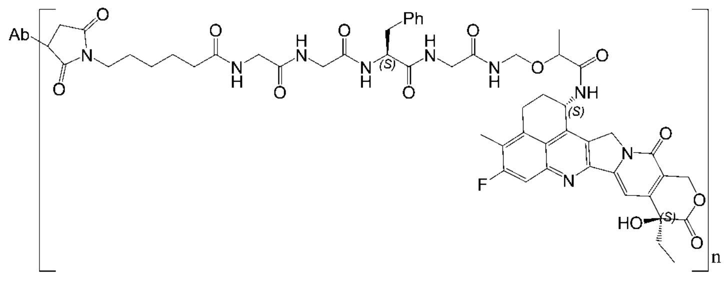

Formula I-1

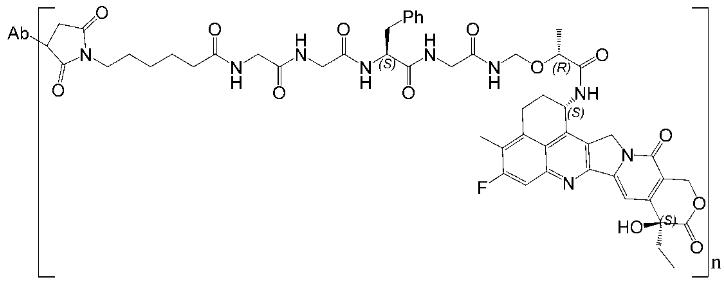

Formula Ia-1

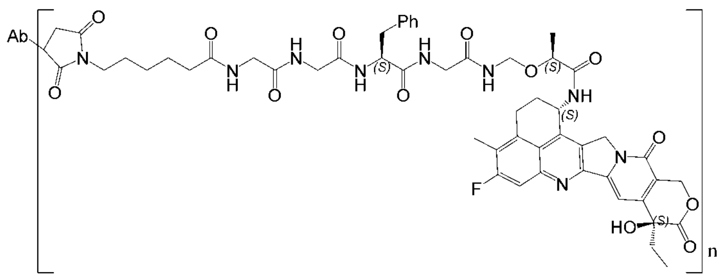

Formula Ib-1.

**2.** The pharmaceutical composition according to claim 1, wherein

in case that the Ab is an antibody targeting HER2 or an antigen-binding fragment thereof, it comprises a variable region of a heavy chain (HV) and a variable region of a light chain (LV), wherein, a sequence of the variable region of the heavy chain comprises a heavy chain complementarity determining region 1 (HCDR1), a heavy chain complementarity determining region 2 (HCDR2) and a heavy chain complementarity determining region 3 (HCDR3), a sequence of the variable region of the light chain comprises a light chain complementarity determining region 1 (LCDR1), a light chain complementarity determining region 2 (LCDR2) and a light chain complementarity determining region 3 (LCDR3), wherein the amino acid sequence of HCDR1 is represented by SEQ ID NO:1, the amino acid sequence of HCDR2 is represented by SEQ ID NO:2, the amino acid sequence of HCDR3 is represented by SEQ ID NO:3, and/or the amino acid sequence of LCDR1 is represented by SEQ ID NO:4, the amino acid sequence of LCDR2 is represented by SEQ ID NO:5, the amino acid sequence of LCDR3 is represented by SEQ ID NO:6;
further preferably, in case that the Ab is an antibody targeting HER2 or an antigen-binding fragment thereof, it comprises a variable region of a heavy chain (HV) and a variable region of a light chain (LV), wherein the amino acid sequence of HV is represented by SEQ ID NO:7, the amino acid sequence of LV is represented by SEQ ID NO:8;
more preferably, in case that the Ab is an antibody targeting HER2 or an antigen-binding fragment thereof, it comprises a heavy chain (HC) and a light chain (LC), wherein the amino acid sequence of HC is represented by SEQ ID NO:9, the amino acid sequence of LC is represented by SEQ ID NO:10; in case that the Ab is an antibody targeting HER3 or an antigen-binding fragment thereof, it comprises a variable region of a heavy chain (HV) and a variable region of a light chain (LV), wherein, a sequence of the variable region of the heavy chain comprises a heavy chain complementarity determining region 1 (HCDR1), a heavy chain complementarity determining region 2 (HCDR2) and a heavy chain complementarity determining region 3 (HCDR3), a sequence of the variable region of the light chain comprises a light chain complementarity determining region 1 (LCDR1), a light chain

complementarity determining region 2 (LCDR2) and a light chain complementarity determining region 3 (LCDR3), wherein the amino acid sequence of HCDR1 is represented by SEQ ID NO:11, the amino acid sequence of HCDR2 is represented by SEQ ID NO:12, the amino acid sequence of HCDR3 is represented by SEQ ID NO:13, and/or the amino acid sequence of LCDR1 is represented by SEQ ID NO:14, the amino acid sequence of LCDR2 is represented by SEQ ID NO:15, the amino acid sequence of LCDR3 is represented by SEQ ID NO:16;

further preferably, in case that the Ab is an antibody targeting HER3 or an antigen-binding fragment thereof, it comprises a variable region of a heavy chain (HV) and a variable region of a light chain (LV), wherein the amino acid sequence of HV is represented by SEQ ID NO:17, the amino acid sequence of LV is represented by SEQ ID NO:18;

more preferably, in case that the Ab is an antibody targeting HER3 or an antigen-binding fragment thereof, it comprises a heavy chain (HC) and a light chain (LC), wherein the amino acid sequence of HC is represented by SEQ ID NO:19, the amino acid sequence of LC is represented by SEQ ID NO:20; in case that the Ab is an antibody targeting EGFR or an antigen-binding fragment thereof, it comprises a variable region of a heavy chain (HV) and a variable region of a light chain (LV), wherein, a sequence of the variable region of the heavy chain comprises a heavy chain complementarity determining region 1 (HCDR1), a heavy chain complementarity determining region 2 (HCDR2) and a heavy chain complementarity determining region 3 (HCDR3), a sequence of the variable region of the light chain comprises a light chain complementarity determining region 1 (LCDR1), a light chain complementarity determining region 2 (LCDR2) and a light chain complementarity determining region 3 (LCDR3), wherein the amino acid sequence of HCDR1 is represented by SEQ ID NO:21, the amino acid sequence of HCDR2 is represented by SEQ ID NO:22, the amino acid sequence of HCDR3 is represented by SEQ ID NO:23, and/or the amino acid sequence of LCDR1 is represented by SEQ ID NO:24, the amino acid sequence of LCDR2 is represented by SEQ ID NO:25, the amino acid sequence of LCDR3 is represented by SEQ ID NO:26;

in case that the Ab is an antibody targeting EGFR or an antigen-binding fragment thereof, it comprises a variable region of a heavy chain (HV) and a variable region of a light chain (LV), wherein the amino acid sequence of HCDR1 is represented by SEQ ID NO:31 or SEQ ID NO:35 or SEQ ID NO:38, the amino acid sequence of HCDR2 is represented by SEQ ID NO:22, the amino acid sequence of HCDR3 is represented by SEQ ID NO:23 or SEQ ID NO:32, and/or the amino acid sequence of LCDR1 is represented by SEQ ID NO:24, the amino acid sequence of LCDR2 is represented by SEQ ID NO:25, the amino acid sequence of LCDR3 is represented by SEQ ID NO:26;

in case that the Ab is an antibody targeting EGFR or an antigen-binding fragment thereof, it comprises a variable region of a heavy chain (HV) and a variable region of a light chain (LV), wherein the amino acid sequence of HCDR1 is represented by SEQ ID NO:31 or SEQ ID NO:35 or SEQ ID NO:38, the amino acid sequence of HCDR2 is represented by SEQ ID NO:22, the amino acid sequence of HCDR3 is represented by SEQ ID NO:32, and the amino acid sequence of LCDR1 is represented by SEQ ID NO:24, the amino acid sequence of LCDR2 is represented by SEQ ID NO:25, the amino acid sequence of LCDR3 is represented by SEQ ID NO:26;

further preferably, in case that the Ab is an antibody targeting EGFR or an antigen-binding fragment thereof, it comprises a variable region of a heavy chain (HV) and a variable region of a light chain (LV), wherein the amino acid sequence of HV is represented by SEQ ID NO:27, the amino acid sequence of LV is represented by SEQ ID NO:28;

further preferably, in case that the Ab is an antibody targeting EGFR or an antigen-binding fragment thereof, it comprises a variable region of a heavy chain (HV) and a variable region of a light chain (LV), wherein the amino acid sequence of HV is represented by SEQ ID NO:33 or SEQ ID NO:36 or SEQ ID NO:39, the amino acid sequence of LV is represented by SEQ ID NO:28;

more preferably, in case that the Ab is an antibody targeting EGFR or an antigen-binding fragment thereof, it comprises a heavy chain (HC) and a light chain (LC), wherein the amino acid sequence of HC is represented by SEQ ID NO:29, the amino acid sequence of LC is represented by SEQ ID NO:30; more preferably, in case that the Ab is an antibody targeting EGFR or an antigen-binding fragment thereof, it comprises a heavy chain (HC) and a light chain (LC), wherein the amino acid sequence of HC is represented by SEQ ID NO:34 or SEQ ID NO:37 or SEQ ID NO:40, the amino acid sequence of LC is represented by SEQ ID NO:30.

**3.** The pharmaceutical composition according to any one of claims 1-2, wherein the antibody-drug conjugate represented by formula I has the following structure:

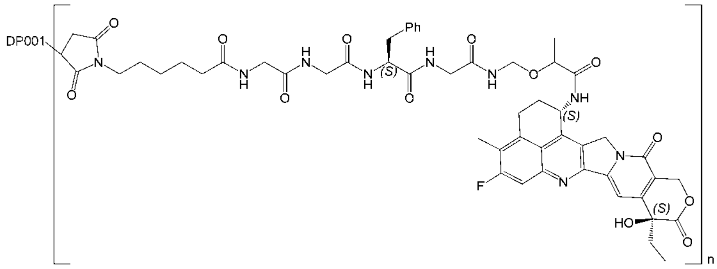

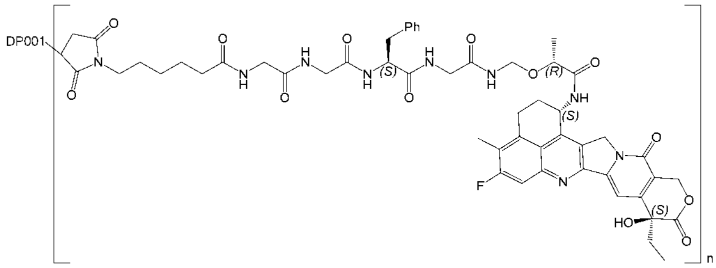

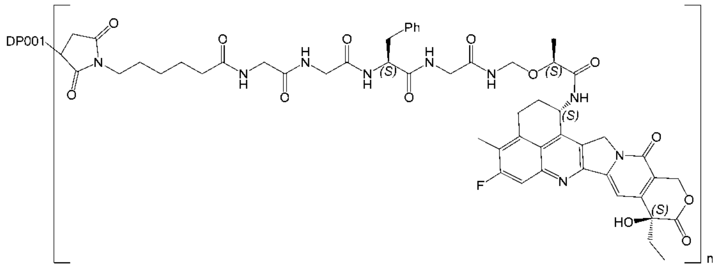

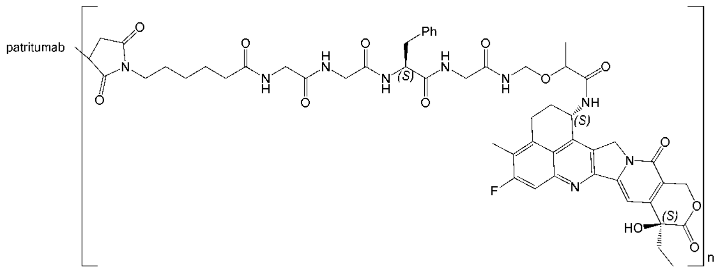

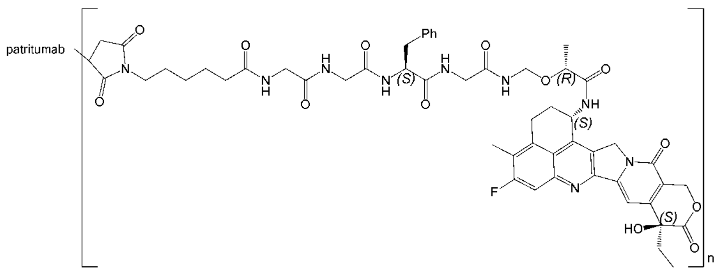

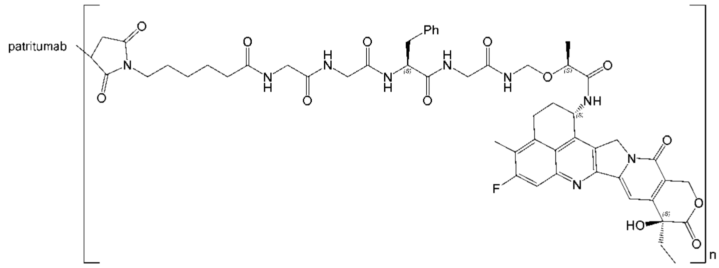

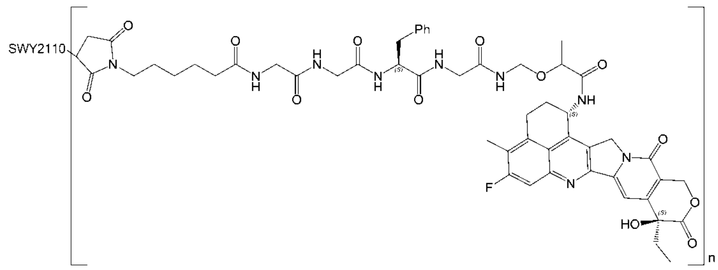

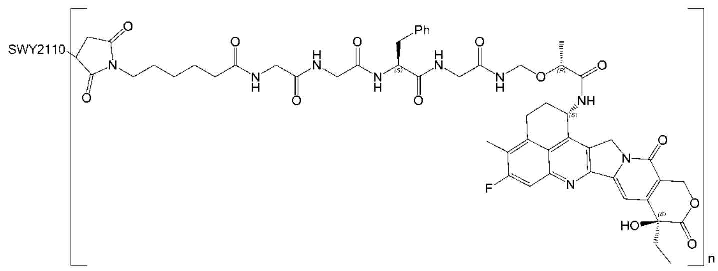

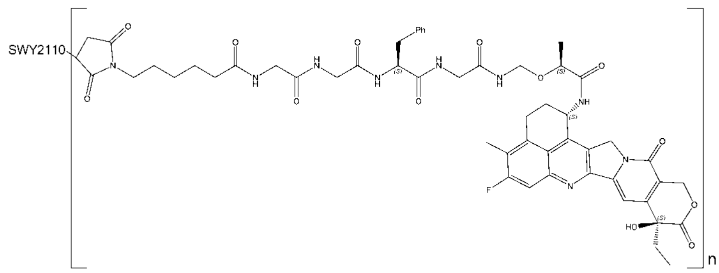

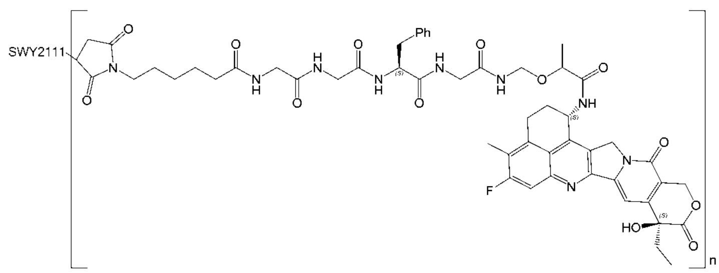

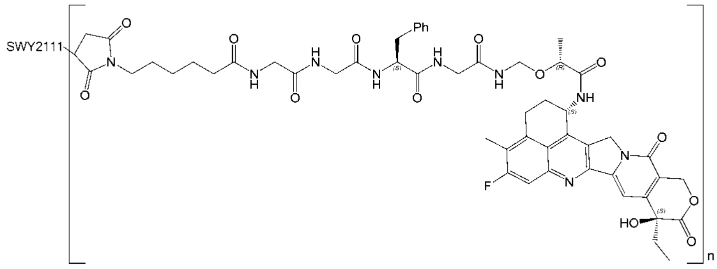

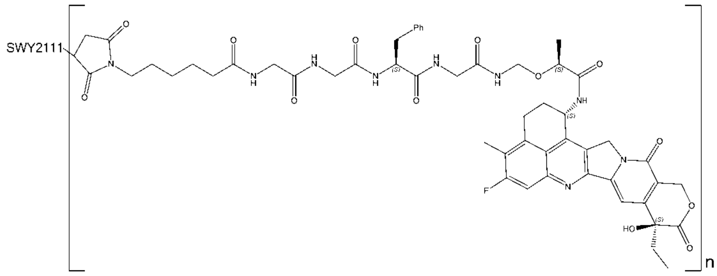

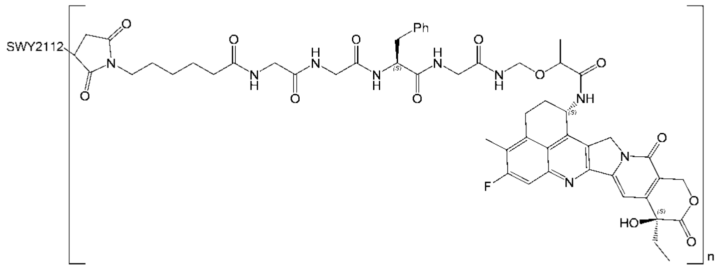

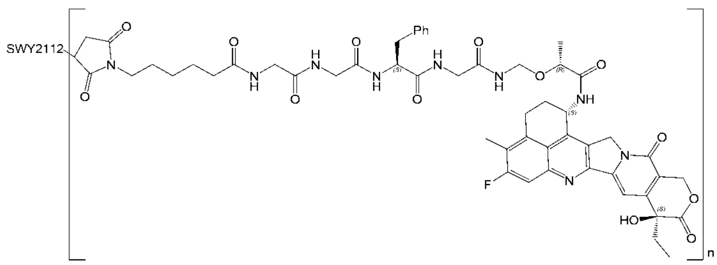

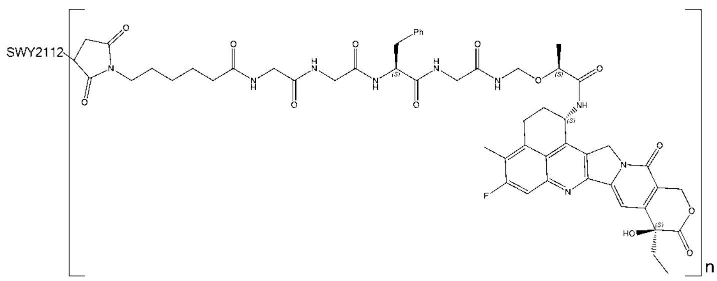

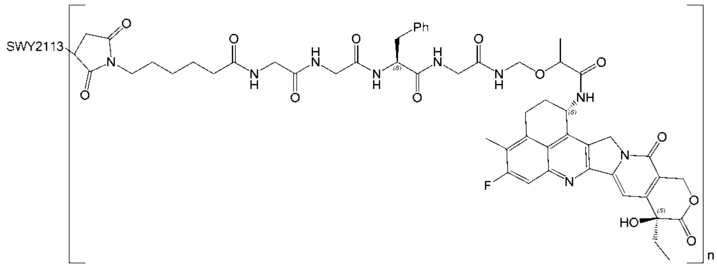

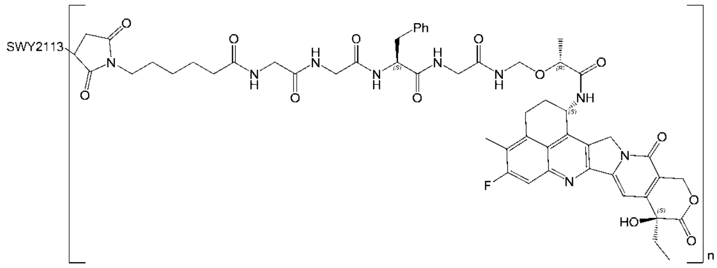

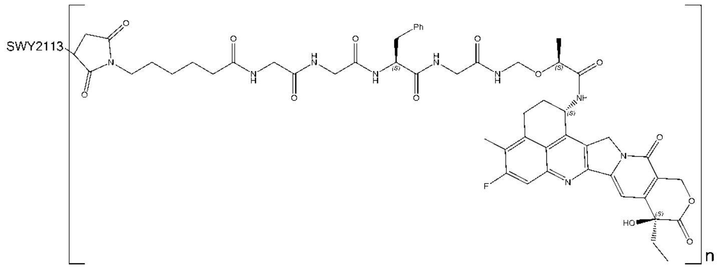

wherein:

DP001 is an antibody having two heavy chains with the amino acid sequence represented by SEQ ID NO:9 and two light chains with the amino acid sequence represented by SEQ ID NO:10;
Patritumab is an antibody having two heavy chains with the amino acid sequence represented by SEQ ID NO:19 and two light chains with the amino acid sequence represented by SEQ ID NO:20;
SWY2110 is an antibody having two heavy chains with the amino acid sequence represented by SEQ ID NO:29 and two light chains with the amino acid sequence represented by SEQ ID NO:30;
SWY2111 is an antibody having two heavy chains with the amino acid sequence represented by SEQ ID NO:34 and two light chains with the amino acid sequence represented by SEQ ID NO:30;
SWY2112 is an antibody having two heavy chains with the amino acid sequence represented by SEQ ID NO:37 and two light chains with the amino acid sequence represented by SEQ ID NO:30;
SWY2113 is an antibody having two heavy chains with the amino acid sequence represented by SEQ ID NO:40 and two light chains with the amino acid sequence represented by SEQ ID NO:30;
n is an integer of 1-8 or a decimal of 1-8, preferably n is 8.

**4.** The pharmaceutical composition according to any one of claims 1-3, wherein the weight percentage content of the antibody-drug conjugate represented by formula I is 1-30% (e.g. about 2%, about 3%, about 4%, about 5%, about 6%, about 7%, about 8%, about 8.5%, about 9%, about 9.5%, about 10%, about 10.5%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%), preferably 5-25%, 8-20%, 8-15%, 8-10%, 15-25%, based on 100 wt% of the pharmaceutical composition; or
wherein the content of the antibody-drug conjugate represented by formula I is 1-30 mg/ml (e.g. about 1 mg/ml, about 2 mg/ml, about 3 mg/ml, about 4 mg/ml, about 5 mg/ml, about 6 mg/ml, about 7 mg/ml, about 8 mg/ml, about 8.5 mg/ml, about 9 mg/ml, about 9.5 mg/ml, about 10 mg/ml, about 10.5 mg/ml, about 11 mg/ml, about 12 mg/ml, about 13 mg/ml, about 14 mg/ml, about 15 mg/ml, about 16 mg/ml, about 17 mg/ml, about 18 mg/ml, about 19 mg/ml, about 20 mg/ml, about 21 mg/ml, about 22 mg/ml, about 23 mg/ml, about 24 mg/ml, about 25 mg/ml, about 26 mg/ml, about 27 mg/ml, about 28 mg/ml, about 29 mg/ml, about 30 mg/ml), preferably 5-25 mg/ml, further preferably 5-20 mg/ml, 5-15 mg/ml,

15-25 mg/ml.

**5.** The pharmaceutical composition according to any one of claims 1-4, which further contains a buffering agent;

preferably the buffering agent is selected from acetate, succinate, gluconate, histidine salt, oxalate, lactate, phosphate, citrate, tartrate, fumarate, glycylglycine, tris(hydroxymethyl)aminomethane (Tris), 2-(N-morpholino) ethanesulfonic acid (MES), and other organic acid buffering agents, preferably histidine salt, further preferably histidine-histidine hydrochloride;
more preferably, the weight percentage content of the buffering agent is 1-10% (e.g. about 1%, about 2%, about 2.8%, about 2.9%, about 3%, about 3.1%, about 3.2%, about 4%, about 4.5%, about 4.6%, about 4.7%, about 4.8%, about 4.9%, about 5%, about 5.1%, about 5.2%, about 5.3%, about 5.4%, about 5.5%, about 6%, about 6.5%, about 7%, about 8%, about 9%, about 9.9%, about 10%), preferably 1-9.9%, 2-6%, 2-5%, 4-6%, based on 100 wt% of the pharmaceutical composition; or the content of the buffering agent is 1-10 mg/ml (e.g. about 1 mg/ml, about 2 mg/ml, about 3 mg/ml, about 4 mg/ml, about 4.5 mg/ml, about 4.6 mg/ml, about 4.7 mg/ml, about 4.8 mg/ml, about 4.9 mg/ml, about 5 mg/ml, about 5.1 mg/ml, about 5.2 mg/ml, about 5.3 mg/ml, about 5.4 mg/ml, about 5.5 mg/ml, about 6 mg/ml, about 6.5 mg/ml, about 7 mg/ml, about 8 mg/ml, about 9 mg/ml, about 10 mg/ml), preferably 2-6 mg/ml, 2-4 mg/ml, 3-6 mg/ml, 4-6mg/ml;
most preferably, the buffering agent is a histidine-histidine hydrochloride buffering agent, wherein the weight percentage content of histidine is 0.1-1% (e.g. about 0.1%, about 0.2%, about 0.25%, about 0.26%, about 0.27%, about 0.28%, about 0.29%, about 0.3%, about 0.31%, about 0.32%, about 0.33%, about 0.34%, about 0.35%, about 0.4%, about 0.45%, about 0.5%, about 0.55%, about 0.6%, about 0.7%, about 0.8%, about 0.9%, about 1%), preferably 0.1-0.5%, 0.2-0.4%, based on 100 wt% of the pharmaceutical composition; or wherein the content of histidine is 0.1-1mg/ml (e.g. about 0.1 mg/ml, about 0.2 mg/ml, about 0.21 mg/ml, about 0.22 mg/ml, about 0.23 mg/ml, about 0.24 mg/ml, about 0.25 mg/ml, about 0.26 mg/ml, about 0.27 mg/ml, about 0.28 mg/ml, about 0.29 mg/ml, about 0.3 mg/ml, about 0.31 mg/ml, about 0.32 mg/ml, about 0.33 mg/ml, about 0.34 mg/ml, about 0.35 mg/ml, about 0.4 mg/ml, about 0.45 mg/ml, about 0.5 mg/ml, about 0.55 mg/ml, about 0.6 mg/ml, about 0.7 mg/ml, about 0.8 mg/ml, about 0.9 mg/ml, about 1mg/ml), preferably 0.1-0.5 mg/ml, 0.1-0.3 mg/ml, 0.2-0.4mg/ml;
wherein the weight percentage content of histidine hydrochloride is 1-10% (e.g. about 1%, about 2%, about 2.5%, about 2.6%, about 2.7%, about 2.8%, about 2.9%, about 3%, about 3.5%, about 4%, about 4.5%, about 4.6%, about 4.7%, about 4.8%, about 4.9%, about 5%, about 5.1%, about 5.2%, about 5.3%, about 5.4%, about 5.5%, about 6%, about 6.5%, about 7%, about 8%, about 9%, about 9.9%, about 10%), preferably 1-9.9%, 2-6%, 2-4%, 3-6%, 3.5-5.5%, 4-5%, based on 100 wt% of the pharmaceutical composition; or wherein the content of histidine hydrochloride is 1-10 mg/ml (e.g. about 1 mg/ml, about 2 mg/ml, about 2.5 mg/ml, about 2.6 mg/ml, about 2.7 mg/ml, about 2.8 mg/ml, about 2.9 mg/ml, about 3 mg/ml, about 3.5 mg/ml, about 4 mg/ml, about 4.5 mg/ml, about 4.6 mg/ml, about 4.7 mg/ml, about 4.8 mg/ml, about 4.9 mg/ml, about 5 mg/ml, about 5.1 mg/ml, about 5.2 mg/ml, about 5.3 mg/ml, about 5.4 mg/ml, about 5.5 mg/ml, about 6 mg/ml, about 6.5 mg/ml, about 7 mg/ml, about 8 mg/ml, about 9 mg/ml, about 10 mg/ml), preferably 2-6 mg/ml, 2-4 mg/ml, 3-6mg/ml, 4-5.5mg/ml, 4.5-5 mg/ml.

**6.** The pharmaceutical composition according to any one of claims 1-5, which further contains a stabilizer;

preferably the stabilizer is selected from saccharides (such as sucrose and trehalose), polyols (such as mannitol and sorbitol), amino acids (such as L-serine, sodium glutamate, alanine, glycine, and sarcosine), preferably saccharides, further preferably sucrose and/or trehalose;
more preferably, the weight percentage content of the stabilizer is 50-90% (e.g. about 50%, about 60%, about 70%, about 75%, about 80%, about 81%, about 82%, about 83%, about 84%, about 85%, about 86%, about 87%, about 88%, about 89%, about 90%), preferably 60-90%, 70-90%, 80-90%, based on 100 wt% of the pharmaceutical composition; or the content of the stabilizer is 50-110 mg/ml (e.g. about 50 mg/ml, about 60 mg/ml, about 70 mg/ml, about 75 mg/ml, about 80 mg/ml, about 81 mg/ml, about 82 mg/ml, about 83 mg/ml, about 84 mg/ml, about 85 mg/ml, about 86 mg/ml, about 87 mg/ml, about 88 mg/ml, about 89 mg/ml, about 90 mg/ml, about 91 mg/ml, about 92 mg/ml, about 93 mg/ml, about 94 mg/ml, about 95 mg/ml, about 100 mg/ml, about 105 mg/ml, about 110 mg/ml), preferably 70-100 mg/ml, 80-100 mg/ml, 75-95 mg/ml.

**7.** The pharmaceutical composition according to any one of claims 1-6, which further contains a surfactant;

preferably, the surfactant is selected from polysorbates, preferably polysorbate 20 and/or polysorbate 80;
more preferably, the weight percentage content of the surfactant is 0.1-1% (e.g. about 0.1%, about 0.15%, about 0.16%, about 0.17%, about 0.18%, about 0.19%, about 0.2%, about 0.25%, about 0.26%, about 0.27%, about

0.28%, about 0.29%, about 0.3%, about 0.31%, about 0.32%, about 0.33%, about 0.34%, about 0.35%, about 0.4%, about 0.45%, about 0.5%, about 0.55%, about 0.6%, about 0.7%, about 0.8%, about 0.9%, about 1%), preferably 0.2-0.4%, further preferably 0.15-0.4%, 0.15-0.35%, 0.25-0.35%, based on 100 wt% of the pharmaceutical composition; or the content of the surfactant is 0.1-1mg/ml (e.g. about 0.1 mg/ml, about 0.15 mg/ml, about 0.16 mg/ml, about 0.17 mg/ml, about 0.18 mg/ml, about 0.19 mg/ml, about 0.2 mg/ml, about 0.25 mg/ml, about 0.3 mg/ml, about 0.35 mg/ml, about 0.4 mg/ml, about 0.45 mg/ml, about 0.5 mg/ml, about 0.55 mg/ml, about 0.6 mg/ml, about 0.7 mg/ml, about 0.8 mg/ml, about 0.9 mg/ml, about 1mg/ml), preferably 0.1-0.4 mg/ml, 0.15-0.25 mg/ml, 0.2-0.4 mg/ml, 0.25-0.35 mg/ml.

**8.** The pharmaceutical composition according to any one of claims 1-7, wherein the pharmaceutical composition has a pH of 4.0-7.5 (e.g. about 4.0, about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.5, about 7.0), preferably 4.5-7.0, further preferably 4.5-5.5, further preferably 5.0-5.5, most preferably about 5.0.

**9.** The pharmaceutical composition according to any one of claims 1-8, wherein

the pharmaceutical composition is a lyophilized injection, and when the lyophilized injection is diluted with water for injection, the diluted pharmaceutical composition contains about 10 mg/ml of the antibody-drug conjugate represented by formula I, about 0.33 mg/ml of histidine, about 4.79 mg/ml of histidine hydrochloride, about 0.3 mg/ml of polysorbate 80, and about 90 mg/ml of trehalose, based on the total volume of the diluted pharmaceutical composition, and the pH of the diluted pharmaceutical composition is about 5.0, preferably, the antibody-drug conjugate represented by formula I is selected from SWY2110-JSSW-001, SWY2111-JSSW-001, and SWY2113-JSSW-001, preferably SWY2110-JSSW-001; or
the pharmaceutical composition is an injection solution, and the injection solution contains about 10 mg/ml of the antibody-drug conjugate represented by formula I, about 0.33 mg/ml of histidine, about 4.79 mg/ml of histidine hydrochloride, about 0.3 mg/ml of polysorbate 80, and about 90 mg/ml of trehalose, and the pH of the injection solution is about 5.0, preferably, the antibody-drug conjugate represented by formula I is selected from SWY2110-JSSW-001, SWY2111-JSSW-001, and SWY2113-JSSW-001, preferably SWY2110-JSSW-001; or
the pharmaceutical composition contains about 10 mg/ml of the antibody-drug conjugate represented by formula I, about 0.33 mg/ml of histidine, about 4.79 mg/ml of histidine hydrochloride, about 0.3 mg/ml of polysorbate 80, about 90 mg/ml trehalose, and the balance of water, and the pH of the pharmaceutical composition is about 5.0, preferably, the antibody-drug conjugate represented by formula I is selected from SWY2110-JSSW-001, SWY2111-JSSW-001, and SWY2113-JSSW-001, preferably SWY2110-JSSW-001; or
the pharmaceutical composition contains or consists of about 10 parts by weight of the antibody-drug conjugate represented by formula I, about 0.33 parts by weight of histidine, about 4.79 parts by weight of histidine hydrochloride, about 0.3 parts by weight of polysorbate 80, and about 90 parts by weight of trehalose, preferably, the antibody-drug conjugate represented by formula I is selected from SWY2110-JSSW-001, SWY2111-JSSW-001, and SWY2113-JSSW-001, preferably SWY2110-JSSW-001; or
the pharmaceutical composition is a lyophilized injection, and when the lyophilized injection is diluted with water for injection, the diluted pharmaceutical composition contains about 20 mg/ml of the antibody-drug conjugate represented by formula I, about 0.21 mg/ml of histidine, about 2.86 mg/ml of histidine hydrochloride, about 0.2 mg/ml of polysorbate 80, and about 80 mg/ml of sucrose, based on the total volume of the diluted pharmaceutical composition, and the pH of the diluted pharmaceutical composition is about 5.0, preferably, the antibody-drug conjugate represented by formula I is patritumab-JSSW-001; or
the pharmaceutical composition is an injection solution, and the injection solution contains about 20 mg/ml of the antibody-drug conjugate represented by formula I, about 0.21 mg/ml of histidine, about 2.86 mg/ml of histidine hydrochloride, about 0.2 mg/ml of polysorbate 80, and about 80 mg/ml of sucrose, and the pH of the injection solution is about 5.0, preferably, the antibody-drug conjugate represented by formula I is patritumab-JSSW-001; or
the pharmaceutical composition contains about 20 mg/ml of the antibody-drug conjugate represented by formula I, about 0.21 mg/ml of histidine, about 2.86 mg/ml of histidine hydrochloride, about 0.2 mg/ml of polysorbate 80, about 80 mg/ml of sucrose, and the balance of water, and the pH of the pharmaceutical composition is about 5.0, preferably, the antibody-drug conjugate represented by formula I is patritumab-JSSW-001; or
the pharmaceutical composition contains or consists of about 20 parts by weight of the antibody-drug conjugate represented by formula I, about 0.21 parts by weight of histidine, about 2.86 parts by weight of histidine hydrochloride, about 0.2 parts by weight of polysorbate 80, and about 80 parts by weight of sucrose, preferably, the antibody-drug conjugate represented by formula I is patritumab-JSSW-001.

**10.** A method for preparing the pharmaceutical composition according to any one of claims 1-9, comprising the following steps:

S1) preparing the antibody-drug conjugate represented by formula I;
S2) preparing an ultrafiltration-exchanging buffer;
S3) exchanging the antibody-drug conjugate obtained in S1 into the buffer obtained in S2;

preferably, step S2) comprises weighing a buffering agent and a stabilizer according to the amounts in a formula, adding water for injection to dilute to the target formulation volume, stirring and mixing uniformly to obtain the ultrafiltration-exchanging buffer; step S3) comprises exchanging a certain amount of the antibody-drug conjugate obtained in S1 into the ultrafiltration-exchanging buffer obtained in S2 with an ultrafiltration membrane, diluting the antibody-drug conjugate with water for injection to the target concentration, and then adding the surfactant according to its mass fraction or its volume fraction to obtain a stock solution; optionally, the stock solution is lyophilized, preferably, the lyophilization comprises: (1) pre-freezing, (2) primary drying, and (3) secondary drying, wherein

(1) the conditions for pre-freezing are: at -60 to -30°C for 18-1800 minutes, e.g. at -45°C for 180 minutes;
(2) the conditions for the primary drying are: at -25 to 0°C under 0.02-0.5 mbar (absolute pressure) for 300 minutes to 40000 minutes, e.g. at -5°C under 0.06 mbar for 3000 minutes, or at -20°C under 0.20 mbar for 4000 minutes;
(3) the conditions for the secondary drying are: at 15 to 45°C under 0.1-0.5 mbar (absolute pressure) for 90 minutes to 12000 minutes, e.g. at 30°C under 0.20 mbar for 1200 minutes, or at 35°C under 0.25 mbar for 900 minutes.

**11.** Use of the pharmaceutical composition according to any one of claims 1-9 in manufacture of a medicament for treating a proliferative disease, preferably, said proliferative disease is preferably a disease associated with abnormal expression of HER2, HER3 or EGFR, including cancer, preferably, the cancer is selected from breast cancer, ovarian cancer, cervical cancer, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g. small cell lung cancer and non-small cell lung cancer), colon cancer, rectal cancer, colorectal cancer, leukemia (e.g. acute lymphoblastic leukemia, acute myeloid leukemia, acute promyelocytic leukemia, chronic myelocytic leukemia, chronic lymphocytic leukemia), bone cancer, skin cancer, thyroid cancer, pancreatic cancer or lymphoma (e.g. Hodgkin lymphoma, non-Hodgkin lymphoma or relapsed anaplastic large cell lymphoma); optionally, the cancer further comprises triple-negative breast cancer, esophagus cancer, pancreatic cancer, biliary tract cancer, HR-positive breast cancer, lung squamous carcinoma, non-squamous non-small cell lung cancer, head and neck squamous carcinoma (including nasopharyngeal carcinoma), urothelial carcinoma.

**12.** Use of the pharmaceutical composition according to any one of claims 1-9 in manufacture of a medicament for treating a drug-resistant proliferative disease; preferably the drug-resistant proliferative disease is a drug-resistant disease associated with abnormal expression of HER2, HER3 or EGFR, including cancer; more preferably, the drug-resistant cancer is a drug-resistant cancer caused by mutation in HER2, HER3 or EGFR genes, the cancer is preferably selected from breast cancer, ovarian cancer, cervical cancer, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g. small cell lung cancer and non-small cell lung cancer), colon cancer, rectal cancer, colorectal cancer, leukemia (e.g. acute lymphoblastic leukemia, acute myeloid leukemia, acute promyelocytic leukemia, chronic myelocytic leukemia, chronic lymphocytic leukemia), bone cancer, skin cancer, thyroid cancer, pancreatic cancer or lymphoma (e.g. Hodgkin lymphoma, non-Hodgkin lymphoma or relapsed anaplastic large cell lymphoma), optionally, the cancer further comprises triple-negative breast cancer, esophagus cancer, pancreatic cancer, biliary tract cancer, HR-positive breast cancer, lung squamous carcinoma, non-squamous non-small cell lung cancer, head and neck squamous carcinoma (including nasopharyngeal carcinoma), urothelial carcinoma; further preferably the cancer is colon cancer, rectal cancer, lung cancer or pancreatic cancer; wherein, the drug resistance preferably refers to being resistant to receptor tyrosine kinase inhibitors, further preferably resistant to first-generation, second-generation or third-generation EGFR inhibitors, still further preferably resistant to gefitinib, erlotinib, icotinib, afatinib, dacomitinib, osimertinib or almonertinib, especially resistant to gefitinib, afatinib, osimertinib or almonertinib, more preferably resistant to gefitinib or osimertinib.

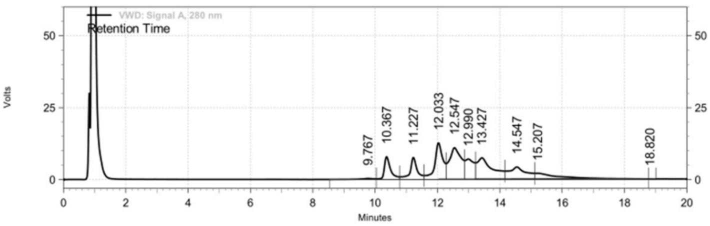


Figure 1

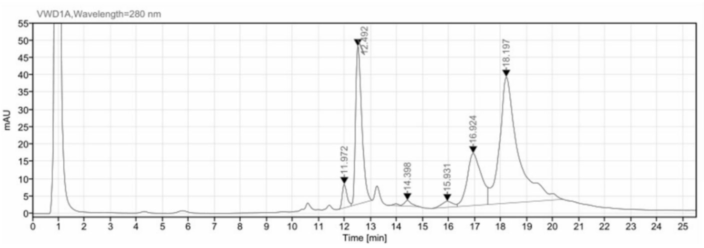


Figure 2

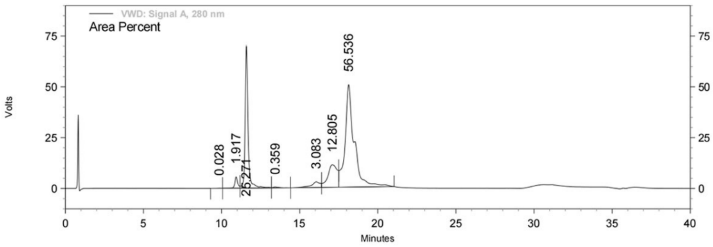


Figure 3

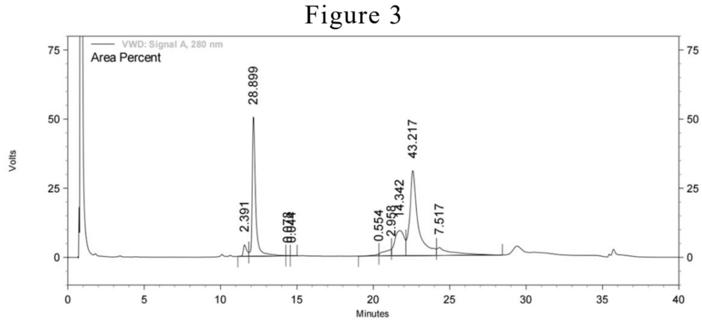


Figure 4

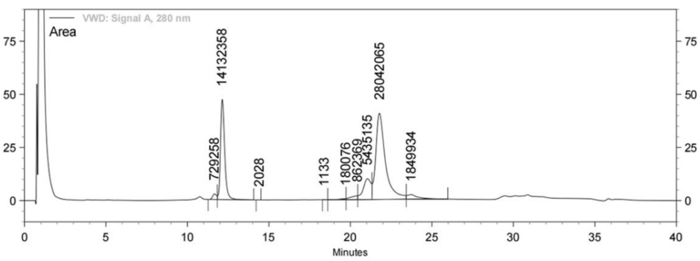


Figure 5

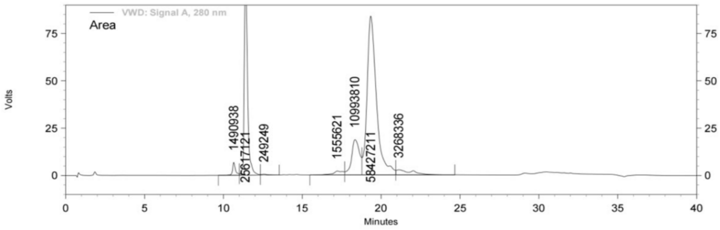


Figure 6

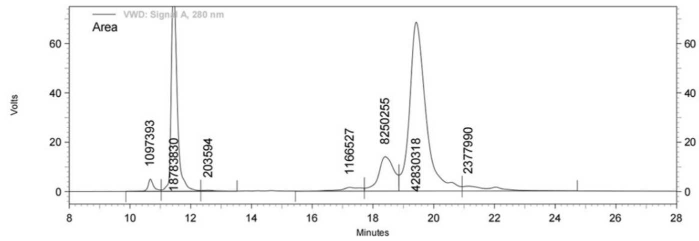


Figure 7

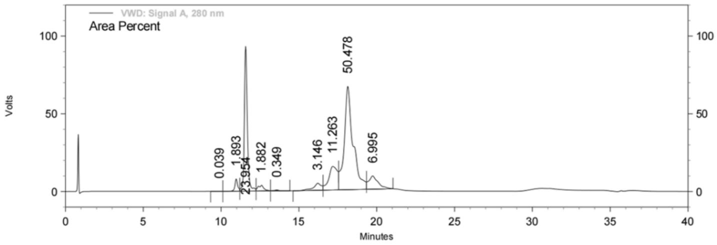


Figure 8

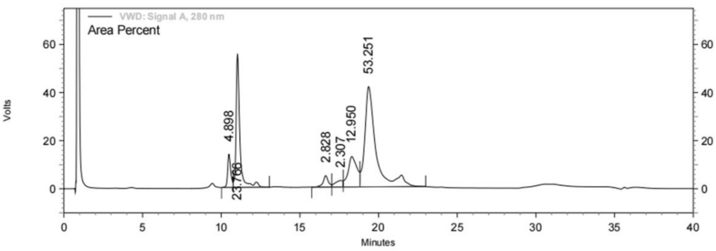


Figure 9

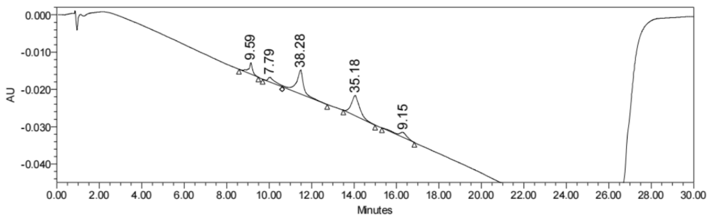


Figure 10

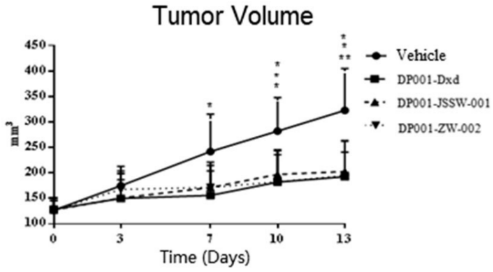


Figure 11

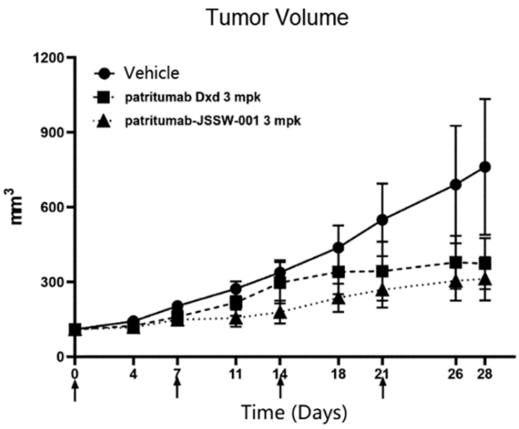


Figure 12

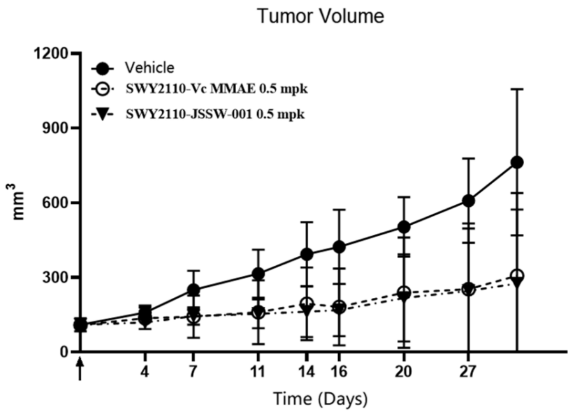


Figure 13

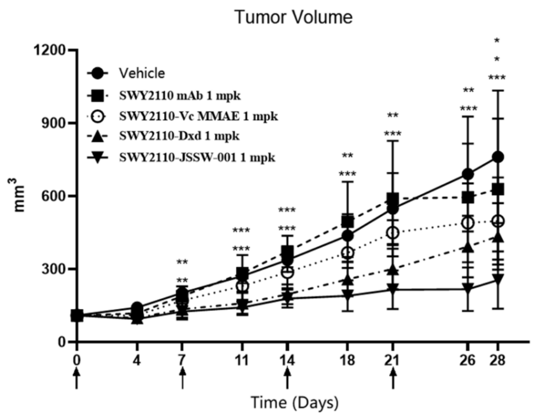


Figure 14

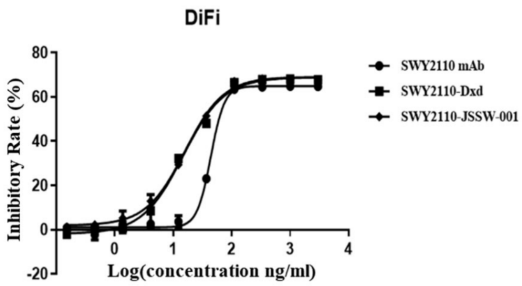


Figure 15

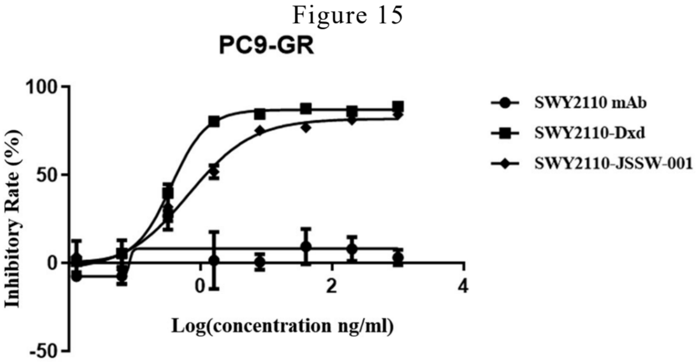


Figure 16

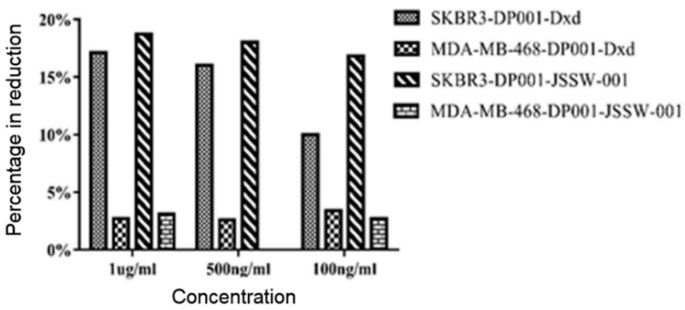


Figure 17

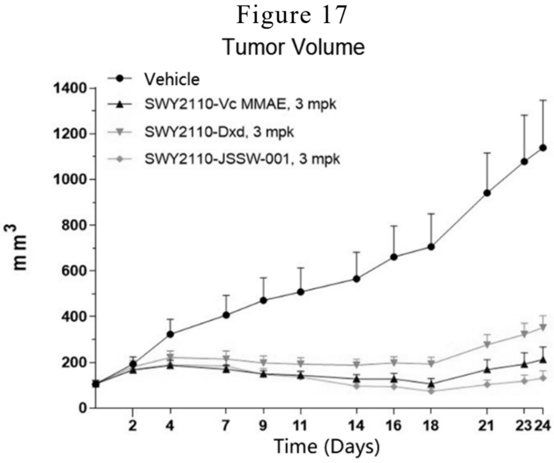


Figure 18

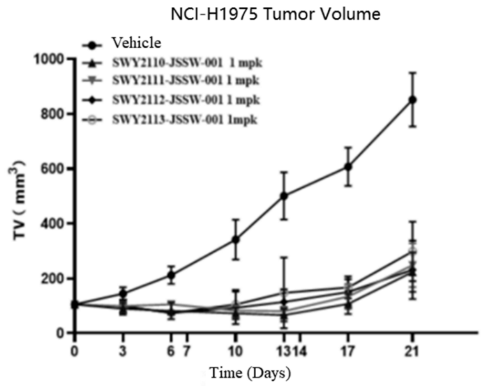


Figure 19

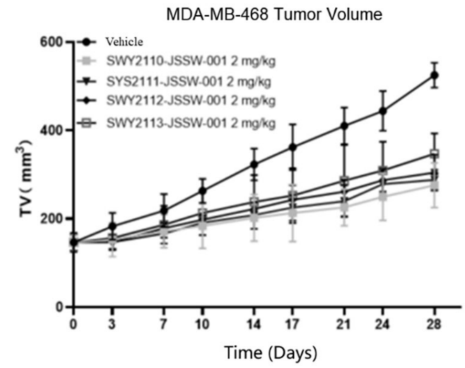


Figure 20

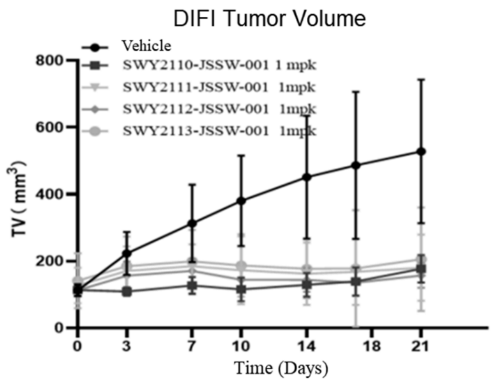


Figure 21

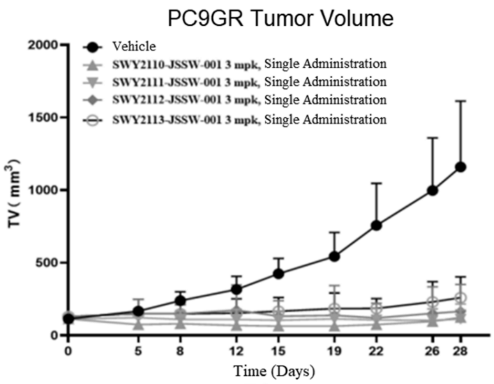


Figure 22

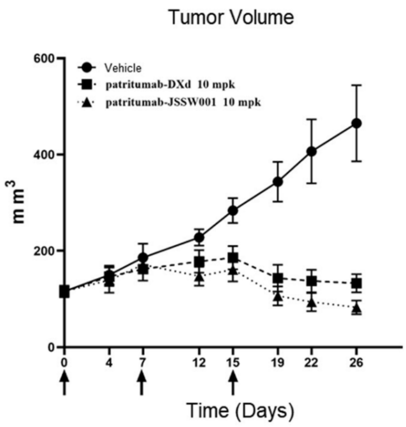


Figure 23

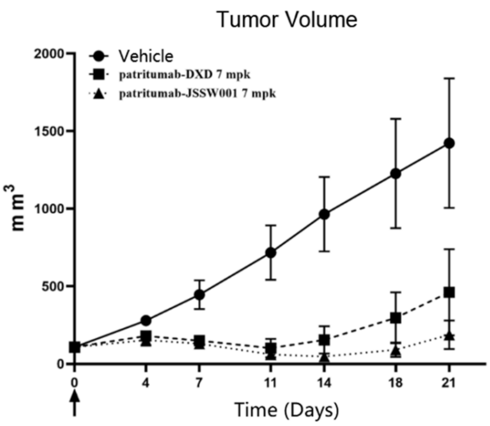


Figure 24

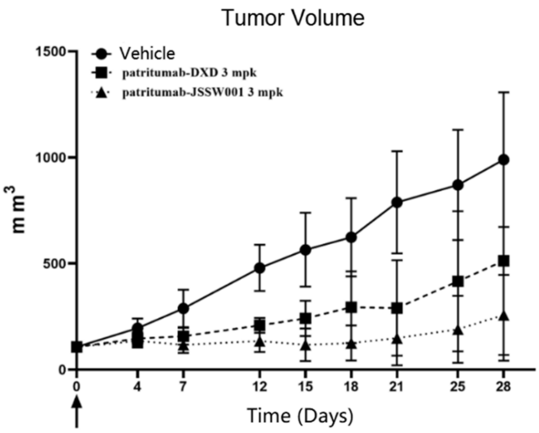


Figure 25

**INTERNATIONAL SEARCH REPORT**

International application No.
**PCT/CN2024/093528**

**A. CLASSIFICATION OF SUBJECT MATTER**

A61K47/68(2017.01)i; C07K16/28(2006.01)i; C07K16/32(2006.01)i; A61K31/4745(2006.01)i; A61K9/19(2006.01)i; A61K9/08(2006.01)i; A61P35/00(2006.01)i; A61P35/02(2006.01)i

According to International Patent Classification (IPC) or to both national classification and IPC

**B. FIELDS SEARCHED**

Minimum documentation searched (classification system followed by classification symbols)

IPC: A61K A61P C07K

Documentation searched other than minimum documentation to the extent that such documents are included in the fields searched

Electronic data base consulted during the international search (name of data base and, where practicable, search terms used)

CNABS: CNTXT; VEN; ENTXT; DWPI; CNKI; CJFD; 读秀, DUXIU; 万方, WANFANG; Elsevier; Pubmed; SpringerLink; ISI Web of Science; STNext; 中国专利生物序列检索系统, China Patents Biological Sequence Search System: 式I结构式, 注射剂, 注射液, 冻干, 粉针剂, 缓冲剂, 缓冲液, 缓冲溶液, 组氨酸, 蔗糖, 海藻糖, 曲妥珠单抗, 权利要求中的序列检索, HER2, HER3, EGFR, DP001, trastuzumab, Patritumab, SWY2110, SWY2111, SWY2112, SWY2113, injection, lyophilization, freeze-drying, buffer, histidine, sucrose, trehalose, mycose

**C. DOCUMENTS CONSIDERED TO BE RELEVANT**

| Category* | Citation of document, with indication, where appropriate, of the relevant passages | Relevant to claim No. |
|---|---|---|
| PX | CN 116135232 A (CSPC JUSHI BIOLOGICAL PHARMACEUTICAL CO., LTD.) 19 May 2023 (2023-05-19)<br>claims 1-16, and description, paragraphs [0056]-[0072], [0140]-[0145], [0289], [0290], [0316], [0317], [0332], [0333], [0346]-[0363], [0481]-[0573], and [0633]-[0772] | 1-8, 10-12 |
| X | CN 113943310 A (SICHUAN BAILI PHARMACEUTICAL CO., LTD. et al.) 18 January 2022 (2022-01-18)<br>claims 21 and 24-27, and description, paragraphs [0183] and [0449] | 1-8, 10-12 |
| X | WO 2020063676 A1 (JIANGSU HENGRUI MEDICINE CO., LTD. et al.) 02 April 2020 (2020-04-02)<br>description, page 11, last paragraph to page 18, paragraph 1, page 28, last paragraph, page 29, paragraphs 1, 6, and 7, and page 30, last paragraph | 1-8, 10-12 |
| Y | CN 112566942 A (DAIICHI SANKYO CO., LTD.) 26 March 2021 (2021-03-26)<br>abstract, description, paragraph [0610], and figures 5 and 6 | 1-8, 10-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 July 2024** | **07 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
**PCT/CN2024/093528**

**C. DOCUMENTS CONSIDERED TO BE RELEVANT**

| Category* | Citation of document, with indication, where appropriate, of the relevant passages | Relevant to claim No. |
|---|---|---|
| Y | CN 104755494 A (DAIICHI SANKYO CO., LTD.) 01 July 2015 (2015-07-01) claim 1 | 1-8, 10-12 |
| Y | WO 2016131409 A1 (SHANGHAI MIRACOGEN INC.) 25 August 2016 (2016-08-25) abstract, description, page 19, paragraph 2 to last paragraph | 1-8, 10-12 |
| A | CN 113943310 A (SICHUAN BAILI PHARMACEUTICAL CO., LTD. et al.) 18 January 2022 (2022-01-18) claims 21 and 24-27, and description, paragraphs [0183] and [0449] | 9 |
| A | WO 2020063676 A1 (JIANGSU HENGRUI MEDICINE CO., LTD. et al.) 02 April 2020 (2020-04-02) description, page 11, last paragraph to page 18, paragraph 1, page 28, last paragraph, page 29, paragraphs 1, 6, and 7, and page 30, last paragraph | 9 |
| A | CN 112566942 A (DAIICHI SANKYO CO., LTD.) 26 March 2021 (2021-03-26) abstract, description, paragraph [0610], and figures 5 and 6 | 9 |
| A | WO 2016131409 A1 (SHANGHAI MIRACOGEN INC.) 25 August 2016 (2016-08-25) abstract, and description, page 19, paragraph 2 to last paragraph | 9 |
| PA | CN 116135232 A (CSPC JUSHI BIOLOGICAL PHARMACEUTICAL CO., LTD.) 19 May 2023 (2023-05-19) description, paragraphs [0481]-[0573] and [0633]-[0772] | 9 |

Form PCT/ISA/210 (second sheet) (July 2022)

**INTERNATIONAL SEARCH REPORT**

International application No.
**PCT/CN2024/093528**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☐ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☑ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/093528**

| Patent document cited in search report | | | Publication date (day/month/year) | Patent family member(s) | | | Publication date (day/month/year) |
|---|---|---|---|---|---|---|---|
| CN | 116135232 | A | 19 May 2023 | AU | 2022389555 | A1 | 04 July 2024 |
| | | | | CA | 3238578 | A1 | 25 May 2023 |
| | | | | WO | 2023088382 | A1 | 25 May 2023 |
| CN | 113943310 | A | 18 January 2022 | JP | 2023545580 | A | 30 October 2023 |
| | | | | AU | 2021361938 | A1 | 25 May 2023 |
| | | | | IL | 302053 | A | 01 June 2023 |
| | | | | EP | 4227309 | A1 | 16 August 2023 |
| | | | | WO | 2022078259 | A1 | 21 April 2022 |
| | | | | US | 2023381332 | A1 | 30 November 2023 |
| WO | 2020063676 | A1 | 02 April 2020 | ZA | 202102544 | B | 25 October 2023 |
| | | | | CA | 3114137 | A1 | 02 April 2020 |
| | | | | BR | 112021004656 | A2 | 01 June 2021 |
| | | | | JP | 2022511351 | A | 31 January 2022 |
| | | | | EP | 3858386 | A1 | 04 August 2021 |
| | | | | EP | 3858386 | A4 | 12 October 2022 |
| | | | | KR | 20210066833 | A | 07 June 2021 |
| | | | | MX | 2021003382 | A | 27 May 2021 |
| | | | | US | 2021353764 | A1 | 18 November 2021 |
| | | | | AU | 2019349207 | A1 | 08 April 2021 |
| | | | | TW | 202027795 | A | 01 August 2020 |
| | | | | TWI | 826543 | B | 21 December 2023 |
| CN | 112566942 | A | 26 March 2021 | AU | 2019311557 | A1 | 04 February 2021 |
| | | | | CA | 3107417 | A1 | 30 January 2020 |
| | | | | CA | 3107417 | C | 17 October 2023 |
| | | | | KR | 20210038904 | A | 08 April 2021 |
| | | | | IL | 280295 | A | 25 March 2021 |
| | | | | JP | 2024050683 | A | 10 April 2024 |
| | | | | TW | 202019972 | A | 01 June 2020 |
| | | | | SG | 11202100653 | YA | 25 February 2021 |
| | | | | US | 2021283269 | A1 | 16 September 2021 |
| | | | | EP | 3828206 | A1 | 02 June 2021 |
| | | | | EP | 3828206 | A4 | 20 April 2022 |
| | | | | JPWO | 2020022363 | A1 | 02 August 2021 |
| | | | | BR | 112021001194 | A2 | 27 April 2021 |
| | | | | WO | 2020022363 | A1 | 30 January 2020 |
| CN | 104755494 | A | 01 July 2015 | LT | 2907824 | T | 11 June 2018 |
| | | | | US | 2016279259 | A1 | 29 September 2016 |
| | | | | US | 9808537 | B2 | 07 November 2017 |
| | | | | CA | 3021435 | A1 | 17 April 2014 |
| | | | | CA | 3021435 | C | 12 October 2021 |
| | | | | BR | 122021014396 | B1 | 05 July 2022 |
| | | | | JP | 2023011799 | A | 24 January 2023 |
| | | | | JP | 2022008581 | A | 13 January 2022 |
| | | | | JP | 7170812 | B2 | 14 November 2022 |
| | | | | JP | 6030267 | B1 | 24 November 2016 |
| | | | | JP | 2017036274 | A | 16 February 2017 |
| | | | | BR | 112015006521 | A2 | 05 September 2017 |
| | | | | BR | 112015006521 | B1 | 03 March 2022 |
| | | | | SI | 2907824 | T1 | 29 June 2018 |
| | | | | CY | 1122789 | T1 | 05 May 2021 |

Form PCT/ISA/210 (patent family annex) (July 2022)

**INTERNATIONAL SEARCH REPORT**
**Information on patent family members**

International application No.
**PCT/CN2024/093528**

| Patent document cited in search report | Publication date (day/month/year) | Patent family member(s) | | | Publication date (day/month/year) |
|---|---|---|---|---|---|
| | | SG | 10201804788 | XA | 30 July 2018 |
| | | PT | 3342785 | T | 25 February 2020 |
| | | PT | 2907824 | T | 07 June 2018 |
| | | NZ | 746440 | A | 29 November 2019 |
| | | US | 2020282073 | A1 | 10 September 2020 |
| | | US | 11633493 | B2 | 25 April 2023 |
| | | KR | 20220100727 | A | 15 July 2022 |
| | | KR | 102456726 | B1 | 20 October 2022 |
| | | JP | 5953378 | B2 | 20 July 2016 |
| | | JPWO | 2014057687 | A1 | 05 September 2016 |
| | | CA | 2885800 | A1 | 17 April 2014 |
| | | CA | 2885800 | C | 04 December 2018 |
| | | ES | 2773710 | T3 | 14 July 2020 |
| | | WO | 2014057687 | A1 | 17 April 2014 |
| | | US | 2024082413 | A1 | 14 March 2024 |
| | | US | 2015297748 | A1 | 22 October 2015 |
| | | US | 10195288 | B2 | 05 February 2019 |
| | | AU | 2020200548 | A1 | 13 February 2020 |
| | | AU | 2020200548 | B2 | 25 August 2022 |
| | | IL | 302494 | A | 01 June 2023 |
| | | IL | 302494 | B1 | 01 July 2024 |
| | | TR | 201809636 | T4 | 23 July 2018 |
| | | KR | 20180030734 | A | 23 March 2018 |
| | | KR | 101901558 | B1 | 21 September 2018 |
| | | HRP | 20180870 | T1 | 13 July 2018 |
| | | MX | 2020010964 | A | 09 November 2020 |
| | | KR | 20150067149 | A | 17 June 2015 |
| | | KR | 101841818 | B1 | 26 March 2018 |
| | | TW | 202233186 | A | 01 September 2022 |
| | | CY | 1120363 | T1 | 10 July 2019 |
| | | KR | 20180105271 | A | 27 September 2018 |
| | | KR | 102052319 | B1 | 05 December 2019 |
| | | KR | 20190135559 | A | 06 December 2019 |
| | | KR | 102087017 | B1 | 10 March 2020 |
| | | SG | 11201502887 | WA | 28 May 2015 |
| | | DK | 3342785 | T3 | 23 March 2020 |
| | | TW | 201420117 | A | 01 June 2014 |
| | | TWI | 615152 | B | 21 February 2018 |
| | | KR | 20220029776 | A | 08 March 2022 |
| | | KR | 102417310 | B1 | 05 July 2022 |
| | | PL | 3342785 | T3 | 07 September 2020 |
| | | TW | 202033499 | A | 16 September 2020 |
| | | TWI | 757740 | B | 11 March 2022 |
| | | MY | 170251 | A | 13 July 2019 |
| | | EP | 3632471 | A1 | 08 April 2020 |
| | | TW | 201811746 | A | 01 April 2018 |
| | | TWI | 650312 | B | 11 February 2019 |
| | | RS | 57278 | B1 | 31 August 2018 |
| | | PL | 2907824 | T3 | 31 August 2018 |
| | | PH | 12018501433 | A1 | 27 February 2019 |

Form PCT/ISA/210 (patent family annex) (July 2022)

**INTERNATIONAL SEARCH REPORT**
**Information on patent family members**

International application No.
**PCT/CN2024/093528**

| Patent document cited in search report | Publication date (day/month/year) | Patent family member(s) | | | Publication date (day/month/year) |
|---|---|---|---|---|---|
| | | KR | 20210132240 | A | 03 November 2021 |
| | | KR | 102369419 | B1 | 02 March 2022 |
| | | DK | 2907824 | T3 | 23 July 2018 |
| | | ZA | 201501825 | B | 25 May 2022 |
| | | HK | 1256631 | A1 | 27 September 2019 |
| | | KR | 20210038728 | A | 07 April 2021 |
| | | KR | 102320907 | B1 | 02 November 2021 |
| | | IL | 238143 | A0 | 31 May 2015 |
| | | IL | 238143 | B | 30 January 2020 |
| | | KR | 20200026323 | A | 10 March 2020 |
| | | KR | 102237639 | B1 | 07 April 2021 |
| | | SI | 3342785 | T1 | 28 February 2020 |
| | | JP | 2016196484 | A | 24 November 2016 |
| | | JP | 6186045 | B2 | 23 August 2017 |
| | | KR | 20230142808 | A | 11 October 2023 |
| | | NZ | 705394 | A | 26 October 2018 |
| | | AU | 2013328111 | A1 | 12 March 2015 |
| | | AU | 2013328111 | B2 | 02 November 2017 |
| | | RS | 60000 | B1 | 30 April 2020 |
| | | IL | 271760 | A | 27 February 2020 |
| | | IL | 271760 | B | 01 August 2022 |
| | | EP | 2907824 | A1 | 19 August 2015 |
| | | EP | 2907824 | A4 | 08 June 2016 |
| | | EP | 2907824 | B1 | 11 April 2018 |
| | | RU | 2015113767 | A | 10 December 2016 |
| | | RU | 2664465 | C2 | 17 August 2018 |
| | | ES | 2671644 | T3 | 07 June 2018 |
| | | MX | 2015003903 | A | 17 July 2015 |
| | | MX | 364484 | B | 29 April 2019 |
| | | TW | 201920098 | A | 01 June 2019 |
| | | TWI | 696611 | B | 21 June 2020 |
| | | HK | 1210477 | A1 | 22 April 2016 |
| | | NZ | 740948 | A | 29 November 2019 |
| | | NZ | 746439 | A | 29 November 2019 |
| | | EP | 3342785 | A1 | 04 July 2018 |
| | | EP | 3342785 | B1 | 25 December 2019 |
| | | JP | 2020180124 | A | 05 November 2020 |
| | | JP | 6952835 | B2 | 27 October 2021 |
| | | BR | 122021014365 | B1 | 05 July 2022 |
| | | KR | 20230086800 | A | 15 June 2023 |
| | | KR | 102584005 | B1 | 27 September 2023 |
| | | HUE | 039000 | T2 | 28 December 2018 |
| | | JP | 2018188455 | A | 29 November 2018 |
| | | JP | 6715888 | B2 | 01 July 2020 |
| | | KR | 20220146669 | A | 01 November 2022 |
| | | KR | 102498405 | B1 | 09 February 2023 |
| | | MX | 2019004502 | A | 21 August 2019 |
| | | LT | 3342785 | T | 10 February 2020 |
| | | PH | 12015500667 | A1 | 18 May 2015 |
| | | US | 2019008981 | A1 | 10 January 2019 |

Form PCT/ISA/210 (patent family annex) (July 2022)

**INTERNATIONAL SEARCH REPORT**
**Information on patent family members**

International application No.
**PCT/CN2024/093528**

| Patent document cited in search report | Publication date (day/month/year) | Patent family member(s) | Publication date (day/month/year) |
|---|---|---|---|
| | | US 10973924 B2 | 13 April 2021 |
| | | ZA 201900820 B | 31 August 2022 |
| | | AU 2018200308 A1 | 01 February 2018 |
| | | AU 2018200308 B2 | 07 November 2019 |
| | | JP 2018008982 A | 18 January 2018 |
| | | JP 6371452 B2 | 08 August 2018 |
| | | AU 2022203560 A1 | 16 June 2022 |
| | | HUE 048748 T2 | 28 August 2020 |
| | | HRP 20200353 T1 | 12 June 2020 |
| | | RU 2018128384 A | 02 October 2018 |
| | | RU 2018128384 A3 | 20 December 2021 |
| | | KR 20230024433 A | 20 February 2023 |
| | | KR 102540419 B1 | 05 June 2023 |
| | | IL 294622 A | 01 September 2022 |
| | | IL 294622 B1 | 01 June 2023 |
| | | IL 294622 B2 | 01 October 2023 |
| WO 2016131409 A1 | 25 August 2016 | US 2018036423 A1 | 08 February 2018 |
| | | US 10792370 B2 | 06 October 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211461614
- US 4816567 A


### Non-patent literature cited in the description


- Antibody-drug conjugates: present and future. **BECK, ALAIN** ; **JANICE M. REICHERT**. MAbs. Taylor & Francis, 2014, vol. 6
- **MCCOMBS** ; **JESSICA R.** ; **SHAWN C. OWEN**. Antibody drug conjugates: design and selection of linker, payload and conjugation chemistry.. *The AAPS journal*, 2015, vol. 17, 339-351
- **KOHLER et al.** *Nature*, 1975
- *CHEMICAL ABSTRACTS*, 1599440-13-7
- *CHEMICAL ABSTRACTS*, 646502-53-6
- General Rule 0512 High Performance Liquid Chromatography. Chinese Pharmacopoeia. 2020
- General Rule 3127 Determination of Molecular Size Variants of Monoclonal Antibodies. Chinese Pharmacopoeia. 2020
- General Rule 3129, Determination of Charge Variants of Monoclonal Antibodies. Chinese Pharmacopoeia. 2020
- General Rule 0512 High Performance Liquid Chromatography and 0431 Mass Spectrometry. Chinese Pharmacopoeia. 2020
- General Rule 0542. Chinese Pharmacopoeia. 2020